# EUROPEAN PATENT SPECIFICATION

(11) **EP 4 013 453 B1**
(45) Date of publication and mention of the grant of the patent: **04.03.2026**
(21) Application number: 20761459.5
(22) Date of filing: 12.08.2020
(51) Int. Cl.: A61K 47/52, A61K 38/22, A61K 47/54, A61P 19/00, C07K 14/58, A61K 9/00, A61K 9/14

(54) **HYDROPHOBIC PEPTIDE SALTS FOR EXTENDED RELEASE COMPOSITIONS**
HYDROPHOBE PEPTIDSALZE FÜR ZUSAMMENSETZUNGEN MIT VERLÄNGERTER FREISETZUNG
SELS PEPTIDIQUES HYDROPHOBES POUR COMPOSITIONS À LIBÉRATION PROLONGÉE

(30) Priority: 12.08.2019 US 201962885705 P; 13.11.2019 US 201962935052 P; 20.01.2020 US 202062963354 P; 23.01.2020 US 202062964848 P; 12.06.2020 US 202063038652 P
(43) Date of publication of application: 22.06.2022
(73) Proprietor: BioMarin Pharmaceutical Inc., Novato, CA 94949 (US)
(72) Inventor: ANG, JooChuan, Novato, CA 94949 (US); BATTU, Sunil, Kumar, Novato, CA 94949 (US); LABER, Joshua, Novato, CA 94949 (US); ESTRADA, Karol, Novato, CA 94949 (US); LEBOWITZ, Jonathan, Novato, CA 94949 (US); CHOU, Tian Wei, Novato, CA 94949 (US)
(74) Representative: D Young & Co LLP
(86) International application number: PCT/US2020/045885
(87) International publication number: WO 2021/030411

(56) References cited:
- WO-A1-2017/020034
- WO-A1-2019/090030
- US-A1- 2010 035 821
- US-A1- 2010 297 021
- KURT D. RISTROPH ET AL: "Hydrophobic ion pairing: encapsulating small molecules, peptides, and proteins into nanocarriers", NANOSCALE ADVANCES, vol. 1, no. 11, 1 January 2019 (2019-01-01), pages 4207 - 4237, XP055736481, DOI: 10.1039/C9NA00308H

## Description

### FIELD OF THE DISCLOSURE

The present disclosure, relates, in general, to hydrophobic salts of hydrophilic peptides that form low solubility materials in aqueous solutions and are capable of extended or sustained release of the peptide component when administered to a subject.

### BACKGROUND

Sustained delivery therapeutics are desirable, for example, to reduce the number of doses, or reduce the amount of drug a subject may receive in order to achieve a therapeutic benefit. However, certain types of active ingredients in drugs are difficult to formulate into sustained release compositions (e.g., enterically coated, dose responsive capsules or tablets, or microspheres, such as liposomes or nanoparticles), that may deliver the therapeutic at certain sites in vivo or have certain slow release properties that allow drug to escape from the particle slowly over time.

US 2010/0035821 relates to cyclic constructs which bind to a natriuretic peptide receptor and include a plurality of amino acid residues, at least one amino acid surrogate and a cyclic linkage including one or two amide bonds, and pharmaceutical compositions and methods of treatment including such cyclic constructs.

WO 2019/090030 A1 relates to methods for encapsulating water-soluble biologic and small molecule active pharmaceutical ingredients (APIs) into nanoparticles by applying nanoprecipitation techniques and ion-pairing the nanoparticles with hydrophobic counterions to produce new API salts that exhibit altered solubilities.

K. Ristroph and R. Prud'homme Nanoscale Adv. 2019, 1, 4207 relates to encapsulating small molecules, peptides and proteins into nanocarriers using hydrophobic ion pairing.

### SUMMARY

The present disclosure relates to compositions comprising salts of electrostatically charged peptides that have low solubility in solution such that the salts form solids or semi-solids in aqueous medium. It is shown here that such salts have slower dissolution rates than the non-salt form of the peptide in aqueous solution, and can be used for extended release therapeutics without having to be reformulated in a typical extended release format.

Provided herein is a composition comprising a hydrophobic salt of a C-type natriuretic peptide (CNP), the salt comprising the CNP complexed with a hydrophobic counterion, wherein the CNP is selected from the group consisting of:
DLRVDTKSRAAWARLLQEHPNARKYKGANKKGLSKGCFGLKLDRIGSMSGLGC (CNP-53) (SEQ ID NO: 56);
LRVDTKSRAAWARLLQEHPNARKYKGANKKGLSKGCFGLKLDRIGSMSGLGC (CNP-52) (SEQ ID NO:15);
RVDTKSRAAWARLLQEHPNARKYKGANKKGLSKGCFGLKLDRIGSMSGLGC (CNP-51) (SEQ ID NO:16); VDTKSRAAWARLLQEHPNARKYKGANKKGLSKGCFGLKLDRIGSMSGLGC (CNP-50) (SEQ ID NO17:);
DTKSRAAWARLLQEHPNARKYKGANKKGLSKGCFGLKLDRIGSMSGLGC (CNP-49) (SEQ ID NO: 18); TKSRAAWARLLQEHPNARKYKGANKKGLSKGCFGLKLDRIGSMSGLGC (CNP-48) (SEQ ID NO: 19); KSRAAWARLLQEHPNARKYKGANKKGLSKGCFGLKLDRIGSMSGLGC (CNP-47) (SEQ ID NO: 20);
SRAAWARLLQEHPNARKYKGANKKGLSKGCFGLKLDRIGSMSGLGC (CNP-46) (SEQ ID NO: 21); RAAWARLLQEHPNARKYKGANKKGLSKGCFGLKLDRIGSMSGLGC (CNP-45) (SEQ ID NO: 22); AAWARLLQEHPNARKYKGANKKGLSKGCFGLKLDRIGSMSGLGC (CNP-44) (SEQ ID NO:23); AWARLLQEHPNARKYKGANKKGLSKGCFGLKLDRIGSMSGLGC (CNP-43) (SEQ ID NO:24); WARLLQEHPNARKYKGANKKGLSKGCFGLKLDRIGSMSGLGC (CNP-42) (SEQ ID NO:25); ARLLQEHPNARKYKGANKKGLSKGCFGLKLDRIGSMSGLGC (CNP-41) (SEQ ID NO:26); RLLQEHPNARKYKGANKKGLSKGCFGLKLDRIGSMSGLGC (CNP-40) (SEQ ID NO:27); LLQEHPNARKYKGANKKGLSKGCFGLKLDRIGSMSGLGC (CNP-39) (SEQ ID NO:28); LQEHPNARKYKGANKKGLSKGCFGLKLDRIGSMSGLGC (SEQ ID NO:2); QEHPNARKYKGANKKGLSKGCFGLKLDRIGSMSGLGC (CNP-37) (SEQ ID NO:3); EHPNARKYKGANKKGLSKGCFGLKLDRIGSMSGLGC (CNP-36) (SEQ ID NO:29); HPNARKYKGANKKGLSKGCFGLKLDRIGSMSGLGC (CNP-35) (SEQ ID NO:30); PNARKYKGANKKGLSKGCFGLKLDRIGSMSGLGC (CNP-34) (SEQ ID NO:4); NARKYKGANKKGLSKGCFGLKLDRIGSMSGLGC (CNP-33) (SEQ ID NO:31); ARKYKGANKKGLSKGCFGLKLDRIGSMSGLGC (CNP-32) (SEQ ID NO:32); RKYKGANKKGLSKGCFGLKLDRIGSMSGLGC (CNP-31) (SEQ ID NO:33); KYKGANKKGLSKGCFGLKLDRIGSMSGLGC (CNP-30) (SEQ ID NO:34); YKGANKKGLSKGCFGLKLDRIGSMSGLGC (CNP-29) (SEQ ID NO:35); KGANKKGLSKGCFGLKLDRIGSMSGLGC (CNP-28) (SEQ ID NO:36); GANKKGLSKGCFGLKLDRIGSMSGLGC (CNP-27) (SEQ ID NO:37);
ANKKGLSKGCFGLKLDRIGSMSGLGC (CNP-26) (SEQ ID NO:38);
NKKGLSKGCFGLKLDRIGSMSGLGC (CNP-25) (SEQ ID NO:39);
KKGLSKGCFGLKLDRIGSMSGLGC (CNP-24) (SEQ ID NO:40);
KGLSKGCFGLKLDRIGSMSGLGC (CNP-23) (SEQ ID NO:41);
GLSKGCFGLKLDRIGSMSGLGC (CNP-22) (SEQ ID NO: 68); LSKGCFGLKLDRIGSMSGLGC (CNP-21) (SEQ ID NO:42); SKGCFGLKLDRIGSMSGLGC (CNP-20) (SEQ ID NO:43);
KGCFGLKLDRIGSMSGLGC (CNP-19) (SEQ ID NO:44); GCFGLKLDRIGSMSGLGC (CNP-18) (SEQ ID NO:45); CFGLKLDRIGSMSGLGC (CNP-17) (SEQ ID NO: 67),
QEHPNARKYKGANKKGLSKGCFGLKLDRIGSNSGLGC [CNP-37(M32N); SEQ ID NO: 46];
MQEHPNARKYKGANKKGLSKGCFGLKLDRIGSMSGLGC (Met-CNP-37; SEQ ID NO:47);
PQEHPNARKYKGANKKGLSKGCFGLKLDRIGSMSGLGC (Pro-CNP-37; SEQ ID NO: 48);
GQEHPNARKYKGANKKGLSKGCFGLKLDRIGSNSGLGC [Gly-CNP-37 (M32N); SEQ ID NO: 49]; MGQEHPNARKYKGANKKGLSKGCFGLKLDRIGSMSGLGC (Met-Gly-CNP-37; SEQ ID NO: 50); GQEHPNARKYKGANKKGLSKGCFGLKLDRIGSMSGLGC (Gly-CNP-37: SEQ ID NO:51) GQEHPNARKYKGANPKGLSKGCFGLKLDRIGSMSGLGC (SEQ ID NO:52);
GQEHPNARKYKGANQKGLSKGCFGLKLDRIGSMSGLGC (SEQ ID NO:53);
GQEHPNARKYKGANQQGLSKGCFGLKLDRIGSMSGLGC (SEQ ID NO:54);
GQEHPNARKYKGANKPGLSKGCFGLKLDRIGSMSGLGC (SEQ ID NO:55),
PGQEHPNARKYKGANKKGLSKGCFGLKLDRIGSMSGLGC(SEQ ID NO: 1);
PGQEHPNARRYRGANRRGLSRGCFGLKLDRIGSMSGLGC(SEQ ID NO: 6);
PGQEHPQARRYRGAQRRGLSRGCFGLKLDRIGSMSGLGC(SEQ ID NO: 5); and
PGQEHPQARKYKGAQKKGLSKGCFGLKLDRIGSMSGLGC(SEQ ID NO: 7).

In various embodiments, the % peptide in the peptide salt by weight is at least about 10% peptide by weight in the peptide salt. In various embodiments, the peptide in the peptide salt by weight is at least about 10%, 20%, 30%, 40%, 50%, 60%, 70% or more. In various embodiments, the % of active peptide in the peptide salt by weight is at least about 5% by weight. In various embodiments, % of active peptide in the peptide salt by weight is at least about 5%, 10%, 15%, 20%, 25%, 30%, 35% or more. In various embodiments, the % active peptide as compared to the total peptide present in the salt is at least about 50%, 60%, 70%. 80% or more. In various embodiments, the hydrophobic peptide salts have slow dissolution, and are not immediately soluble at 1 mg/mL in 1x phosphate buffered saline (PBS).

In various embodiments, the hydrophobic counterion is complexed to the electrostatically charged peptide via a non-covalent bond.

In various embodiments, the hydrophobic peptide salt further comprises a polyvalent cation complexed to the peptide-hydrophobic counterion complex. In various embodiments, the electrostatically charged peptide, hydrophobic counterion, and polyvalent cation are complexed via non-covalent bonds. In various embodiments, the polyvalent cation complexed to the peptide-hydrophobic counterion complex is a metal cation.

In various embodiments, the peptide salt comprising the hydrophobic counterion has a cLogP of about 0 to about 10, or the conjugate acid of the hydrophobic counterion has a pKa of -2 to 5, or both. In various embodiments, the peptide salt comprising the hydrophobic counterion has a cLogP of about 2 to about 9, or the conjugate acid of the hydrophobic counterion has a pKa less than about 5, or both. In various embodiments, the peptide salt comprising the hydrophobic counterion has a cLogP of about 2 to about 9, and the conjugate acid of the hydrophobic counterion has a pKa less than about 5. In various embodiments, the peptide salt comprising the hydrophobic counterion has a cLogP of about 2 to about 9, and the conjugate acid of the hydrophobic counterion has a pKa of about 0 to about 5.

In various embodiments, the hydrophobic counterion is selected from the group consisting of a deprotonated fatty acid, a deprotonated bile acid, naphthoate and derivatives thereof, nicotinate and derivatives thereof, an alkyl sulfonate, a dialkyl sulfosuccinate, a phospholipid, an alkyl sulfonate, an aryl sulfonate, an alkylbenzene sulfonate, an alkyl sulfate, an aryl sulfate, a dextran sulfate, an alkylbenzene sulfate, an ionic surfactant, and a combination of any of the foregoing. In various embodiments, the hydrophobic counterion is selected from the group consisting of palmitate, deoxycholate, oleate, pamoate, nicotinate, dodecyl sulfate, docusate, myristate, palmitate, stearate, phosphatidylethanolamine (PE), phosphatidylcholine (PC), phosphatidylserine (PS), phosphatidylinositol (PL), phosphatidate, decanoate, 2-naphthalenesulfonate, 1-heptanesulfonate, 1-octanesulfonate monohydrate, 1-decanesulfonate, dodecyl sulfate, dextran sulfate, and dodecyl benzenesulfonate. In various embodiments, the hydrophobic counterion is oleate, deoxycholate, decanoate, pamoate, docusate or dodecyl sulfate. In various embodiments, the counterion is selected from the group consisting of oleate, pamoate, deoxycholate, decanoate and docusate.

In various embodiments, the polyvalent cation has a charge of +2, +3 or +4, or higher. In various embodiments, the polyvalent cation has a charge of +2, +3 or +4. In certain embodiments, the polyvalent cation has a charge of +2. In certain embodiments, the polyvalent cation has a charge of +3. In certain embodiments, the polyvalent cation has a charge of +4. In various embodiments, the cation is a metal cation. In various embodiments, the cation comprises a metal selected from the group consisting of beryllium (Be), magnesium (Mg), calcium (Ca), strontium (Sr), barium (Ba), zinc (Zn), cadmium (Cd), boron (B), aluminum (Al), gallium (Ga), indium (In), thallium (TI), iron (Fe), manganese (Mn), cobalt (Co), nickel (Ni), titanium (Ti), vanadium (V), platinum (Pt), copper (Cu) and gold (Au). In various embodiments, the cation comprises zinc or calcium. In various embodiments, the cation is Mg²⁺, Zn²⁺ or Ca²⁺. In various embodiments, the cation is Zn²⁺ or Ca²⁺. In certain embodiments, the cation is Zn²⁺. In certain embodiments, the cation is Ca²⁺.

In various embodiments, the peptide salt is in solid, semi-solid, gel, crystalline, amorphous, nanoparticle, microparticle, amorphous nanoparticle, amorphous microparticle, crystalline nanoparticle or crystalline microparticle form. In various embodiments, the peptide salt is in solid form. In various embodiments, the peptide salt is in an amorphous form. In various embodiments, the peptide salt is in gel form. In various embodiments, the peptide salt is suspended in or linked to a gel.

In various embodiments, the CNP is a CNP variant as defined in the claims. CNP and CNP variants contemplated herein are described more fully in the Detailed Description. According to the present invention, the CNP is complexed to the hydrophobic counterion to form a hydrophobic CNP salt complex. In various embodiments, the hydrophobic CNP salt further comprises a polyvalent cation complexed to the CNP-hydrophobic counterion complex, forming a CNP-cation-hydrophobic counterion salt complex. In various embodiments, the polyvalent cation is a metal cation.

In various embodiments, the CNP is selected from the group consisting of:
PGQEHPNARKYKGANKKGLSKGCFGLKLDRIGSMSGLGC (Pro-Gly-CNP-37; SEQ ID NO: 1)
LQEHPNARKYKGANKKGLSKGCFGLKLDRIGSMSGLGC (CNP-38) (SEQ ID NO:2);
QEHPNARKYKGANKKGLSKGCFGLKLDRIGSMSGLGC (CNP-37) (SEQ ID NO:3);
PNARKYKGANKKGLSKGCFGLKLDRIGSMSGLGC (CNP-34) (SEQ ID NO:4) and salts thereof.

In various embodiments, the CNP salt useful for forming the hydrophobic CNP salt herein, is CNP-acetate.

In various embodiments, the CNP is selected from the group consisting of
PGQEHPQARRYRGAQRRGLSRGCFGLKLDRIGSMSGLGC (SEQ ID NO: 5);
PGQEHPNARKYKGANKKGLSKGCFGLKLDRIGSMSGLGC (SEQ ID NO: 1);
PGQEHPNARRYRGANRRGLSRGCFGLKLDRIGSMSGLGC (SEQ ID NO: 6);
PGQEHPQARRYRGAQRRGLSRGCFGLKLDRIGSMSGLGC (SEQ ID NO: 5); and
PGQEHPQARKYKGAQKKGLSKGCFGLKLDRIGSMSGLGC (SEQ ID NO: 7).

In various disclosures, the CNP variant peptide further comprises an acetyl group. In various embodiments, the acetyl group is on the N-terminus of the peptide. In various embodiments, the acetyl group is on an amino acid side chain within the peptide sequence. In various embodiments, the peptide further comprises an OH or an NH₂ group at the C-terminus.

In various disclosures, the CNP variant is selected from the group consisting of
Ac-PGQEHPQARRYRGAQRRGLSRGCFGLKLDRIGSMSGLGC-OH (SEQ ID NO: 8);
Ac-PGQEHPNARKYKGANKKGLSKGCFGLKLDRIGSMSGLGC-NH₂ (SEQ ID NO: 9);
Ac-PGQEHPNARRYRGANRRGLSRGCFGLKLDRIGSMSGLGC-OH (SEQ ID NO: 10);
Ac-PGQEHPNARRYRGANRRGLSRGCFGLKLDRIGSMSGLGC-NH₂ (SEQ ID NO: 11);
Ac-PGQEHPQARRYRGAQRRGLSRGCFGLKLDRIGSMSGLGC-NH₂ (SEQ ID NO: 12);
Ac- PGQEHPQARKYKGAQKKGLSKGCFGLKLDRIGSMSGLGC-NH₂ (SEQ ID NO: 13); and
Ac- PGQEHPQARKYKGAQKKGLSKGCFGLKLDRIGSMSGLGC-OH (SEQ ID NO: 14).

In various embodiments, the hydrophobic counterion is oleate, deoxycholate, decanoate, pamoate, docusate or dodecyl sulfate. In various embodiments, if a polyvalent cation is present, the polyvalent cation comprises zinc or calcium. In various embodiments, if a polyvalent cation is present, the polyvalent cation comprises magnesium, zinc or calcium. In various embodiments, the cation is Mg²⁺, Zn²⁺ or Ca²⁺. In various embodiments, the polyvalent cation is Zn²⁺ or Ca²⁺. In various embodiments, the polyvalent cation is Zn²⁺. In various embodiments, the polyvalent cation is Ca²⁺.

In various embodiments, the composition further comprises an excipient, diluent or carrier. In various embodiments, the excipient, diluent or carrier is a pharmaceutically acceptable excipient, diluent or carrier. Also provided is a sterile pharmaceutical composition comprising the hydrophobic salt composition described herein.

Further contemplated by the disclosure is a modified release composition comprising a hydrophobic peptide salt described herein. In various embodiments, the modified release composition is an extended release composition, sustained release composition or delayed release composition.

In various embodiments, the extended release composition comprises a hydrophobic peptide salt, wherein the peptide salt solid, semi-solid, gel, crystalline, amorphous, nanoparticle, microparticle, amorphous nanoparticle, amorphous microparticle, crystalline nanoparticle or crystalline microparticle is resuspended in an aqueous solution or in oil. In various embodiments, the aqueous solution is water, saline, or buffer.

In various embodiments, the oil comprises a triglyceride or a fatty acid. In various embodiments, the fatty acid is saturated or unsaturated. In various embodiments, the fatty acid is a short chain, medium chain or long chain fatty acid. In various embodiments, when the fatty acid is in a triglyceride, the fatty acid is saturated or unsaturated, and can be a medium chain or long chain fatty acid.

In various embodiments, the fatty acid is a C-6 to C-20 fatty acid. In various embodiments, the fatty acid is a C-6, C-8, C-10, C-12, C-14, C-16, C-18, or C-20 fatty acid. In various embodiments, the fatty acid is hexanoic acid, octanoic acid, decanoic acid, or dodecanoic acid.

In various embodiments, for the extended release composition, (i) less than about 20% of peptide is released by day 1; and (ii) about 90% of peptide is released weekly, or about 90% of peptide is released bi-weekly, or about 90% of peptide is released monthly, at pH 7 to 7.6.

In various embodiments, less than about 20% of peptide is released by day 1 at pH 7 to 7.6. It is further contemplated that (i) less than about 30%, or about 40%, or about 50%, or about 60% of peptide is released by day 1, at pH 7.0 to 7.6; and (ii) about 90% of peptide is released weekly, or about 90% of peptide is released bi-weekly, or about 90% of peptide is released monthly, at pH 7 to 7.6. It is further contemplated that (i) less than about 30%, or about 40%, or about 50%, or about 60% of peptide is released by day 1, at pH 7.0 to 7.6; and (ii) about 70%, about 80%, or about 90% of peptide is released weekly; or about 70%, about 80%, or about 90% of peptide is released bi-weekly; or about 70%, about 80%, or about 90% of peptide is released every three weeks; or about 70%, about 80%, or about 90% of peptide is released monthly, at pH 7 to 7.6.

In various embodiments, (i) less than about 25%, about 30%, about 35%, about 40%, about 45%, about 50%, about 55%, about 60%, about 65%, about 70%, or about 75% of peptide is released by day 1, at pH 7.0 to 7.6; and (ii) about 90% of peptide is released weekly, or about 90% of peptide is released bi-weekly, or about 90% of peptide is released monthly, at pH 7 to 7.6. It is further contemplated that (i) less than about 25%, about 30%, about 35%, about 40%, about 45%, about 50%, about 55%, about 60% about 65%, about 70%, or about 75% of peptide is released by day 1, at pH 7.0 to 7.6; and (ii) about 70%, about 80%, or about 90% of peptide is released weekly; or about 70%, about 80%, or about 90% of peptide is released bi-weekly; or about 70%, about 80%, or about 90% of peptide is released every three weeks; or about 70%, about 80%, or about 90% of peptide is released monthly, at pH 7 to 7.6; or alternatively ii) about 70%, about 75%, about 80%, about 85%, or about 90% of peptide is released weekly; or about 70%, about 75%, about 80%, about 85%, or about 90% of peptide is released bi-weekly; or about 70%, about 75%, about 80%, about 85%, or about 90% of peptide is released every three weeks; or about 70%, about 75%, about 80%, about 85%, or about 90% of peptide is released monthly, at pH 7 to 7.6

In various embodiments, about 90% of peptide is released weekly, at pH 7 to 7.6. In various embodiments, about 90% of peptide is released bi-weekly, at pH 7 to 7.6. In various embodiments, about 90% of peptide is released monthly, at pH 7 to 7.6. It is further contemplated that the release can be at a pH between pH 7.0 to 7.6, between pH 7.1 to 7.5, between pH 7.2 to 7.4, between pH 7.2 to 7.6, or between pH 7.0 to 7.4.

In various embodiments, the extended release composition comprises an excipient, diluent or carrier. In various embodiments, the excipient, diluent or carrier is a pharmaceutically acceptable excipient, diluent or carrier. In various embodiments, provided is a sterile pharmaceutical composition comprising the extended release composition.

Also provided herein but not part of the present invention is a method of making a hydrophobic peptide salt composition as defined in the claims. Ionic surfactants are good candidates as counterions since the polar headgroup is perpetually charged irrespective of complexation pH. Modulation of pH at which complexation occurs results in different amounts of charge on the peptide and this allows for the control of peptide:surfactant complexation stoichiometry and possibly control over resulting precipitate size. Metal cations can be used as bridges for anionic side chains of peptide amino acids to bind to anionic hydrophobic counterions. The order and rate of cation and counterion addition to the peptide is important to minimize precipitation of metal cation with anionic hydrophobic counterions.

In various embodiments, the disclosure but not part of the present invention contemplates a method of making a composition comprising a salt of an electrostatically charged peptide as defined in the claims comprising, a) contacting an electrostatically charged peptide in an aqueous solution with a hydrophobic counterion in solution; b) mixing the electrostatically charged peptide solution with the hydrophobic counterion solution in a manner sufficient for the peptide and counterion to form a complex, wherein the formation of the peptide-counterion complex results in formation of a solid, semi-solid, gel, crystalline, amorphous, nanoparticle, microparticle, amorphous nanoparticle, amorphous microparticle, crystalline nanoparticle or crystalline microparticle form comprising the hydrophobic peptide salt. According to the invention, the peptide salt is a hydrophobic CNP salt.

In various disclosures, the method optionally comprises before step (b), contacting the electrostatically charged peptide in solution with a polyvalent cation in aqueous solution, forming a peptide-cation complex. In various embodiments, the polyvalent cation is a metal cation.

In various disclosures, the mixing is carried out by dropwise addition of the hydrophobic counterion solution to the electrostatically charged peptide solution. In various disclosures, the solutions are mixed by vortexing after addition of each drop of hydrophobic counterion solution, or other means for mixing known in the art.

In various disclosures, the method further comprises step (c) washing the peptide salt in buffer or water. In various disclosures, the washing is carried out in an aqueous solution, e.g., buffer or water.

In various disclosures, the method further comprises step (d) obtaining the peptide salt by centrifugation to form a peptide salt pellet. In various disclosures, if the salt is in gel form, the salt is obtained by centrifugation or by decanting the liquid phase followed by lyophiliation or other drying methods

In various disclosures, the method further comprises step (e) removing water from the peptide salt pellet. It is contemplated that water or another aqueous solution can be removed from the pellet by lyophilization or drying using techniques known in the art.

In various instances, the method further comprises resuspending the pellet in an aqueous solution or oil. In various disclosures, the aqueous solution is water, saline, or buffer. In various disclosures the oil comprises a triglyceride or a fatty acid. In various disclosures, the fatty acid is saturated or unsaturated. In various disclosures, the fatty acid in the triglyceride is saturated or unsaturated, or combinations thereof.

The fatty acid may be the oil itself or in a triglyceride. In various embodiments, the fatty acid is a short chain, medium chain or long chain fatty acid. In various embodiments, when the fatty acid is in a triglyceride, the fatty acid is saturated or unsaturated, and can be a medium chain or long chain fatty acid. In various embodiments, the fatty acid is a C-6 to C-20 fatty acid.

In various embodiments, the fatty acid is a C-6, C-8, C-10, C-12, C-14, C-16, C-18 or C-20 fatty acid. In various embodiments, the fatty acid is hexanoic acid, octanoic acid, decanoic acid, or dodecanoic acid.

In various disclosures, the synthetic method contemplates using a peptide:
hydrophobic counterion ratio of at least one molar equivalent of hydrophobic counterion to the total number of positively charged amino acids in the peptide. In various embodiments the peptide: hydrophobic counterion ratio used in the synthetic method is between 1:1 to 1:20, or from 1:1 to 1:50. The peptide: counterion ratio used in the synthetic method can be between 1:2 to 1:15, 1:2 to 1:10, 1:2 to 1:8, 1:3 to 1:10, or 1:4 to 1:10. In various embodiments, the peptide: counterion ratio is 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19 or 1:20. In various embodiments, the peptide: hydrophobic counterion ratio used in the synthetic method is at least two molar equivalents of hydrophobic counterion to total number of positively charged amino acids in the peptide. In various embodiments, the peptide: hydrophobic counterion ratio used in the synthetic method is at least three molar equivalents of hydrophobic counterion to total number of positively charged amino acids in the peptide.

In various disclosures, the synthetic method contemplates using a peptide: cation ratio of at least one molar equivalents of cation to total number of negatively charge amino acids in the peptide. In various embodiments, the peptide: cation ratio used in the synthetic method is between 1:1 to 1:10. The peptide: cation ratio used in the synthetic method can be 1:2 to 1:10, 1:3 to 1:10, 1:1 to 1:5, 1:2 to 1:5, of 1:2 to 1:8. In various embodiments, the peptide: cation ratio used in the synthetic method is 1:1, 1:2, 1:3, 1:4, 1:5, 1:6, 1:7, 1:8, 1:9, or 1:10. In various embodiments, the peptide: cation ratio used in the synthetic method at least two molar equivalents of cation to total number of negatively charged amino acids in the peptide. In various embodiments, the peptide: cation ratio used in the synthetic method is at least three molar equivalents of cation to total number of negatively charged amino acids in the peptide. Further contemplated are combinations of the peptide: cation and peptide: hydrophobic counterion ratios described above.

An exemplary ratio is one counterion per positive charge on a peptide. For the polyvalent cation, an exemplary ratio is approximately 1 metal cation per negative charge site in the peptide, and 2 polyvalent cations per negative charge site, or a 2X molar excess. For example, one or two polyvalent cations such as Zn²⁺ or Ca²⁺ can be used in combination with 6-8 counterions, or more if a hydrophobic interaction is involved.

In various embodiments, the hydrophobic counterion is complexed via a non-covalent bond.

In various embodiments, if the salt complex further comprises a polyvalent cation complexed to the peptide-counterion complex, the cation is complexed via a non-covalent bond. In various embodiments, the electrostatically charged peptide, hydrophobic counterion, and cation are complexed via a non-covalent bonds.

In various embodiments, the CNP is selected from the group consisting of:
PGQEHPNARKYKGANKKGLSKGCFGLKLDRIGSMSGLGC (Pro-Gly-CNP-37) (SEQ ID NO: 1).

Also provided is a composition according to the claims for use in treating a bone growth disorder or skeletal dysplasia in a subject in need thereof.

In various embodiments, the bone growth disorder or skeletal dysplasia is selected from the group consisting of osteoarthritis, hypophosphatemic rickets, achondroplasia, hypochondroplasia, short stature, dwarfism, osteochondrodysplasias, thanatophoric dysplasia, osteogenesis imperfecta, achondrogenesis, chondrodysplasia punctata, homozygous achondroplasia, chondrodysplasia punctata, camptomelic dysplasia, congenital lethal hypophosphatasia, perinatal lethal type of osteogenesis imperfecta, short-rib polydactyly syndromes, hypochondroplasia, rhizomelic type of chondrodysplasia punctata, Jansen-type metaphyseal dysplasia, spondyloepiphyseal dysplasia congenita, atelosteogenesis, diastrophic dysplasia, congenital short femur, Langer-type mesomelic dysplasia, Nievergelt-type mesomelic dysplasia, Robinow syndrome, Reinhardt syndrome, acrodysostosis, peripheral dysostosis, Kniest dysplasia, fibrochondrogenesis, Roberts syndrome, acromesomelic dysplasia, micromelia, Morquio syndrome, Kniest syndrome, metatrophic dysplasia, and spondyloepimetaphyseal dysplasia, NPR2 mutation, SHOX mutation (Turner's syndrome/Leri Weill), PTPN11 mutations (Noonan's syndrome), and idiopathic short stature.

In various embodiments the CNP variants are useful as an adjunct or alternative to growth hormone for treating idiopathic short stature and other skeletal dysplasias.

In various embodiments, the bone growth disorder, skeletal dysplasia or short stature disorder results from an NPR2 mutation, SHOX mutation (Turner's syndrome/Leri Weill), or PTPN11 mutations (Noonan's syndrome).

In various embodiments, the bone growth disorder, skeletal dysplasia or short stature disorder results from an NPR2 mutation, SHOX mutation (Turner's syndrome/Leri Weill), or PTPN11 mutations (Noonan's syndrome), or insulin growth factor 1 receptor (IGF1R).

In various embodiments, the CNP variants are useful to treat growth plate disorders and short stature, including familial short stature, dominant familial short stature which is also known as dominant inherited short stature, or idiopathic short stature. In various embodiments, the short stature or growth plate disorder is a result of a mutation in collagen (COL2A1, COL11A1, COL9A2, COL10), aggrecan (ACAN), indian hedgehog (IHH), PTPN11, NPR2, NPPC, or FGFR3.

In various embodiments, the growth plate disorder or short stature is associated with one or more mutations in a gene associated with a RASopathy.

In various embodiments, the bone growth disorder, skeletal dysplasia or short stature disorder results from a RASopathy. In various embodiments, the RASopathy is Noonan syndrome, Costello syndrome, Cardiofaciocutaneous syndrome, Neurofibromatosis Type 1, or LEOPARD syndrome.

In one embodiment, the RASopathy is hereditary gingival fibromatosis type 1.

In various embodiments, the CNP variants are useful to treat growth plate disorders and short stature, including familial short stature, dominant familial short stature which is also known as dominant inherited short stature, or idiopathic short stature. In various embodiments, the short stature or growth plate disorder is a result of a mutation in collagen (COL2A1, COL11A1, COL9A2, COL10), aggrecan (ACAN), indian hedgehog (IHH), PTPN11, NPR2, NPPC, FGFR3, or insulin growth factor 1 receptor (IGF1R).

In various embodiments, the short stature is associated with one or more mutations in a gene associated with a RASopathy.

In various embodiments, the CNP variants are useful to treat a subject with short stature having a height SDS of less than -1.0, -1.5, -2.0, -2.5, or -3.0, and having at least one parent with a height SDS of less than -1.0, -1.5, -2.0 or -2.5, optionally wherein the second parent has height within the normal range. In various embodiments, the CNP variants are useful to treat a subject with short stature having a height SDS of between -2.0 to -3.0. In various embodiments, the CNP variants are useful to treat a subject with short stature having a height SDS of between -2.0 to -2.5. In various embodiments, the short stature is associated with one or more mutations in a gene associated with short stature, such as, collagen (COL2A1, COL11A1, COL9A2, COL10), aggrecan (ACAN), indian hedgehog (IHH), PTPN11, NPR2, NPPC, FGFR3, or insulin growth factor 1 receptor (IGF1R), or combinations thereof. In various embodiments, the short stature is associated with one or more mutations in a gene associated with a RASopathy.

In various embodiments, the short stature is a result of mutations in multiple genes as determined by polygenic risk score (PRS). In various embodiments, the subject has a mutation in NPR2 and a low PRS. In various embodiments, the subject has a mutation in FGFR3 and a low PRS. In various embodiments, the subject has a mutation in NPR2 and a low PRS. In various embodiments, the subject has a mutation in IGF1R and a low PRS. In various embodiments, the subject has a mutation in NPPC and a low PRS. In various embodiments, the subject has a mutation in SHOX and a low PRS. In various embodiments, the subject has one or more mutation in one or more of FGFR3, IGF1R, NPPC, NPR2 and SHOX, and a low PRS. In various embodiments, the PRS is 1 or 2. In various embodiments, the PRS is 1. In various embodiments, the PRS is 2.

In various embodiments, the CNP variant is PGQEHPNARKYKGANKKGLSKGCFGLKLDRIGSMSGLGC (Pro-Gly-CNP-37) (SEQ ID NO: 1). In various disclosures (not part of the present invention) the peptide further comprises an acetyl group. In various embodiments, the acetyl group is on the N-terminus of the peptide. In various embodiments, the acetyl group is on an amino acid side chain within the peptide sequence. In various embodiments, the peptide further comprises an OH or an NH₂ group at the C-terminus. In various embodiments, the variant comprises one or more linker groups as described herein. In various embodiments, the linker is a hydrolysable linker. In various embodiments, the variant is a hydrophobic salt of an electrostatically charged CNP peptide, the salt comprising the electrostatically charged CNP peptide complexed with a hydrophobic counterion.

The disclosure (not part of the present invention) also contemplates a method of elongating a bone or increasing long bone growth in a subject in need thereof, comprising administering to the subject a sustained release composition comprising a salt of C-type natriuretic peptide (CNP), and wherein the administering elongates a bone or increases long bone growth, including compositions and extended release compositions as described herein.

In various embodiments, the CNP is a CNP variant. In various embodiments, the CNP is selected from the group consisting of:
PGQEHPNARKYKGANKKGLSKGCFGLKLDRIGSMSGLGC (Pro-Gly-CNP-37; SEQ ID NO: 1)
LQEHPNARKYKGANKKGLSKGCFGLKLDRIGSMSGLGC (CNP-38) (SEQ ID NO:2);
QEHPNARKYKGANKKGLSKGCFGLKLDRIGSMSGLGC (CNP-37) (SEQ ID NO:3);
PNARKYKGANKKGLSKGCFGLKLDRIGSMSGLGC (CNP-34) (SEQ ID NO:4); and,
pharmaceutical salts thereof. In various embodiments, the CNP is a CNP-acetate.

In various embodiments, the CNP is selected from the group consisting of
PGQEHPQARRYRGAQRRGLSRGCFGLKLDRIGSMSGLGC (SEQ ID NO: 5);
PGQEHPNARKYKGANKKGLSKGCFGLKLDRIGSMSGLGC (SEQ ID NO: 1);
PGQEHPNARRYRGANRRGLSRGCFGLKLDRIGSMSGLGC (SEQ ID NO: 6);
PGQEHPNARRYRGANRRGLSRGCFGLKLDRIGSMSGLGC (SEQ ID NO: 6);
PGQEHPQARRYRGAQRRGLSRGCFGLKLDRIGSMSGLGC (SEQ ID NO: 5); and
PGQEHPQARKYKGAQKKGLSKGCFGLKLDRIGSMSGLGC (SEQ ID NO: 7).

In various disclosures, but not claimed in the present invention, the CNP variant further comprises an acetyl group. In various embodiments, the acetyl group is on the N-terminus of the peptide. In various embodiments, the acetyl group is on a side group of an amino acid of the peptide. In various embodiments, the peptide further comprises an OH or an NH₂ group at the C-terminus.

In various disclosures, but not claimed in the present invention, the variant is selected from the group consisting of
Ac-PGQEHPQARRYRGAQRRGLSRGCFGLKLDRIGSMSGLGC-OH (SEQ ID NO: 8);
Ac-PGQEHPNARKYKGANKKGLSKGCFGLKLDRIGSMSGLGC-NH₂; (SEQ ID NO: 9)
Ac-PGQEHPNARRYRGANRRGLSRGCFGLKLDRIGSMSGLGC-OH (SEQ ID NO: 10);
Ac-PGQEHPNARRYRGANRRGLSRGCFGLKLDRIGSMSGLGC-NH₂ (SEQ ID NO: 11);
Ac-PGQEHPQARRYRGAQRRGLSRGCFGLKLDRIGSMSGLGC-NH₂ (SEQ ID NO: 12);
Ac- PGQEHPQARKYKGAQKKGLSKGCFGLKLDRIGSMSGLGC-NH₂ (SEQ ID NO: 13); and
Ac- PGQEHPQARKYKGAQKKGLSKGCFGLKLDRIGSMSGLGC-OH (SEQ ID NO: 14).

In various embodiments, the composition is administered subcutaneously, intradermally, intraarticularly, orally, or intramuscularly.

In various embodiments, the composition is administered once daily, once weekly, once every two weeks, once every three weeks, once every 4 weeks, once every 6 weeks, once every two months, once every three months or once every six months.

In various embodiments, the composition is an extended release composition.

In various embodiments, the hydrophobic CNP salt further comprises a cation in complex with the CNP and hydrophobic counterion. In various embodiments, the hydrophobic CNP salt is a purified salt. In various embodiments, the salt has at least about 80%, 85%, 90%, 91%, 92%, 93%, 94%, 95%, 96%, 97%, 98%, 99% or 99.5% purity, or more.

In various embodiments, the hydrophobic salt comprising a hydrophobic counterion selected from the group consisting of oleate, deoxycholate, decanoate, pamoate, docusate or dodecyl sulfate. In various embodiments, hydrophobic salt comprises a cation of Zn²⁺ or Ca²⁺.

In various embodiments, the hydrophobic salt is selected from the group consisting of CNP-oleate, CNP-pamoate, CNP-deoxycholate, CNP-decanoate and CNP-docusate. In various embodiments, he hydrophobic salt is selected from the group consisting of CNP-Ca⁺²(Oleate), CNP-Ca⁺²(Pamoate), CNP-Ca⁺²(deoxycholate), CNP-Ca⁺²(decanoate), CNP-Ca⁺²(docusate), CNP-Zn⁺²(Oleate), CNP-Zn⁺²(Pamoate), CNP-Zn⁺²(deoxycholate), CNP-Zn⁺²(decanoate), and CNP-Zn⁺²(docusate).

In various embodiments, the hydrophobic salt comprises a CNP selected from the group consisting of: PGQEHPNARKYKGANKKGLSKGCFGLKLDRIGSMSGLGC (Pro-Gly-CNP-37; SEQ ID NO: 1), LQEHPNARKYKGANKKGLSKGCFGLKLDRIGSMSGLGC (CNP-38) (SEQ ID NO:2); QEHPNARKYKGANKKGLSKGCFGLKLDRIGSMSGLGC (CNP-37) (SEQ ID NO:3); PNARKYKGANKKGLSKGCFGLKLDRIGSMSGLGC (CNP-34) (SEQ ID NO:4), and salts thereof. In various embodiments, the hydrophobic salt comprises a CNP which is CNP-acetate.

In various embodiments, the CNP variant is PGQEHPNARKYKGANKKGLSKGCFGLKLDRIGSMSGLGC (Pro-Gly-CNP-37) (SEQ ID NO: 1). In various disclosures (not presently claimed), the peptide further comprises an acetyl group. In various embodiments, the acetyl group is on the N-terminus of the peptide. In various embodiments, the acetyl group is on a side group of an amino acid of the peptide. In various embodiments, the peptide further comprises an OH or an NH₂ group at the C-terminus. In various embodiments, the variant comprises one or more linker groups as described herein. In various embodiments, the linker is a hydrolysable linker. In various embodiments, the peptide comprises a hydrophobic salt of an electrostatically charged peptide, the salt comprising the electrostatically charged peptide complexed with a hydrophobic counterion.

The disclosure also provides a composition comprising a hydrophobic peptide salt, as described herein for use in treating skeletal dysplasia or bone-related disorder as described herein. In certain embodiments, the disclosure provides use of a composition comprising a hydrophobic peptide salt, as described herein in the preparation of a medicament for treating skeletal dysplasia or bone-related disorder as described herein. According to the invention, the hydrophobic peptide salt is a hydrophobic CNP salt as described herein.

### BRIEF DESCRIPTION OF THE DRAWINGS

Figures 1A-1D show dissolution profiles of the different hydrophobic CNP salts in water at pH 6.5.
Figure 2 shows dissolution profiles of the different hydrophobic CNP salts in water at pH 6.5. The control is CNP-acetate.
Figure 3 shows the effects of a CNP variant (Pro-Gly-CNP37) on cells carrying either NPR2 homozygous or heterozygous mutations, as measured by cGMP stimulation.
Figure 4 shows the nucleotide and predicted protein sequence of the first exon in NPR2 mutant clones transfected into RCS cells.
Figure 5 shows exemplary NPR2 mutations analyzed for response to CNP
Figure 6 shows exemplary mutations associated with short stature in FGFR3, IGF1R, NPPC, NPR2 and SHOX.

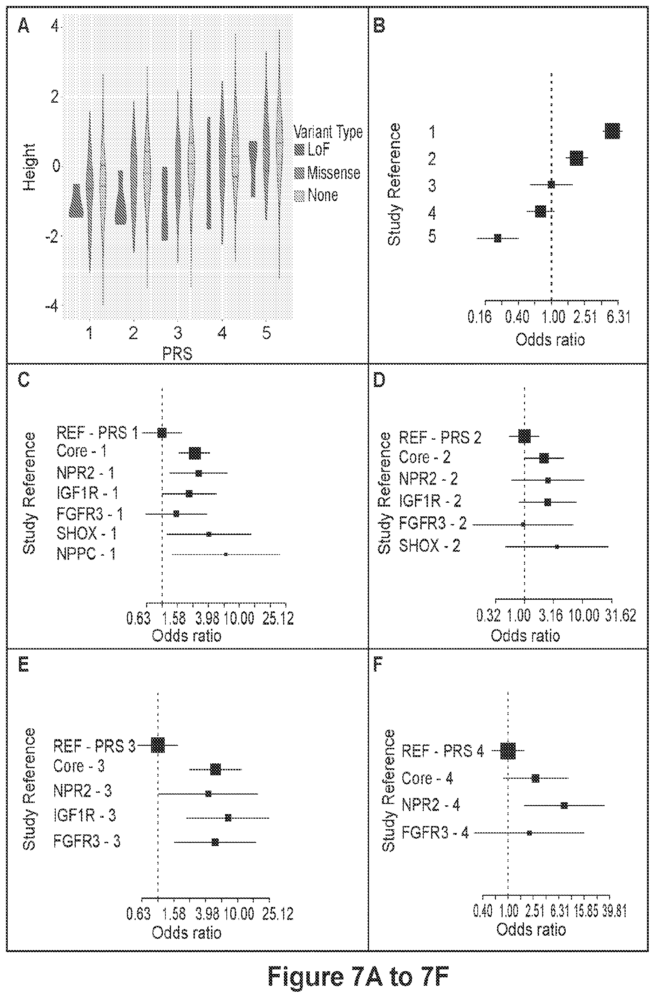

Figures 7A-7F illustrate the combined effect of PRS and rare coding variants on Height. Fig. 7A. Effects on height as a quantitative trait, samples were divided in five groups based on their PRS, violin-plots with horizontal lines representing the 25%, 50% and 75% percentile of height. Samples were grouped by carrying status of missense, loss of function or None in any of the five core genes. Fig. 7B. Effect reflected on Odds ratios for "Idiopathic Short Stature" or ISS. Odds for ISS using PRS =3 as reference vs the other PRS groups. Fig. 7C. Odds for ISS using PRS =1 as reference vs having missense and/or loss of function variants in core genes. Fig. 7D. Odds for ISS using PRS =1 non-carriers as reference vs having missense and/or loss of function variants in core genes. Fig. 7E. Odds for ISS using PRS =2 non-carriers as reference vs having missense and/or loss of function variants in core genes. Fig. 7F. Odds for ISS using PRS =3 non-carriers as reference vs having missense and/or loss of function variants in core genes.
Figure 8A shows the release profile of CNP Zn-pamoate salt. Data shown is an average of 3 wells. Figure 8B show the release profiles of different CNP peptide salts. Data shown is an average of 4 wells.
Figures 9A-9B illustrate the dissolution profiles of CNP pamoate salts as cumulative release profile (Figure 9A) or % released (Figure 9B). Data shown is an average of 3 wells for the 1xPBS data, and 2 wells for the 1x PBS+0.05% PS80 data.
Figures 10A to 10C show the dissolution profiles of the docusate salts either after lyophilization and storage (Figure 10A), or made fresh and then analyzed (Figures 10B-10C).
Figure 11 shows the release profile of CNP salt over 7 days in vivo.

### DETAILED DESCRIPTION

The present disclosure relates to salts of hydrophilic peptides that are solid, semi-solid gel or other salt form that are capable of extended release of the peptide active ingredient when put in aqueous solutions. For example, it is shown herein, that unexpectedly, complexation of a hydrophilic C-type natriuretic peptide (CNP) with charged, hydrophobic counterions generates low solubility peptide salts under aqueous conditions. The present disclosure shows that the peptide-hydrophobic counterion salt complex itself, including salts comprising a peptide-counterion-cation complex, can be used in a modified or extended release composition without having to encapsulate the peptide complex into a liposome or microsphere/nanoparticle. Such compositions are useful for extended release applications, e.g., in treating skeletal dysplasias and bone growth disorders as described herein.

As used in the specification and the appended claims, the indefinite articles "a" and "an" and the definite article "the" include plural as well as singular referents unless the context clearly dictates otherwise.

The term "about" or "approximately" means an acceptable error for a particular value as determined by one of ordinary skill in the art, which depends in part on how the value is measured or determined. In certain embodiments, the term "about" or "approximately" means within 1, 2, 3, or 4 standard deviations. In certain embodiments, the term "about" or "approximately" means within 30%, 25%, 20%, 15%, 10%, 9%, 8%, 7%, 6%, 5%, 4%, 3%, 2%, 1%, 0.5%, or 0.05% of a given value or range. Whenever the term "about" or "approximately" precedes the first numerical value in a series of two or more numerical values, it is understood that the term "about" or "approximately" applies to each one of the numerical values in that series.

The term "electrostatically charged peptide" as used herein refers to a peptide, e.g., a string of amino acids from 5 to 100 amino acids, that comprises charged amino acids. The peptide can have positively charged amino acids, negatively charged amino acids, or a mixture of both, such that the electrostatically charged peptide has an overall net charge and is capable of interacting with other charged moieties, e.g., a cation, anion or a counterion having charged species opposite to those in the peptide to which the counterion may bind. The electrostatically charged peptide can have a net positive or a net negative charge. When the peptide has a net positive charge, it can interact with a charged moiety that has one or more negative charges. When the peptide has a net negative charge, it can interact with a charged moiety that has one or more positive charges.

The term "hydrophobic counterion" as used herein refers to a group of electrostatically charged moieties that are hydrophobic in nature and are capable of interacting with to hydrophilic peptides. In various embodiments, the hydrophobic counterion is selected based on its cLogP value, the pKa value of its conjugate acid, or both. In various embodiments, a hydrophobic counterion has a cLogP of about 0 to about 10, or a pKa of its conjugate acid of about -2 to about 5, or both. In various embodiments, the hydrophobic counterion has a net negative charge and can interact with an electrostatically charged peptide having a net positive charge. In various embodiments, hydrophobic counterions include deprotonated fatty acids, deprotonated bile acids, naphthoate and derivatives thereof, nicotinate and derivatives thereof, alkyl sulfonates, dialkyl sulfosuccinates, phospholipids, alkyl sulfonates, aryl sulfonates, alkylbenzene sulfonates, alkyl sulfates, aryl sulfates, dextran sulfates, ionic surfactants, and alkylbenzene sulfates. In some embodiments, the hydrophobic counterion is a zwitterion (e.g., phosphatidylethanolamine). In various embodiments, hydrophobic counterions include, but are not limited to, palmitate, deoxycholate, oleate, pamoate, nicotinate, dodecyl sulfate, docusate, myristate, palmitate, stearate, phosphatidylethanolamine (PE), phosphatidylcholine (PC), phosphatidylserine (PS), phosphatidylinositol (PL), phosphatidate, decanoate, 2-naphthalenesulfonate, 1-heptanesulfonate, 1-octanesulfonate monohydrate, 1-decanesulfonate, dodecyl sulfate, dextran sulfate, and dodecyl benzenesulfonate. In some embodiments, the hydrophobic counterion has a net positive charge and can interact with an electrostatically charged peptide having a net negative charge.

The term "peptide salt" or "hydrophobic peptide salt", as used herein refers to a complex between an electrostatically charged peptide and a counterion, e.g., a hydrophobic counterion, such that the moieties are in a complex and form a salt. The peptide and counterion may be complexed non-covalently. In various embodiments, the peptide-counterion salt further comprises a polyvalent cation, such that the complex contains a peptide-cation-counterion in complex. Peptide salt or hydrophobic peptide salt refers to both a peptide-counterion complex and a peptide-cation-counterion complex.

In various embodiments, the peptide and cation are complexed non-covalently. In various embodiments, the peptide, cation, and hydrophobic counterion in the peptide salt are complexed via non-covalent bonds.

The term "C-type natriuretic peptide" or "CNP" refers to a small, single chain peptide having a 17-amino acid loop structure at the C-terminal end (GenBank Accession No. NP_077720, for the CNP precursor protein, NPPC) and variants thereof. CNP is initially produced from the natriuretic peptide precursor C (NPPC) gene as a single chain 126-amino acid pre-pro polypeptide, which is cleaved to generate pro-CNP, and an active 53-amino acid peptide (CNP-53), which is secreted and cleaved again by an unknown enzyme to produce the mature 22-amino acid peptide (CNP-22). "CNP salt" or "hydrophobic CNP salt" refers to salts as described herein comprising a counterion, such as a hydrophobic counterion, respectively, and optionally, further comprising a polyvalent cation, and comprising a CNP or CNP variant.

In various disclosures (not part of the present invention) a "CNP variant" is at least about 40%, 50%, 60%, 70%, 75%, 80%, 85%, 90%, or 95% homologous to the wild type NPPC over the same number of amino acid residues. In various embodiments, a CNP variant peptide may comprise from about 1 to about 53, or 1 to 38, or 1 to 37, or 1 to 35, or 1 to 34, or 1 to 33, or 1 to 32, or 1 to 31, or 1 to 27, or 1 to 22, or 10 to 35, or about 15 to about 37 residues of the NPPC polypeptide. In one embodiment, a CNP variant may comprise a sequence of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, or 53 amino acids derived from the NPPC polypeptide.

Provided herein are modified release compositions comprising a hydrophobic peptide salt described herein. Modified-release compositions include those that deliver a drug with a delay after its administration (delayed-release dosage) or for a prolonged period of time (extended-release dosage). Various embodiments of a peptide salt provided herein include modified-release compositions, such as extended release, sustained or controlled release, and delayed release. The term "extended release composition" refers to a composition formulated in a manner in order to make the active ingredient/drug available over an extended period of time following administration (US Pharmacopeia). Extended-release dosage include sustained-release (SR) or controlled-release (CR) forms. Sustained release maintains drug release over a sustained period but not necessarily at a constant rate, while CR maintains drug release over a sustained period at a nearly constant rate (Pharmaceutics: Drug Delivery and Targeting, Yvonne Perrie, Thomas Rades, Pharmaceutical Press, 2009). Delayed-release compositions or products are modified to delay release of the drug substance for some period of time after initial administration.

The term "effective amount" refers to a dosage sufficient to produce a desired result on a health condition, pathology, or disease of a subject or for a diagnostic purpose. The desired result may comprise a subjective or objective improvement in the recipient of the dosage. "Therapeutically effective amount" refers to that amount of an agent effective to produce the intended beneficial effect on health. An appropriate "effective" amount in any individual case may be determined by one of ordinary skill in the art using routine experimentation. It will be understood that the specific dose level and frequency of dosage for any particular patient may be varied and will depend upon a variety of factors, including the activity of the specific compound employed; the bioavailability, metabolic stability, rate of excretion and length of action of that compound; the mode and time of administration of the compound; the age, body weight, general health, sex, and diet of the patient; and the severity of the particular condition.

"Treatment" refers to prophylactic treatment or therapeutic treatment or diagnostic treatment. In certain embodiments, "treatment" refers to administration of a compound or composition to a subject for therapeutic, prophylactic or diagnostic purposes.

A "prophylactic" treatment is a treatment administered to a subject who does not exhibit signs or symptoms of a disease or exhibits only early signs of the disease, for the purpose of decreasing the risk of developing pathology. The compounds or compositions of the disclosure may be given as a prophylactic treatment to reduce the likelihood of developing a pathology or to minimize the severity of the pathology, if developed.

A "therapeutic" treatment is a treatment administered to a subject who exhibits signs or symptoms of pathology for the purpose of diminishing or eliminating those signs or symptoms. The signs or symptoms may be biochemical, cellular, histological, functional or physical, subjective or objective. The compounds of the disclosure may also be given as a therapeutic treatment or for diagnosis.

"Pharmaceutical composition" or "formulation" refers to a composition suitable for pharmaceutical use in subject animal, including humans and mammals. A pharmaceutical composition comprises a therapeutically effective amount of a hydrophobic peptide salt, e.g., a CNP salt, optionally another biologically active agent, and optionally a pharmaceutically acceptable excipient, carrier or diluent. In an embodiment, a pharmaceutical composition encompasses a composition comprising the active ingredient(s), and the inert ingredient(s) that make up the carrier, as well as any product that results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present disclosure encompass any composition made by admixing a compound of the disclosure and a pharmaceutically acceptable excipient, carrier or diluent.

"Pharmaceutically acceptable carrier" refers to any of the standard pharmaceutical carriers, buffers, and the like, such as a phosphate buffered saline solution, 5% aqueous solution of dextrose, and emulsions (e.g., an oil/water or water/oil emulsion). Non-limiting examples of excipients include adjuvants, binders, fillers, diluents, disintegrants, emulsifying agents, wetting agents, lubricants, glidants, sweetening agents, flavoring agents, and coloring agents. Suitable pharmaceutical carriers, excipients and diluents are described in Remington's Pharmaceutical Sciences, 19th Ed. (Mack Publishing Co., Easton, 1995). Preferred pharmaceutical carriers depend upon the intended mode of administration of the active agent. Typical modes of administration include enteral (e.g., oral) or parenteral (e.g., subcutaneous, intramuscular, intravenous or intraperitoneal injection; or topical, transdermal, or transmucosal administration).

A "pharmaceutically acceptable salt" is a salt that can be formulated into a compound for pharmaceutical use, including but not limited to metal salts (e.g., sodium, potassium, magnesium, calcium, *etc*.) and salts of ammonia or organic amines.

By "pharmaceutically acceptable" or "pharmacologically acceptable" is meant a material that is not biologically or otherwise undesirable, i.e., the material may be administered to an individual without causing any undesirable biological effects or without interacting in a deleterious manner with any of the components of the composition in which it is contained or with any components present on or in the body of the individual.

"Physiological conditions" refer to conditions in the body of an animal (e.g., a human). Physiological conditions include, but are not limited to, body temperature and an aqueous environment of physiologic ionic strength, pH and enzymes. Physiological conditions also encompass conditions in the body of a particular subject which differ from the "normal" conditions present in the majority of subjects, e.g., which differ from the normal human body temperature of approximately 37 °C or differ from the normal human blood pH of approximately 7.4.

As used herein, the term "subject" encompasses mammals and non-mammals. Examples of mammals include, but are not limited to, any member of the mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish, and the like. The term does not denote a particular age or gender. In various embodiments, the subject is human. In various embodiments the subject is a child or adolescent. In various embodiments, the subject is an infant.

### Electrostatically Charged Peptides and Peptide Salts

Peptide therapeutics are attractive biological therapeutic agents, but are often disadvantaged by low stability and short half-life in solution (Tang et al., Eur J Pharm Sci. 102:63-70, 2017). Attempts to improve efficacy of peptide therapeutics include attempts to encapsulate hydrophilic peptides into biodegradable particles such as liposomes or polymer particles. However, this has been difficult due to the cationic nature of these peptides and their ability to electrostatically interact with liposomes of negatively charged polymers (Griesser et al., Int J Pharmaceutics 520:267-274, 2017). Generation of hydrophobic ion pairs between hydrophilic peptides and hydrophobic moieties has been one means used to enable better encapsulation of hydrophilic polymers into microparticles or liposomes (Lu et al., Mol. Pharmaceutics 15:216-225, 2018). Hydrophobic ion pairs are formed when charged residues in the peptides interact with the oppositely charged ions in the hydrophobic moiety (Tang et al., *supra*)*.* In certain instances, it can lead to precipitation of the hydrophobic ion pair out of solution making it easier to encapsulate into a liposome or polymeric nanoparticle (Griesser et al., *supra*)

It has been discovered herein that hydrophobic ion complexes between hydrophilic CNP peptides and hydrophobic counterions generate CNP peptide salts. Generation of hydrophobic ion pairs between hydrophilic peptides and hydrophobic counterions can be enhanced by first contacting the hydrophilic peptide with a polyvalent cation (e.g., a metal cation) to enhance the interaction between the peptide and hydrophobic counterion. For example, the polyvalent cation can complex with the negatively-charged functional groups of the hydrophilic peptide, increasing the number of positive charges on the hydrophilic peptide available for complexation with the hydrophobic counterion, such as a hydrophobic anion. Thus, the polyvalent cation can bridge together the negative charges of the hydrophilic peptide and the negative charges of the hydrophobic counterion. Additionally, the present disclosure shows that the peptide-hydrophobic counterion salt complex or peptide-cation-hydrophobic counterion salt complex itself can be used in a modified or extended release composition without having to encapsulate the peptide complex into a liposome or microsphere/nanoparticle.

Electrostatically charged peptides can be a string of amino acids from 5 to 100 amino acids that comprise charged amino acids and have an overall net charge. The peptide can have positively charged amino acids, negatively charged amino acids, or a mixture of both, such that the electrostatically charged peptide is capable of interacting with other charged moieties, e.g., a cation, anion or a counterion or a combination thereof having charged species opposite to those in the peptide. In various embodiments, the electrostatically charged peptide has a net positive charge. An electrostatically charged peptide having a net positive charge can complex with a hydrophobic counterion having negative charges, such as a hydrophobic counterion having a net negative charge. In various embodiments, the electrostatically charged peptide has a net negative charge. An electrostatically charged peptide having a net negative charge can complex with a hydrophobic counterion having positive charges, such as a hydrophobic counterion having a net positive charge. In various embodiments, the electrostatically charged peptides have at least two amino acids having the same type of charge (e.g., two positively charged amino acids or two negatively charged amino acids).

Hydrophilic peptides refer to peptides that have high solubility in aqueous solution. Hydrophilic peptides contemplated herein include peptides between 5 and 100 amino acids, that have a net charge of between +3 to +15, or between +4 and +15, or between +3 to +12, or between +4 and +12, or +3, +4, +5, +6, +7, +8, +9, +10, +11, +12, +13, +14, or +15 or any range between these numbers. In various embodiments, the hydrophilic peptide has solubility of greater than 10 mg/mL in aqueous solution, or greater than 5 mg/mL solubility. In various embodiments, hydrophilic peptide also refers to peptides that have high solubility in aqueous solution, e.g., having cLogP of less than 1.

### Hydrophobic counterions

In order to generate a salt of an electrostatically charged peptide as described herein, the peptide is complexed with a counterion. In the case of hydrophilic peptides, the counterion is a hydrophobic counterion. In various embodiments, the hydrophobic counterion has a net negative charge and forms a salt with a hydrophilic peptide having a net positive charge.

It is contemplated that the counterion is complexed to the charged peptide via non-covalent bonding. The counterion may be non-covalently associated with the peptide via electrostatic interactions.

When a hydrophobic counterion is used, the hydrophobic counterion exhibits a cLogP of about 0 to about 10, or its conjugate acid exhibits a pKa from about -2 to about 5, or both. In various embodiments, the hydrophobic counterion has a cLogP of about 2 to about 9, about 3 to 8, about 4 to 7, or about 5 to 9. In various embodiments, the cLogP is about 0, 1, 2, 3, 4, 5, 6, 7, 8, 9, or 10. In various embodiments, the conjugate acid of the hydrophobic counterion has a pKa of about -1 to 4, 0 to 3, 0 to 5, 1 to 4 or 2 to 5. In various embodiments, the conjugate acid of the hydrophobic counterion has pKa of about -2, -1, 0, 1, 2, 3, 4, or 5. Further contemplated is a hydrophobic counterion having a combination of any of these values and ranges in between the values. In various embodiments, the hydrophobic counterion has a cLogP of about 2 to about 9, or its conjugate acid has a pKa less than about 5, or both. In various embodiments, the hydrophobic counterion has a cLogP of about 2 to about 9, and its conjugate acid has a pKa less than about 5.

In various embodiments, the counterion is an anion. In various embodiments, the counterion is a zwitterion. In various embodiments, the counterion is an anionic or zwitterionic detergent. In various embodiments, the hydrophobic counterion is selected from the group consisting of a deprotonated fatty acid, a deprotonated bile acid, a naphthoate and derivatives thereof, a nicotinate and derivatives thereof, an alkyl sulfonate, a dialkyl sulfosuccinate, a phospholipid, an alkyl sulfonate, an aryl sulfonate, an alkylbenzene sulfonate, an alkyl sulfate, an aryl sulfate, a dextran sulfate, an alkylbenzene sulfate, and ionic surfactants. In various embodiments, the hydrophobic counterion is selected from the group consisting of palmitate, deoxycholate, oleate, pamoate, nicotinate, dodecyl sulfate, docusate, myristic acid, palmitic acid, stearic acid, phosphatidylethanolamine (PE), phosphatidylcholine (PC), phosphatidylserine (PS), phosphatidylinositol (PL), phosphatidic acid, sodium decanoate, sodium 2-naphthalenesulfonate, sodium 1-heptanesulfonate, sodium 1-octanesulfonate monohydrate, sodium 1-decanesulfonate, sodium dodecyl sulfate, and sodium dodecyl benzenesulfonate. In various embodiments, the hydrophobic counterion is oleate, pamoate, deoxycholate, decanoate or docusate.

In various embodiments, at least one hydrophobic counterion is complexed to the hydrophilic peptide (when no polyvalent cation is present) or to the complex comprising the hydrophilic peptide and polyvalent cation (when cation is present). In various embodiments, at least two hydrophobic counterions are complexed to the hydrophilic peptide (when no cation is present) or to the complex comprising the hydrophilic peptide and polyvalent cation (when cation is present). In various embodiments, at least three hydrophobic counterions are complexed to the hydrophilic peptide (when no cation is present) or to the complex comprising the hydrophilic peptide and polyvalent cation (when cation is present). In various embodiments, at least four hydrophobic counterions are complexed to the hydrophilic peptide (when no cation is present) or to the complex comprising the hydrophilic peptide and polyvalent cation (when cation is present). In various embodiments, each positive charge of the hydrophilic peptide is complexed to a hydrophobic counterion. For example, if a peptide has four positively charged amino acids, then it can complex to four hydrophobic counterions. Similarly, if the peptide has four positively charged amino acids and is complexed to two cations, for a total of six positive charges, then the peptide can complex to six hydrophobic counterions. In various embodiments, not all of the positive charges of the hydrophilic peptide (when no cation is present) or of the complex comprising the hydrophilic peptide and polyvalent cation (when cation is present) are complexed to a hydrophobic counterion. For example, if a peptide has four positively charged amino acids, then it can complex to three hydrophobic counterions, or two hydrophobic counterions, or to one hydrophobic counterion. Similarly, if the peptide has four positively charged amino acids and is complexed to two cations, for a total of six positive charges, then the peptide can complex to five hydrophobic counterions, or to four hydrophobic counterions, or to three hydrophobic counterions, or two hydrophobic counterions, or to one hydrophobic counterion.

### Polyvalent Cations

In various embodiments, the peptide salt further comprises a polyvalent cation complexed to the peptide-counterion complex. It is contemplated that the cation is complexed to the charged peptide via a non-covalent bond. The cation may be non-covalently associated with the peptide via electrostatic interactions.

It is contemplated that the polyvalent cation has a charge of +2, +3 or +4 or higher. In embodiments, the cation has a charge of +2. In embodiments, the cation has a charge of +3. In embodiments, the cation has a charge of +4. In various embodiments, the polyvalent cation is a metal cation. Metal cations include those of the Group II and Group III metals. Suitable polyvalent cations can comprise a metal selected from the group consisting of beryllium (Be), magnesium (Mg), calcium (Ca), strontium (Sr), barium (Ba), zinc (Zn), cadmium (Cd), boron (B), aluminum (Al), gallium (Ga), indium (In), thallium (TI), iron (Fe), manganese (Mn), cobalt (Co), nickel (Ni), titanium (Ti), vanadium (V), platinum (Pt), copper (Cu) and gold (Au). In various embodiments, the polyvalent cation comprises magnesium, zinc, or calcium. In various embodiments, the polyvalent cation comprises zinc or calcium. In various embodiments, the polyvalent cation comprises zinc. In various embodiments, the polyvalent cation comprises calcium. In various embodiments, the polyvalent cation is selected from the group consisting of Mg²⁺, Zn²⁺, and Ca²⁺. In various embodiments, the polyvalent cation is Zn²⁺ or Ca²⁺. In various embodiments, the polyvalent cation is Zn²⁺. In various embodiments, the polyvalent cation is Ca²⁺.

In various embodiments, at least one polyvalent cation is complexed to the hydrophilic peptide. In various embodiments, at least two cations are complexed to the hydrophilic peptide. In various embodiments, at least three cations are complexed to the hydrophilic peptide. In various embodiments, each negative charge of the hydrophilic peptide is complexed to a polyvalent cation. For example, if a peptide has four negatively charged amino acids, then it can complex to four polyvalent cations, or three polyvalent cations, or two polyvalent cations, or one polyvalent cation.

### Peptide Salts

In various embodiments, the peptide salt is in solid, semi-solid, gel, crystalline, amorphous, nanoparticle, microparticle, amorphous nanoparticle, amorphous microparticle, crystalline nanoparticle or crystalline microparticle form. In various embodiments, the peptide salt is in solid, semi-solid, or gel form. In various embodiments, the peptide salt is in solid or gel form. In various embodiments, the peptide salt is in solid form. In various embodiments, the peptide salt is in amorphous form. In various embodiments, the peptide salt is in gel form. In various embodiments, the peptide salt is suspended in or linked to a gel.

It is contemplated that the peptide salt is in particle form, and in particular, as solid particles. In various embodiments, the particle is between 1 and 10,000 micrometers (um), between 1 um to 2000 um, between 2 um to 1000 um, between 5 um to 500 um, between 10 um to 1000 um, between 50 um to 500 um, between 100 um to 800 um, between 200 um to 600 um, between 300 um to 500 um, between 100 um to 300 um, between 50 um to 100 um, or between 10 um to 50 um. In various embodiments, the particle is a nanoparticle. In various embodiments, the nanoparticle is between 5 nanometers (nm) to 1000 nm, between 8 nm to 900 nm, between 10 nm to 800 nm, between 20 nm to 600 nm, between 50 nm to 500 nm, between 50 nm to 400 nm, between 20 to 300 nm, between 300 to 800 nm, between 200 to 600 nm, between 100 nm to 300 nm or between 50 nm to 200 nm.

In some embodiments, the hydrophilic peptide of the peptide salt is CNP or a CNP variant, as described herein, and the hydrophobic counterion is selected from the group consisting of palmitate, deoxycholate, oleate, pamoate, nicotinate, dodecyl sulfate, docusate, myristic acid, palmitic acid, stearic acid, phosphatidylethanolamine (PE), phosphatidylcholine (PC), phosphatidylserine (PS), phosphatidylinositol (PL), phosphatidic acid, sodium decanoate, sodium 2-naphthalenesulfonate, sodium 1-heptanesulfonate, sodium 1-octanesulfonate monohydrate, sodium 1-decanesulfonate, sodium dodecyl sulfate, and sodium dodecyl benzenesulfonate. In some embodiments, the hydrophilic peptide salt is CNP or a CNP variant, as described herein, and the hydrophobic counterion is selected from the group consisting of oleate, pamoate, deoxycholate, and decanoate. In various embodiments, the peptide salt is selected from the group consisting of CNP-oleate, CNP-pamoate, CNP-deoxycholate, and CNP-decanoate, In some embodiments, the hydrophilic peptide salt is CNP or a CNP variant, as described herein, and the hydrophobic counterion is selected from the group consisting of oleate, pamoate, deoxycholate, decanoate and docusate. In various embodiments, the peptide salt is selected from the group consisting of CNP-oleate, CNP-pamoate, CNP-deoxycholate, CNP-decanoate and CNP-docusate. In various embodiments, the peptide salt is selected from the group consisting of CNP-oleate, CNP-pamoate, and CNP-docusate. In various embodiments, the peptide salt is selected from the group consisting of CNP-deoxycholate, CNP-decanoate and CNP-docusate. In various embodiments, the peptide salt is selected from the group consisting of CNP-oleate and CNP-pamoate. In various embodiments, the peptide salt is CNP-oleate. In various embodiments, the peptide salt is CNP-pamoate. In various embodiments, the peptide salt is CNP-docusate.

In various embodiments, the hydrophilic peptide of the peptide salt is Pro-Gly CNP37 (PG-CNP37), and the hydrophobic counterion is selected from the group consisting of palmitate, deoxycholate, oleate, pamoate, nicotinate, dodecyl sulfate, docusate, myristic acid, palmitic acid, stearic acid, phosphatidylethanolamine (PE), phosphatidylcholine (PC), phosphatidylserine (PS), phosphatidylinositol (PL), phosphatidic acid, sodium decanoate, sodium 2-naphthalenesulfonate, sodium 1-heptanesulfonate, sodium 1-octanesulfonate monohydrate, sodium 1-decanesulfonate, sodium dodecyl sulfate, and sodium dodecyl benzenesulfonate. In some embodiments, the hydrophilic peptide salt is PG-CNP37, as described herein, and the hydrophobic counterion is selected from the group consisting of oleate, pamoate, deoxycholate, and decanoate. In various embodiments, the peptide salt is selected from the group consisting of PG-CNP37-oleate, PG-CNP37-pamoate, PG-CNP37-deoxycholate, and PG-CNP37-decanoate, In some embodiments, the hydrophilic peptide salt is PG-CNP37, as described herein, and the hydrophobic counterion is selected from the group consisting of oleate, pamoate, deoxycholate, decanoate and docusate. In various embodiments, the peptide salt is selected from the group consisting of PG-CNP37-oleate, PG-CNP37-pamoate, PG-CNP37-deoxycholate, PG-CNP37-decanoate and PG-CNP37-docusate. In various embodiments, the peptide salt is selected from the group consisting of PG-CNP37-oleate, PG-CNP37-pamoate, and PG-CNP37-docusate. In various embodiments, the peptide salt is selected from the group consisting of PG-CNP37-deoxycholate, PG-CNP37-decanoate and PG-CNP37-docusate. In various embodiments, the peptide salt is selected from the group consisting of PG-CNP37-oleate and PG-CNP37-pamoate. In various embodiments, the peptide salt is PG-CNP37-oleate. In various embodiments, the peptide salt is PG-CNP37-pamoate. In various embodiments, the peptide salt is PG-CNP37-docusate.

In various embodiments, the peptide-counterion salt further comprises a polyvalent cation. In some embodiments, the hydrophilic peptide of the peptide salt is CNP or a CNP variant, as described herein; the hydrophobic counterion is selected from the group consisting of palmitate, deoxycholate, oleate, pamoate, nicotinate, dodecyl sulfate, docusate, myristic acid, palmitic acid, stearic acid, phosphatidylethanolamine (PE), phosphatidylcholine (PC), phosphatidylserine (PS), phosphatidylinositol (PL), phosphatidic acid, sodium decanoate, sodium 2-naphthalenesulfonate, sodium 1-heptanesulfonate, sodium 1-octanesulfonate monohydrate, sodium 1-decanesulfonate, sodium dodecyl sulfate, and sodium dodecyl benzenesulfonate; and the polyvalent cation comprises a metal selected from the group consisting of beryllium (Be), magnesium (Mg), calcium (Ca), strontium (Sr), barium (Ba), zinc (Zn), cadmium (Cd), boron (B), aluminum (Al), gallium (Ga), indium (In), thallium (TI), iron (Fe), manganese (Mn), cobalt (Co), nickel (Ni), titanium (Ti), vanadium (V), platinum (Pt), copper (Cu) and gold (Au). In various embodiments, the hydrophilic peptide salt is CNP, as described herein, the hydrophobic counterion is selected from the group consisting of oleate, pamoate, deoxycholate, and decanoate; and the polyvalent cation is Zn²⁺ or Ca²⁺. In various embodiments, the peptide salt is selected from the group consisting of CNP-Ca⁺²(Oleate), CNP-Ca⁺²(Pamoate), CNP-Ca⁺²(deoxycholate), CNP-Ca⁺²(decanoate), CNP-Zn⁺²(Oleate), CNP-Zn⁺²(Pamoate), CNP-Zn⁺²(deoxycholate) and CNP-Zn⁺²(decanoate). In various embodiments, the hydrophilic peptide salt is CNP, as described herein, the hydrophobic counterion is selected from the group consisting of oleate, pamoate, deoxycholate, decanoate and docusate; and the polyvalent cation is Zn²⁺ or Ca²⁺. In various embodiments, the hydrophilic peptide salt is CNP, as described herein, the hydrophobic counterion is selected from the group consisting of oleate, pamoate, and docusate; and the polyvalent cation is Zn²⁺ or Ca²⁺. In various embodiments, the hydrophilic peptide salt is CNP, as described herein, the hydrophobic counterion is selected from the group consisting of deoxycholate, decanoate and docusate; and the polyvalent cation is Zn²⁺ or Ca²⁺. In various embodiments, the hydrophilic peptide salt is CNP, as described herein, the hydrophobic counterion is selected from the group consisting of oleate and pamoate; and the polyvalent cation is Zn²⁺ or Ca²⁺. In various embodiments, the hydrophilic peptide salt is CNP, as described herein, the hydrophobic counterion is oleate; and the polyvalent cation is Zn²⁺ or Ca²⁺. In various embodiments, the hydrophilic peptide salt is CNP, as described herein, the hydrophobic counterion is pamoate; and the polyvalent cation is Zn²⁺ or Ca²⁺.

In various embodiments, the peptide salt is selected from the group consisting of CNP-Ca⁺²(Oleate), CNP-Ca⁺²(Pamoate), CNP-Ca⁺²(deoxycholate), CNP-Ca⁺²(decanoate), CNP-Ca⁺²(docusate), CNP-Zn⁺²(Oleate), CNP-Zn⁺²(Pamoate), CNP-Zn⁺²(deoxycholate), CNP-Zn⁺²(decanoate), and CNP-Zn⁺²(docusate). In various embodiments, the peptide salt is selected from the group consisting of CNP-Ca⁺²(Oleate), CNP-Ca⁺²(Pamoate), CNP-Ca⁺²(docusate), CNP-Zn⁺²(Oleate), CNP-Zn⁺²(Pamoate), and CNP-Zn⁺²(docusate). In various embodiments, the peptide salt is selected from the group consisting of CNP-Ca⁺²(Oleate), CNP-Ca⁺²(Pamoate), and CNP-Ca⁺²(docusate). In various embodiments, the peptide salt is selected from the group consisting of CNP-Ca⁺²(deoxycholate), CNP-Ca⁺²(decanoate), and CNP-Ca⁺²(docusate). In various embodiments, the peptide salt is selected from the group consisting of CNP-Ca⁺²(Oleate) and CNP-Ca⁺²(Pamoate). In various embodiments, the peptide salt is CNP-Ca⁺²(Oleate). In various embodiments, the peptide salt is CNP-Ca⁺²(Pamoate). In various embodiments, the peptide salt is selected from the group consisting of CNP-Zn⁺²(Oleate), CNP-Zn⁺²(Pamoate), CNP-Zn⁺²(deoxycholate), CNP-Zn⁺²(decanoate), and CNP-Zn⁺²(docusate). In various embodiments, the peptide salt is selected from the group consisting of CNP-Zn⁺²(Oleate), CNP-Zn⁺²(Pamoate), and CNP-Zn⁺²(docusate). In various embodiments, the peptide salt is selected from the group consisting of CNP-Zn⁺²(deoxycholate), CNP-Zn⁺²(decanoate), and CNP-Zn⁺²(docusate). In various embodiments, the peptide salt is selected from the group consisting of CNP-Zn⁺²(Oleate) and CNP-Zn⁺²(Pamoate). In various embodiments, the peptide salt is CNP-Zn⁺²(Oleate). In various embodiments, the peptide salt is CNP-Zn⁺²(Pamoate).

In various embodiments, the peptide salt is selected from the group consisting of PG-CNP37-Ca⁺²(Oleate), PG-CNP37-Ca⁺²(Pamoate), PG-CNP37-Ca⁺²(deoxycholate), PG-CNP37-Ca⁺²(decanoate), PG-CNP37-Ca⁺²(docusate), PG-CNP37-Zn⁺²(Oleate), PG-CNP37-Zn⁺²(Pamoate), PG-CNP37-Zn⁺²(deoxycholate), PG-CNP37-Zn⁺²(decanoate), and PG-CNP37-Zn⁺²(docusate). In various embodiments, the peptide salt is selected from the group consisting of PG-CNP37-Ca⁺²(Oleate), PG-CNP37-Ca⁺²(Pamoate), PG-CNP37-Ca⁺²(docusate), PG-CNP37-Zn⁺²(Oleate), PG-CNP37-Zn⁺²(Pamoate), and CNP-Zn⁺²(docusate). In various embodiments, the peptide salt is selected from the group consisting of PG-CNP37-Ca⁺²(Oleate), PG-CNP37-Ca⁺²(Pamoate), and PG-CNP37-Ca⁺²(docusate). In various embodiments, the peptide salt is selected from the group consisting of CNP-Ca⁺²(deoxycholate), PG-CNP37-Ca⁺²(decanoate), and PG-CNP37-Ca⁺²(docusate). In various embodiments, the peptide salt is selected from the group consisting of PG-CNP37-Ca⁺²(Oleate) and PG-CNP37-Ca⁺²(Pamoate). In various embodiments, the peptide salt is PG-CNP37-Ca⁺²(Oleate). In various embodiments, the peptide salt is PG-CNP37-Ca⁺²(Pamoate). In various embodiments, the peptide salt is selected from the group consisting of PG-CNP37-Zn⁺²(Oleate), PG-CNP37-Zn⁺²(Pamoate), PG-CNP37-Zn⁺²(deoxycholate), PG-CNP37-Zn⁺²(decanoate), and PG-CNP37-Zn⁺²(docusate). In various embodiments, the peptide salt is selected from the group consisting of PG-CNP37-Zn⁺²(Oleate), PG-CNP37-Zn⁺²(Pamoate), and PG-CNP37-Zn⁺²(docusate). In various embodiments, the peptide salt is selected from the group consisting of PG-CNP37-Zn⁺²(deoxycholate), PG-CNP37-Zn⁺²(decanoate), and PG-CNP37-Zn⁺²(docusate). In various embodiments, the peptide salt is selected from the group consisting of PG-CNP37-Zn⁺²(Oleate) and PG-CNP37-Zn⁺²(Pamoate). In various embodiments, the peptide salt is PG-CNP37-Zn⁺²(Oleate). In various embodiments, the peptide salt is PG-CNP37-Zn⁺²(Pamoate).

### Methods of Making (not part of the presently claimed invention)

Contemplated herein is also a method of making a composition comprising a hydrophobic peptide salt as described herein.

In various embodiments, the disclosure provides a method of making a composition comprising a salt of an electrostatically charged peptide comprising, a) contacting an electrostatically charged peptide in an aqueous solution with a hydrophobic counterion in solution; b) mixing the electrostatically charged peptide solution with the hydrophobic counterion solution in a manner sufficient for the peptide and counterion to form a complex, wherein the formation of the peptide-counterion complex results in formation of a solid, semi-solid, gel, crystalline, amorphous, nanoparticle, microparticle, amorphous nanoparticle, amorphous microparticle, crystalline nanoparticle or crystalline microparticle form comprising the peptide salt. In various embodiments, when the peptide-counterion salt further comprises a polyvalent cation. the method comprises before step (b), contacting the electrostatically charged peptide in solution with a polyvalent cation in aqueous solution, forming a peptide-cation complex. The peptide-cation complex is then contacted with a hydrophobic counterion to form a peptide-cation-counterion complex.

In various embodiments, the disclosure provides a method of making a composition comprising a salt of an electrostatically charged peptide comprising (a), contacting the electrostatically charged peptide in solution with a polyvalent cation in aqueous solution, forming a peptide-cation complex, b) contacting the peptide-cation complex in an aqueous solution with a hydrophobic counterion in solution; and c) mixing the peptide-cation complex solution with the hydrophobic counterion solution in a manner sufficient for the peptide-cation and counterion to form a complex, wherein the formation of the peptide-cation counterion complex results in formation of a solid, semi-solid, gel, crystalline, amorphous, nanoparticle, microparticle, amorphous nanoparticle, amorphous microparticle, crystalline nanoparticle or crystalline microparticle form comprising the peptide salt.

In various embodiments, the mixing is carried out by dropwise addition of the hydrophobic counterion solution to the peptide solution. The solutions are mixed by vortexing after addition of each drop of hydrophobic counterion solution, or other means for mixing known in the art.

In various embodiments, the method further comprises step (c) or (d) washing the peptide salt in buffer or water. In various embodiments, the washing is carried out in an aqueous solution, e.g., buffer or water.

In various embodiments, the method further comprises step (d) or (e) obtaining the peptide salt by centrifugation to form a peptide salt pellet. In various embodiments, if the salt is in gel form, the salt is obtained by centrifugation or by decanting the liquid phase followed by lyophilization or other drying methods.

In various embodiments, the method further comprises step (e) or (f) removing water from the peptide salt pellet. It is contemplated that water or another aqueous solution can be removed from the pellet by lyophilization or drying using techniques known in the art.

In various embodiments, the method further comprises resuspending the pellet in an aqueous solution or oil. In various embodiments, the aqueous solution is water, saline, or buffer. In various embodiments, the oil comprises a triglyceride or a fatty acid. In various embodiments, the fatty acid is saturated or unsaturated. In various embodiments, the fatty acid in the triglyceride is saturated or unsaturated, or combinations thereof.

The fatty acid may be the oil itself or in a triglyceride. In various embodiments, the fatty acid is a short chain, medium chain or long chain fatty acid. In various embodiments, the fatty acid in a triglyceride is saturated or unsaturated, and can be a medium chain or long chain fatty acid. In various embodiments, the fatty acid is a C-6 to C-20 fatty acid. In various embodiments, the fatty acid is a C-6, C-8, C-10, C-12, C-14, C-16, C-18 or C-20 fatty acid. In various embodiments, the fatty acid is hexanoic acid, octanoic acid, decanoic acid, or dodecanoic acid.

In various embodiments, the method contemplates use of at least one molar equivalent of hydrophobic counterion, such as a hydrophobic anion, to: 1) the total number of charged amino acids if cation is present; or 2) the total number of positive charges if no cation is present. Accordingly, in various embodiments, the method contemplates the use of at least one equivalent of hydrophobic counterion to the total number of positive charges in the hydrophilic peptide when no cation is present, or to the total number of positive charges in the complex comprising hydrophilic peptide and cation when the polyvalent cation is present. This ratio is referred to herein as the peptide: hydrophobic counterion ratio. In various embodiments, the peptide: hydrophobic counterion ratio is at least one molar equivalent of hydrophobic counterion to total number of positively charged amino acids in the peptide (when no cation is present) or the complex comprising peptide and cation (when cation is present). In various embodiments the peptide: hydrophobic counterion ratio is between 1:1 to 1:20, or from 1:1 to 1:50. The peptide: counterion ratio can be between 1:2 to 1:15, 1:2 to 1:10, 1:2 to 1:8, 1:3 to 1:10, or 1:4 to 1:10. In various embodiments, the peptide: hydrophobic counterion ratio is 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19 or 1:20. In various embodiments, the peptide: hydrophobic counterion ratio is 1:1. In various embodiments, the peptide: hydrophobic counterion ratio is 1:2. In various embodiments, the peptide: hydrophobic counterion ratio is 1:3. In various embodiments, the peptide: hydrophobic counterion ratio is 1:4. In various embodiments, the peptide: hydrophobic counterion ratio is 1:5. In various embodiments, the peptide: hydrophobic counterion ratio is 1:6. In various embodiments, the peptide: hydrophobic counterion ratio is 1:7. In various embodiments, the peptide: hydrophobic counterion ratio is 1:8. In various embodiments, the peptide: hydrophobic counterion ratio is 1:9. In various embodiments, the peptide: hydrophobic counterion ratio is 1:10. In various embodiments, the peptide: hydrophobic counterion ratio is at least two molar equivalents of hydrophobic counterion to total number of positively charged amino acids in the peptide (when no cation is present) or the complex comprising the peptide and cation (when cation is present). In various embodiments, the peptide: hydrophobic counterion ratio is at least three molar equivalents of hydrophobic counterion to total number of positively charged amino acids in the peptide (when no cation is present) or the complex comprising the peptide and cation (when cation is present). In various embodiments, the peptide: hydrophobic counterion ratio is at least four molar equivalents of hydrophobic counterion to total number of positively charged amino acids in the peptide (when no cation is present) or the complex comprising the peptide and cation (when cation is present). In some embodiments, the peptide: hydrophobic counterion ratio is less than one molar equivalent of hydrophobic counterion to total number of positive charges in the peptide (when no cation is present) or to the complex comprising the peptide and cation (when cation is present). In various embodiments, the peptide salt has one less molar equivalent of hydrophobic counterion to positive charges in the peptide (when no cation is present) or the complex comprising the peptide and cation (when cation is present). In various embodiments, the peptide salt has two less molar equivalents of hydrophobic counterion to positive charges in the peptide (when no cation is present) or the complex comprising the peptide and cation (when cation is present). In various embodiments, the peptide salt has three less molar equivalents of hydrophobic counterion to positive charges in the peptide (when no cation is present) or the complex comprising the peptide and cation (when cation is present). In various embodiments, the method contemplates introducing, e.g., 0.9, 0.8, 0.7, 0.75, 0.6, 0.5, 0.4, 0.3, 0.25, 0.2 or 0.1 molar equivalents of hydrophobic counterion to the total number of positive charges on the peptide (when no cation is present) or the complex comprising the peptide and cation (when cation is present).

In various embodiments, the method contemplates including at least one molar equivalent of cation to total number of negatively charged amino acids in the peptide. This ratio is referred to herein as the peptide: cation ratio. In various embodiments, the peptide: cation ratio is between 1:1 to 1:10. The peptide: cation ratio can be 1:2 to 1:10, 1:3 to 1:10, 1:1 to 1:5, 1:2 to 1:5, of 1:2 to 1:8. In various embodiments, the peptide: cation ratio is 1:1, 1:2, 1:3, 1:4, 1:5, 1:6, 1:7, 1:8, 1:9, or 1:10. In various embodiments, the peptide: cation ratio is 1:1. In various embodiments, the peptide: cation ratio is 1:2. In various embodiments, the peptide: cation ratio is 1:3. In various embodiments, the peptide: cation ratio is 1:4. In various embodiments, the peptide: cation ratio is 1:5. In various embodiments, the peptide: cation ratio is 1:6. In various embodiments, the peptide: cation ratio is 1:7. In various embodiments, the peptide: cation ratio is 1:8. In various embodiments, the peptide: cation ratio is 1:9. In various embodiments, the peptide: cation ratio is 1:10. In various embodiments, the method contemplates including less than one molar equivalent of cation to total number of negatively charged amino acids in the peptide. In various embodiments, the method contemplates introducing, e.g., 0.9, 0.8, 0.7, 0.75, 0.6, 0.5, 0.4, 0.3, 0.25, 0.2 or 0.1 molar equivalents of cation to the total number of negative charges on the peptide.

### C-type natriuretic peptides

C-type natriuretic peptide (CNP) (Biochem. Biophys. Res. Commun., 168: 863-870 (1990) (GenBank Accession No. NP_077720, for the CNP precursor protein, NPPC) (J. Hypertens., 10: 907-912 (1992)) is a small, single chain peptide in a family of peptides (ANP, BNP, CNP) having a 17-amino acid loop structure (Levin et al., N. Engl. J. Med., 339: 863-870 (1998)) and have important roles in multiple biological processes. CNP interacts with natriuretic peptide receptor-B (NPR-B, GC-B) to stimulate the generation of cyclic- guanosine monophosphate (cGMP) (J. Hypertens., 10: 1111-1114 (1992)). CNP is expressed more widely, including in the central nervous system, reproductive tract, bone and endothelium of blood vessels (Hypertension, 49: 419-426 (2007)).

In humans, CNP is initially produced from the natriuretic peptide precursor C (NPPC) gene as a single chain 126-amino acid pre-pro polypeptide (Biochem. Biophys. Res. Commun., 168: 863-870 (1990)). Removal of the signal peptide yields pro-CNP, and further cleavage by the endoprotease furin generates an active 53-amino acid peptide (CNP-53), which is secreted and cleaved again by an unknown enzyme to produce the mature 22-amino acid peptide (CNP-22) (Wu, J. Biol. Chem. 278: 25847-852 (2003)). CNP-53 and CNP-22 differ in their distribution, with CNP-53 predominating in tissues, while CNP-22 is mainly found in plasma and cerebrospinal fluid (J. Alfonzo, Recept. Signal. Transduct. Res., 26: 269-297 (2006)). Both CNP-53 and CNP-22 bind similarly to NPR-B.

In various embodiments, CNP of the disclosure include truncated CNP ranging from human CNP-17 (hCNP-17) to human CNP-53 (hCNP-53), and having wild-type amino acid sequences derived from hCNP-53. Such truncated CNP peptides include:
DLRVDTKSRAAWARLLQEHPNARKYKGANKKGLSKGCFGLKLDRIGSMSGLGC (CNP-53) (SEQ ID NO: 56);
LRVDTKSRAAWARLLQEHPNARKYKGANKKGLSKGCFGLKLDRIGSMSGLGC (CNP-52) (SEQ ID NO:15);
RVDTKSRAAWARLLQEHPNARKYKGANKKGLSKGCFGLKLDRIGSMSGLGC (CNP-51) (SEQ ID NO:16);
VDTKSRAAWARLLQEHPNARKYKGANKKGLSKGCFGLKLDRIGSMSGLGC (CNP-50) (SEQ ID NO17:);
DTKSRAAWARLLQEHPNARKYKGANKKGLSKGCFGLKLDRIGSMSGLGC (CNP-49) (SEQ ID NO: 18)
TKSRAAWARLLQEHPNARKYKGANKKGLSKGCFGLKLDRIGSMSGLGC (CNP-48) (SEQ ID NO:19);
KSRAAWARLLQEHPNARKYKGANKKGLSKGCFGLKLDRIGSMSGLGC (CNP-47) (SEQ ID NO:20);
SRAAWARLLQEHPNARKYKGANKKGLSKGCFGLKLDRIGSMSGLGC (CNP-46) (SEQ ID NO:21);
RAAWARLLQEHPNARKYKGANKKGLSKGCFGLKLDRIGSMSGLGC (CNP-45) (SEQ ID NO:22);
AAWARLLQEHPNARKYKGANKKGLSKGCFGLKLDRIGSMSGLGC (CNP-44) (SEQ ID NO:23);
AWARLLQEHPNARKYKGANKKGLSKGCFGLKLDRIGSMSGLGC (CNP-43) (SEQ ID NO:24);
WARLLQEHPNARKYKGANKKGLSKGCFGLKLDRIGSMSGLGC (CNP-42) (SEQ ID NO:25);
ARLLQEHPNARKYKGANKKGLSKGCFGLKLDRIGSMSGLGC (CNP-41) (SEQ ID NO:26);
RLLQEHPNARKYKGANKKGLSKGCFGLKLDRIGSMSGLGC (CNP-40) (SEQ ID NO:27);
LLQEHPNARKYKGANKKGLSKGCFGLKLDRIGSMSGLGC (CNP-39) (SEQ ID NO:28);
LQEHPNARKYKGANKKGLSKGCFGLKLDRIGSMSGLGC (CNP-38) (SEQ ID NO:2);
QEHPNARKYKGANKKGLSKGCFGLKLDRIGSMSGLGC (CNP-37) (SEQ ID NO:3);
EHPNARKYKGANKKGLSKGCFGLKLDRIGSMSGLGC (CNP-36) (SEQ ID NO:29);
HPNARKYKGANKKGLSKGCFGLKLDRIGSMSGLGC (CNP-35) (SEQ ID NO:30);
PNARKYKGANKKGLSKGCFGLKLDRIGSMSGLGC (CNP-34) (SEQ ID NO:4);
NARKYKGANKKGLSKGCFGLKLDRIGSMSGLGC (CNP-33) (SEQ ID NO:31);
ARKYKGANKKGLSKGCFGLKLDRIGSMSGLGC (CNP-32) (SEQ ID NO:32);
RKYKGANKKGLSKGCFGLKLDRIGSMSGLGC (CNP-31) (SEQ ID NO:33);
KYKGANKKGLSKGCFGLKLDRIGSMSGLGC (CNP-30) (SEQ ID NO:34);
YKGANKKGLSKGCFGLKLDRIGSMSGLGC (CNP-29) (SEQ ID NO:35);
KGANKKGLSKGCFGLKLDRIGSMSGLGC (CNP-28) (SEQ ID NO:36);
GANKKGLSKGCFGLKLDRIGSMSGLGC (CNP-27) (SEQ ID NO:37);
ANKKGLSKGCFGLKLDRIGSMSGLGC (CNP-26) (SEQ ID NO:38);
NKKGLSKGCFGLKLDRIGSMSGLGC (CNP-25) (SEQ ID NO:39);
KKGLSKGCFGLKLDRIGSMSGLGC (CNP-24) (SEQ ID NO:40);
KGLSKGCFGLKLDRIGSMSGLGC (CNP-23) (SEQ ID NO:41);
GLSKGCFGLKLDRIGSMSGLGC (CNP-22) (SEQ ID NO: 68);
LSKGCFGLKLDRIGSMSGLGC (CNP-21) (SEQ ID NO:42);
SKGCFGLKLDRIGSMSGLGC (CNP-20) (SEQ ID NO:43);
KGCFGLKLDRIGSMSGLGC (CNP-19) (SEQ ID NO:44);
GCFGLKLDRIGSMSGLGC (CNP-18) (SEQ ID NO:45); and
CFGLKLDRIGSMSGLGC (CNP-17) (SEQ ID NO: 67).

In various embodiments, the CNP variant peptides are modified CNP-37 or CNP-38 peptides, optionally having mutation(s)/substitution(s) at the furin cleavage site (underlined), and/or containing glycine or proline-glycine at the N-terminus. Exemplary CNP-37 variants include:
QEHPNARKYKGANKKGLSKGCFGLKLDRIGSNSGLGC [CNP-37(M32N); SEQ ID NO: 46];
MQEHPNARKYKGANKKGLSKGCFGLKLDRIGSMSGLGC (Met-CNP-37; SEQ ID NO:47);
PQEHPNARKYKGANKKGLSKGCFGLKLDRIGSMSGLGC (Pro-CNP-37; SEQ ID NO: 48);
GQEHPNARKYKGANKKGLSKGCFGLKLDRIGSNSGLGC [Gly-CNP-37 (M32N); SEQ ID NO: 49];
PGQEHPNARKYKGANKKGLSKGCFGLKLDRIGSMSGLGC (Pro-Gly-CNP-37; SEQ ID NO: 1);
MGQEHPNARKYKGANKKGLSKGCFGLKLDRIGSMSGLGC (Met-Gly-CNP-37; SEQ ID NO: 50); and
GQEHPNARKYKGANKKGLSKGCFGLKLDRIGSMSGLGC (Gly-CNP-37: SEQ ID NO:51)
GQEHPNARKYKGANPKGLSKGCFGLKLDRIGSMSGLGC (SEQ ID NO:52);
GQEHPNARKYKGANQKGLSKGCFGLKLDRIGSMSGLGC (SEQ ID NO:53);
GQEHPNARKYKGANQQGLSKGCFGLKLDRIGSMSGLGC (SEQ ID NO:54); and
GQEHPNARKYKGANKPGLSKGCFGLKLDRIGSMSGLGC (SEQ ID NO:55).

In various embodiments, the CNP is selected from the group consisting of
PGQEHPQARRYRGAQRRGLSRGCFGLKLDRIGSMSGLGC(SEQ ID NO: 5);
PGQEHPNARKYKGANKKGLSKGCFGLKLDRIGSMSGLGC(SEQ ID NO: 1);
PGQEHPNARRYRGANRRGLSRGCFGLKLDRIGSMSGLGC(SEQ ID NO: 6);
PGQEHPQARRYRGAQRRGLSRGCFGLKLDRIGSMSGLGC(SEQ ID NO: 5); and
PGQEHPQARKYKGAQKKGLSKGCFGLKLDRIGSMSGLGC(SEQ ID NO: 7).

In various disclosures, but not part of the presently claimed invention, the CNP variant peptide further comprises an acetyl group. In various embodiments, the acetyl group is on the N-terminus of the peptide. In various embodiments, the acetyl group is on an amino acid side chain within the peptide sequence. In various embodiments, the peptide further comprises an OH or an NH₂ group at the C-terminus.

In additional disclosures, for any of the CNP and CNP variants described herein that have asparagine (Asn/N) residue(s) and/or glutamine (Gln/Q) residue(s), whether they have a wild-type sequence or a non-natural amino acid sequence, any Asn residue(s) and/or any Gln residue(s) can independently be substituted with any other natural or unnatural amino acids, including conservative substitutions such as Asn to Gln. Such substitution(s) are designed in part to minimize or avoid any potential deamidation of asparagine and/or glutamine.

Additional CNP peptides and variants are disclosed in U.S. Patent 8,198,242.

In various embodiments, the CNP is selected from the group consisting of:
PGQEHPNARKYKGANKKGLSKGCFGLKLDRIGSMSGLGC (Pro-Gly-CNP-37; SEQ ID NO: 1)
LQEHPNARKYKGANKKGLSKGCFGLKLDRIGSMSGLGC (CNP-38) (SEQ ID NO:2);
QEHPNARKYKGANKKGLSKGCFGLKLDRIGSMSGLGC (CNP-37) (SEQ ID NO:3);
PNARKYKGANKKGLSKGCFGLKLDRIGSMSGLGC (CNP-34) (SEQ ID NO:4);
and, pharmaceutical salts thereof. In various disclosures, the CNP is a CNP-acetate.

In various embodiments, the hydrophobic counterion is oleate, deoxycholate, decanoate, pamoate, docusate or dodecyl sulfate. In various embodiments, if a polyvalent cation is present, the cation comprises zinc, magensium or calcium. In various embodiments, if the cation is present, the cation comprises zinc. In various embodiments, the cation comprises calcium. In various embodiments, if a polyvalent cation is present, the cation is Zn²⁺, Mg²⁺, or Ca²⁺. In various embodiments, if a cation is present, the cation is Zn²⁺. In various embodiments, if a cation is present, the cation is Ca²⁺.

In various embodiments, the disclosure provides a hydrophobic salt of C-type natriuretic peptide comprising a CNP peptide in complex with a hydrophobic counterion. In various embodiments, the salt further comprises a polyvalent cation, optionally a metal cation. In various embodiments, the salt is a purified CNP salt.

Methods of purifying hydrophobic salts are known in the art and contemplated herein. In various embodiments, the hydrophobic salt has a purity of at least about 80%, 85%, 90%, 91%, 92%, 93%, 94%, 95%, 96%, 97%, 98%, 99%, 99.5% or more.

### Methods of Use

Achondroplasia is a result of an autosomal dominant mutation in the gene for fibroblast growth factor receptor 3 (FGFR-3), which causes an abnormality of cartilage formation. FGFR-3 normally has a negative regulatory effect on chondrocyte growth, and hence bone growth. In achondroplasia, the mutated form of FGFR-3 is constitutively active, which leads to severely shortened bones. In humans activating mutations of FGFR-3 are the primary cause of genetic dwarfism. Mice having activated FGFR-3 serve as a model of achondroplasia, the most common form of the skeletal dysplasias, and overexpression of CNP rescues these animals from dwarfism. Accordingly, CNP and functional variants of CNP are potential therapeutics for treatment of the various skeletal dysplasias

By stimulating matrix production, proliferation and differentiation of chondrocytes and increasing long bone growth, the CNP salts of the disclosure are useful for treating mammals, including humans, suffering from a bone-related disorder, such as a skeletal dysplasia. Non-limiting examples of CNP-responsive bone-related disorders and skeletal dysplasias include achondroplasia, hypochondroplasia, short stature, dwarfism, osteochondrodysplasias, thanatophoric dysplasia, osteogenesis congenita, achondrogenesis, chondrodysplasia congenit, homozygous achondroplasia, chondrodysplasia congenit, camptomelic dysplasia, congenital lethal hypophosphatasia, perinatal lethal type of osteogenesis congenita, short-rib polydactyly syndromes, hypochondroplasia, rhizomelic type of chondrodysplasia congenit, Jansen-type metaphyseal dysplasia, spondyloepiphyseal dysplasia congenital, atelosteogenesis, diastrophic dysplasia, congenital short femur, Langer-type mesomelic dysplasia, Nievergelt-type mesomelic dysplasia, Robinow syndrome, Reinhardt syndrome, acrodysostosis, peripheral dysostosis, Kniest dysplasia, fibrochondrogenesis, Roberts syndrome, acromesomelic dysplasia, micromelia, Morquio syndrome, Kniest syndrome, metatrophic dysplasia, spondyloepimetaphyseal dysplasia, NPR2 mutation, SHOX mutation (Turner's syndrome/Leri Weill), and PTPN11 mutations (Noonan's syndrome).

By stimulating matrix production, proliferation and differentiation of chondrocytes and increasing long bone growth, the CNP variants of the disclosure are useful for treating mammals, including humans, suffering from a bone-related disorder, such as a skeletal dysplasia. Non-limiting examples of CNP-responsive bone-related disorders and skeletal dysplasias include achondroplasia, hypochondroplasia, short stature, dwarfism, osteochondrodysplasias, thanatophoric dysplasia, osteogenesis congenita, achondrogenesis, chondrodysplasia congenit, homozygous achondroplasia, chondrodysplasia congenit, camptomelic dysplasia, congenital lethal hypophosphatasia, perinatal lethal type of osteogenesis congenita, short-rib polydactyly syndromes, hypochondroplasia, rhizomelic type of chondrodysplasia congenit, Jansen-type metaphyseal dysplasia, spondyloepiphyseal dysplasia congenital, atelosteogenesis, diastrophic dysplasia, congenital short femur, Langer-type mesomelic dysplasia, Nievergelt-type mesomelic dysplasia, Robinow syndrome, Reinhardt syndrome, acrodysostosis, peripheral dysostosis, Kniest dysplasia, fibrochondrogenesis, Roberts syndrome, acromesomelic dysplasia, micromelia, Morquio syndrome, Kniest syndrome, metatrophic dysplasia, and spondyloepimetaphyseal dysplasia. Short stature, growth plate disorder, bone-related disorder or skeletal dysplasias contemplated herein include disorders related to NPR2 mutation, SHOX mutation (Turner's syndrome/Leri Weill), PTPN11 mutations (Noonan's syndrome) and IGF1R mutation.

Short stature, growth plate disorder, bone-related disorder or skeletal dysplasias contemplated herein include disorders related to NPR2 mutation, SHOX mutation (Turner's syndrome/Leri Weill), and PTPN11 mutations (Noonan's syndrome).

Additional short stature and growth plate disorders contemplated by the methods include disorders related to mutations in collagen (COL2A1, COL11A1, COL9A2, COL10), aggrecan (ACAN), indian hedgehog (IHH), PTPN11, NPR2, NPPC, or FGFR3.

Additional short stature and growth plate disorders contemplated by the methods include disorders related to mutations in collagen (COL2A1, COL11A1, COL9A2, COL10), aggrecan (ACAN), indian hedgehog (IHH), PTPN11, NPR2, NPPC, FGFR3, or IGF1R.

Further, the CNP salts are useful as an adjunct or alternative to growth hormone for treating idiopathic short stature and other skeletal dysplasias described herein.

Growth plate disorders include disorders that result in short stature or abnormal bone growth and that may be the result of a genetic mutation in a gene involved in bone growth, including collagen (COL2A1, COL11A1, COL9A2, COL10), aggrecan (ACAN), indian hedgehog (IHH), PTPN11, NPR2, NPPC, or FGFR3. In various embodiments, growth plate disorders include disorders that result in short stature or abnormal bone growth and that may be the result of a genetic mutation in a gene involved in bone growth, including collagen (COL2A1, COL11A1, COL9A2, COL10), aggrecan (ACAN), indian hedgehog (IHH), PTPN11, NPR2, NPPC, FGFR3, or IGF1R. In various embodiments, the growth plate disorder or short stature is associated with one or more mutations in a gene associated with a RASopathy. In various embodiments, a subject with a growth plate disorder is heterozygous for a mutation in a growth plate gene. In various embodiments, the mutation is a loss-of-function mutation. In various embodiments, the mutation is a gain-of-function mutation. Growth plate disorders include, but are not limited to, familial short stature, dominant familial short stature which is also known as dominant inherited short stature, or idiopathic short stature. See, e.g., Plachy et al., J Clin Endocrinol Metab 104: 4273-4281, 2019.

Mutations in ACAN can give rise to familial osteochondritis dissecans and short stature and eventually osteoarthritis, characterized by areas of bone damage (or lesions) caused by the detachment of cartilage and sometimes bone from the end of the bone at a joint. It has been suggested that the disorganized cartilage network in growing bones impairs their growth, leading to short stature. A mutation associated with ACAN and short stature includes Val2303Met. See Stattin et al., Am J Hum Genet 86(2):126-37, 2010. It is contemplated that patients with a mutation in ACAN resulting in short stature would benefit from treatment with CNP as administration may be able to increase height in these patients by the known interaction of CNP with FGFR3.

The natriuretic peptide system, including receptor NPR2, has been shown to be involved in regulation of endochondral bone growth (Vasques et al., Horm Res Pediat 82:222-229, 2014). Studies have shown that homozygous or compound heterozygous loss-of-function mutations in NPR2 cause acromesomelic dysplasia type Maroteaux (AMDM), which is a skeletal dysplasia having extremely short stature (Vasquez et al., 2014, *supra*)*.* There are reports implicating heterozygous loss-of-function (such as dominant negative) NPR2 mutations as a cause of short stature, whereas gain-of-function NPR2 heterozygous mutations have been found to be responsible for tall stature (Vasquez et al., 2014, *supra*)*.* In view of CNP's interaction with NPR2 to stimulate cGMP generation, increasing cGMP levels is desirable in these conditions and would have therapeutic benefit in the management of the complications from these diseases and conditions.

Heterozygous mutations of NPR2 are believed to result in idiopathic short stature and other forms of short stature. Mutations in the NPR2 gene are set out below and described in Amano et al., J Clin Endocrinol Metab 99:E713-718, 2014, Hisado-Oliva et al., J Clin Endocrinol Metab 100:E1133-1142, 2015 and Vasques et al., J Clin Endocrinol Metab 98:E1636-1644, 2013. It is contemplated that a subject having short stature to be treated with a CNP variant as described herein has a height SDS of less than -1.0, -1.5, -2.0, -2.5, or -3.0, and has at least one parent with a height SDS of less than -1.0, -1.5, -2.0 or -2.5, optionally wherein the second parent has height within the normal range. In various embodiments, the CNP variants are useful to treat a subject with short stature having a height SDS of between -2.0 to -3.0. In various embodiments, the CNP variants are useful to treat a subject with short stature having a height SDS of between -2.0 to -2.5. However, because *de novo* mutations in NPR2 can result in short stature as defined by a height SDS of less than -1.5, -2.0, -2.5, or -3.0, treatment of individuals who are heterozygous carriers of a deleterious mutation in NPR2 with neither parent having short stature is also contemplated. Further contemplated is treatment of individuals who are heterozygous for deleterious mutations in other growth plate genes with CNP to improve stature and/or enhance bone growth.

Exemplary NPR2 mutations in patients that may be treated with a CNP variant include:

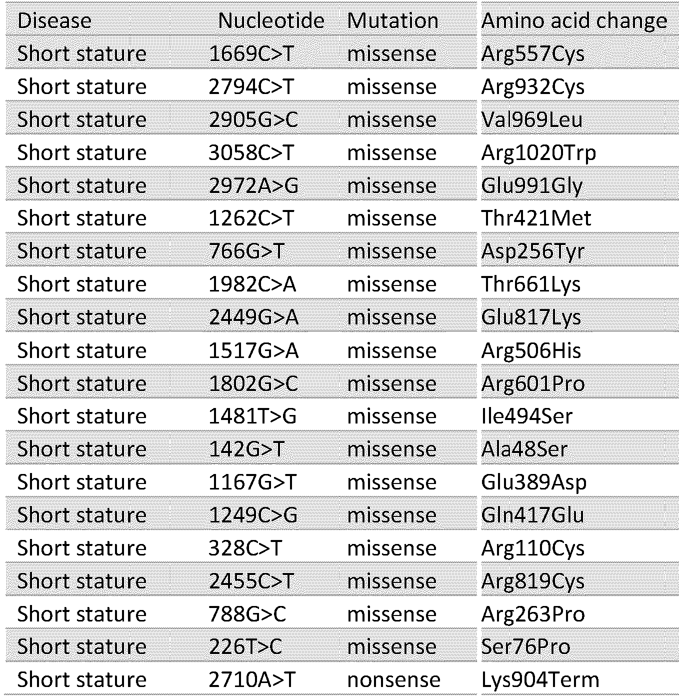

| | |
|---|---|
| 9:35809194:C:G | Leul009Val |
| 9:35802761:G:C | Leu615Phe |
| 9:35799645:C:T | Pro301Ser |
| 9:35792928:C:T | Arg174Cys |
| 9:35801728:C:G | His508Asp |
| 9:35792713:T:C | Val102Ala |
| 9:35793980:T:A | Tyr250Ter |
| 9:35807085:C:T | Thr861Ile |
| 9:35793906:A:G | Ile226Val |
| 9:35808558:G:A | Arg921Gln |
| 9:35802741:G:A | Glu609Lys |
| 9:35802594:G:A | Arg601His |
| 9:35808663:T:A | Leu956Gln |
| 9:35808545:G:C | Gly917Arg |

NPPC's role in skeletal growth is well documented. (Hisado-Oliva et al., Genetics Medicine 20:91-97, 2018). The NPPC knock out mouse showed severe disproportionate form of dwarfism including shortening of limbs and endochondral ossification (Hisado-Oliva et al., 2018*, supra*)*.* Human genome wide studies have shown a link between NPPC and height (Hisado-Oliva et al., 2018, *supra*)*.* Although CNP haploinsufficiency has been believed to be a cause of short stature in humans, a recent study identified heterozygous mutations in families with short stature and hands (Hisado-Oliva et al., 2018, *supra*)*.* These studies observed significant reduction in cGMP production as measured in heterozygous state (Hisado-Oliva et al., 2018, *supra*)*.* Mutations in NPPC include a 355G>T missense mutation causing a Gly119Cys change and a 349C>G missense mutation causing a Arg117Gly change. A CNP variant rescuing CGMP production may provide therapeutic benefit in the management of a disorder in patients having heterozygous loss-of-function NPPC mutations.

Leri-Weill dyschondrosteosis (LWD) is a rare genetic disorder characterized by shortening of the forearms and lower legs, abnormal misalignment of the wrist (Madelung deformity of the wrist), and associated short stature. LWD is caused by a heterozygous mutation in the short stature homeobox-containing (SHOX) gene or its regulatory elements located on the pseudoautosomal region 1 (PAR1) of the sex chromosomes. (See the Rare Disease Database and Carmona et al., Hum Mol Genet 20:1547-1559, 2011). The disorder Langer mesomelic dysplasia arises when there are two SHOX mutations, and may result from a mutation on each chromosome, either a homozygous or compound heterozygous mutations. A subset of SHOX mutations give rise to idiopathic short stature. Turner syndrome results due to a deletion on the X chromosome that can include the SHOX gene. SHOX has been identified as involved in the regulation of *FGFR3* transcription and contributes to control of bone growth (Marchini et al., Endocr Rev. 37: 417-448, 2016). SHOX deficiency leads to increased FGFR3 signaling, and there is some evidence to support that SHOX has direct interactions with CNP/NPR2 as well (Marchini, *supra*)*.* Given the association of SHOX with FGFR3 and bone growth, it is contemplated that a subject having a homozygous or heterozygous SHOX mutation would benefit from treatment with CNP variants as described herein.

RASopathies are a group of rare genetic conditions caused by mutations in genes of the Ras/mitogen-activated protein kinase (MAPK) pathway. RASopathies are a group of disorders characterized by increased signaling through RAS/MAPK pathway. This pathway leads to downstream activation of the RAF/MEK/ERK pathway. Short stature is a characteristic feature of certain RASopathies. For example, CNP signaling inhibits RAF and leads to decreased MEK and ERK activation.

Treatment of RASopathies are contemplated herein. RASopathies associated with short stature include Noonan syndrome, Costello syndrome, Cardiofaciocutaneous syndrome, Neurofibromatosis Type 1, and LEOPARD syndrome. Hereditary gingival fibromatosis type 1 is also a RASopathy contemplated herein. RASopathy patients (including Noonan syndrome, Costello syndrome, Cardiofaciocutaneous syndrome, Neurofibromatosis Type 1, LEOPARD syndrome, hereditary gingival fibromatosis type 1) include patients with heterozygous variants in one or more of the following genes: BRAF, CBL, HRAS, KRAS, LZTR1, MAP2K1, MAP2K2, MRAS, NF1, NRAS, PPP1CB, PTPN11, RAF1, RRAS, RIT1, SHOC2, SOS1, or SOS2 (Tajan et al. Endocr. Rev. 2018;39(5):676-700).

CFC is caused by mutations in several genes in the Ras/MAPK signaling pathway, including K-Ras, B-Raf, Mek1 and Mek2. Costello syndrome, also called faciocutaneoskeletal (FCS) syndrome is caused by activating mutations in the H-Ras gene. Hereditary gingival fibromatosis type I (HGF) is caused by dominant mutations in the SOS1 gene (*Son of Sevenless* homolog 1), which encodes a guanine nucleotide exchange factor (SOS) that acts on the Ras subfamily of small GTPases. Neurofibromatosis type I (NF1) is caused by mutations in the neurofibromin 1 gene, which encodes a negative regulator of the Ras/MAPK signaling pathway. Noonan syndrome (NS) is caused by mutations in one of several genes, including PTPN11, which encodes SHP2, and SOS1, as well as K-Ras and Raf-1.

CNP has been demonstrated to be an effective therapy in RASopathy models. Ono et al. generated mice deficient in *Nf1* in type II collagen producing cells (Ono et al., Hum. Mol. Genet. 2013;22(15):3048-62). These mice demonstrated constitutive ERK1/2 activation, and decreased chondrocyte proliferation, and maturation. Daily injections of CNP in these mice led to decreased ERK phosphorylation and corrected the short stature. A mouse model of Cardiofaciocutaneous syndrome using a *Braf* mutation (p.Q241R) (Inoue et al. Hum. Mol. Genet. 2019;28(1):74-83) exhibited decreased body length and reduced growth plate width with smaller proliferative and hypertrophic zones compared to wild type, and CNP administration led to increases in body length in these animals.

Mutations in multiple genes can cause Noonan syndrome, which is characterized by short stature, heart defects, bleeding problems, and skeletal malformations. Mutations in the PTPN11 gene cause about half of all cases of Noonan's syndrome. SOS1 gene mutations cause an additional 10 to 15 percent, and RAF1 and RIT1 genes each account for about 5 percent of cases. Mutations in other genes each account for a small number of cases. The cause of Noonan syndrome in 15 to 20 percent of people with this disorder is unknown.

The PTPN11, SOS1, RAF1, and RIT1 genes all encode for proteins that are important in the RAS/MAPK cell signaling pathway, which is needed for cell division and growth (proliferation), differentiation, and cell migration. Many of the mutations in the genes associated with Noonan syndrome cause the resulting protein to be turned on (active) and this prolonged activation alters normal RAS/MAPK signaling, which disrupts the regulation of cell growth and division, leading to the characteristic features of Noonan syndrome. See, e.g., Chen et al., Proc Natl Acad Sci U S A. 111(31):11473-8, 2014, Romano et al., Pediatrics. 126(4):746-59, 2010, and Milosavljević et al., Am J Med Genet 170(7):1874-80, 2016. It is contemplated that a subject having mutations that activate the MAPK pathway would benefit from treatment with CNP variants as described herein to improve bone growth and short stature. It is also contemplated that a subject having mutations that activate the MAPK pathway would benefit from treatment with CNP variants as described herein to improve other comorbidities associated with an overactive MAPK pathway in other cells throughout the body where the NPR2 receptor is expressed on its surface.

Mutations in the PTPN11 gene, which encodes the non-receptor protein tyrosine phosphatase SHP-2, lead to disorders characterized by short stature such as Noonan's Syndrome (Musente et al., Eur J Hum Genet 11:201-206 (2003). Musente (*supra*) identifies numerous mutations in the PTPN11 gene that lead to short stature. Gain of function mutations lead to overactive signaling through SHP2 and inhibit Growth Hormone-induced IGF-1 release, thereby contributing to a decrease in bone growth (Rocca Serra-Nédélec, PNAS 109:4257-4262, 2012). It is contemplated that a subject having a homozygous or heterozygous PTPN11 mutation would benefit from treatment with CNP variants as described herein to improve bone growth and short stature.

Mutations in the Indian hedgehog (IHH) gene, which is related to regulation of endochondral ossification, have also been associated with short stature syndromes (Vasques et al., J Clin Endocrinol Metab. 103:604-614, 2018). Many IHH mutations identified segregate with short stature in a dominant inheritance pattern. Given the association of IHH with bone growth and ossification, it is contemplated that subjects having a homozygous or heterozygous IHH mutation will benefit from treatment with a CNP variant as described herein.

Mutations in FGFR3, including N540K and K650N, lead to short stature and hypochondroplasia.

Insulin-like growth factor 1 receptor (IGF1R) is a heterotetrameric (α2β2) transmembrane glycoprotein with an intrinsic kinase activity. IGF1R has been shown to have a role in prenatal and postnatal growth. Heterozygous mutations in IGF1R have been identified in Small for gestational age children (SGA) and individuals with familial short stature (Kawashima et al., Endocrine J. 59:179-185, 2012). Mutations in IGF1R associated with short stature include R108Q/K115N, R59T, R709Q, G1050K, R481Q, V599E, and G1125A (Kawashima, *supra*)*.*

Height is a highly heritable trait that can be influenced by the combined effect of hundreds or thousands genes (Wood et al., Nature Genetics, 46:1173-1189, 2014). Short stature in an individual can be the result of the combined effect of these genes, without a single gene being the primary contributor. It is contemplated that such individuals with short stature defined by a height SDS of less than -1.0, -1.5, -2.0, -2.5, or -3.0, can be beneficially treated with a CNP variant given the ability of CNP to increase the length of normal animals, for example, enhance bone growth and length of bones.

In various embodiments, the CNP variants are useful to treat a subject with short stature having a height SDS of less than -1.0, -1.5, -2.0, -2.5, or -3.0, and having at least one parent with a height SDS of less than -1.0, -1.5, -2.0 or -2.5, optionally wherein the second parent has height within the normal range. In various embodiments, the CNP variants are useful to treat a subject with short stature having a height SDS of between -2.0 to -3.0. In various embodiments, the CNP variants are useful to treat a subject with short stature having a height SDS of between -2.0 to -2.5. In various embodiments, the short stature is associated with one or more mutations in a gene associated with short stature, such as, collagen (COL2A1, COL11A1, COL9A2, COL10), aggrecan (ACAN), indian hedgehog (IHH), PTPN11, NPR2, NPPC, FGFR3, or insulin growth factor 1 receptor (IGF1R), or combinations thereof.

In various embodiments, the short stature is associated with one or more mutations in a gene associated with a RASopathy.

In various embodiments, the short stature is a result of mutations in multiple genes as determined by polygenic risk score (PRS). In various embodiments, the subject has a mutation in NPR2 and a low PRS. In various embodiments, the subject has a mutation in FGFR3 and a low PRS. In various embodiments, the subject has a mutation in NPR2 and a low PRS. In various embodiments, the subject has a mutation in IGF1R and a low PRS. In various embodiments, the subject has a mutation in NPPC and a low PRS. In various embodiments, the subject has a mutation in SHOX and a low PRS. In various embodiments, the subject has one or more mutation in one or more of FGFR3, IGF1R, NPPC, NPR2 and SHOX, and a low PRS. In various embodiments, the PRS is 1 or 2. In various embodiments, the PRS is 1. In various embodiments, the PRS is 2.

In addition, the CNP salts are useful for treating other bone-related conditions and disorders, such as rickets, hypophosphatemic rickets [including X-linked hypophosphatemic rickets (also called vitamin D-resistant rickets) and autosomal dominant hypophosphatemic rickets], and osteomalacia [including tumor-induced osteomalacia (also called oncogenic osteomalacia or oncogenic hypophosphatemic osteomalacia)].

The CNP salts of the disclosure can also be used to treat osteoarthritis. Osteoarthritis is a degenerative disease of the articular cartilage and occurs frequently in the elderly. Osteoarthritis involves destruction of the cartilage and proliferative change in the bone and cartilage resulting from degeneration of articular components, with the change resulting in a secondary arthritis (e.g., synovitis). The extracellular matrix proteins, which are the functional entity of the cartilage, are reduced, and the number of chondrocytes decreases in osteoarthritis (Arth. Rheum. 46(8): 1986-1996 (2002)). By promoting the matrix production, growth and differentiation of chondrocytes, the CNP compositions are useful for countering the undesired effects of FGF-2 and increasing matrix synthesis in subjects suffering from arthritis, including osteoarthritis, thereby treating arthritis, including osteoarthritis.

In certain embodiments, the CNP salts and compositions and formulations comprising the same of the present disclosure are useful for improving one or more of the symptom(s) or physiological consequences of a skeletal dysplasia, wherein the improvement may be increased absolute growth, increased growth velocity, increased qualitative computed tomography (QCT) bone mineral density, improvement in growth plate morphology, increased long bone growth, improvement in spinal morphology, improved elbow joint range of motion and/or decreased sleep apnea. In this regard, it is noted that the terms "improved", "improvement", "increase", "decrease" and grammatical equivalents thereof are all relative terms that when used in relation to a symptom or physiological consequence of a disease state, refer to the state of the symptom or physiological consequence of the disease after treatment with a CNP salt (or composition or formulation comprising the same) of the present invention as compared to the same symptom or physiological consequence of the disease before treatment with a CNP salt (or composition or formulation comprising the same) of the present invention (i.e., as compared to "baseline"). As described above, a "baseline" state can be determined either through measurement of the state in the subject prior to treatment (which can subsequently be compared to the state in the same subject after treatment), or through measurement of that state in a population of subjects suffering from the same affliction that share the same or similar characteristics (e.g., age, sex and/or disease state or progression).

In yet another embodiment, the disclosure provides salts of CNP variants that *in vitro* or *in vivo* stimulate the production of at least about 50%, 60%, 70%, 80%, 90%, 100%, 110%, 120%, 130%, 140% or 150% of the cGMP level produced under the same concentration of wtCNP22 (e.g. 1 uM). In a still further embodiment, the hydrophobic salt comprising the CNP or CNP variants of the disclosure *in vitro* or *in vivo* stimulate the production of at least about 50%, 60%, 70%, 80%, 90%, 100%, 110%, 120%, 130%, 140% or 150% of the cGMP level produced under the same concentration of wtCNP22 (e.g. 1 uM).

It is contemplated that any of the CNP variants described herein are useful in the methods.

In various embodiments, the CNP variant is
PGQEHPNARKYKGANKKGLSKGCFGLKLDRIGSMSGLGC (Pro-Gly-CNP-37 (SEQ ID NO: 1)). In various disclosures (not being part of the present invention) the peptide further comprises an acetyl group. In various embodiments, the acetyl group is on the N-terminus of the peptide. In various embodiments, the acetyl group is on an amino acid side chain within the peptide sequence. In various embodiments, the peptide further comprises an OH or an NH₂ group at the C-terminus. In various embodiments, the variant comprises one or more linker groups as described herein. In various embodiments, the linker is a hydrolysable linker. According to the invention, the peptide comprises a salt of an electrostatically charged peptide, the salt comprising the electrostatically charged peptide complexed with a hydrophobic counterion.

Efficacy of treatment is measured by various parameters. In various embodiments, efficacy is assessed as the change in annualized growth velocity from the baseline period to the intervention period. Efficacy will also be assessed as the change in height SDS from baseline to end of treatment as measured using the CDC growth curves, and growth velocity SDS will be based on the Bone Mineral Density in Childhood Study (Kelly et al., J. Clin. Endocrinol. Metab. 2014;99(6):2104-2112).

QoLISSY, the Quality of Life in Short Stature Youth, is assessed as directed (Quality of Life in Short Stature Youth - The QoLISSY Questionnaire User's Manual. Lengerich: Pabst Science Publishers; 2013).

### Pharmaceutical Compositions

The disclosure provides pharmaceutical compositions, including modified release compositions, comprising a peptide salt described herein, and one or more pharmaceutically acceptable excipients, carriers and/or diluents. In certain embodiments, the compositions further comprise one or more other biologically active agents (*e.g.,* inhibitors of proteases, receptor tyrosine kinases, and/or the clearance receptor NPR-C).

The disclosure provides for modified release compositions comprising a hydrophobic peptide salt as described herein. In various embodiments, the modified release composition is an extended release composition. In various embodiments, the extended release composition comprises a hydrophobic CNP salt as defined in the claims. In various embodiments, the hydrophobic counterion in the CNP salt is oleate, deoxycholate, decanoate, pamoate, docusate or dodecyl sulfate. In various embodiments, if a polyvalent cation is present, the cation comprises zinc or calcium. In various embodiments, if a cation is present, the cation is Zn²⁺ or Ca²⁺.

Precipitated peptide complexes can display extended release characteristics under pH conditions at which precipitation was formed. Precipitated peptide complexes can also be used for further processing into a matrix that provides additional barriers for sustained release such as slow degrading microspheres, hydrogels, and the like. It is contemplated that the hydrophobic CNP salt is a solid, semi-solid, gel, crystalline, amorphous, nanoparticle, microparticle, amorphous nanoparticle, amorphous microparticle, crystalline nanoparticle or crystalline microparticle, and is resuspended in an aqueous solution or in oil. In various embodiments, the aqueous solution is water, saline, or buffer. In various embodiments, the particle is between 1 and 10,000 micrometers (um), between 1 um to 2000 um, between 2 um to 1000 um, between 5 um to 500 um, between 10 um to 1000 um, between 50 um to 500 um, between 100 um to 800 um, between 200 to 600 um, between 300um to 500 um, between 100 um to 300 um, between 50 um to 100 um, or between 10 um to 50 um. In various embodiments the particle is a nanoparticle. In various embodiments, the nanoparticle is approximately between 5 nanometers (nm) to 1000 nm, between 8 nm to 900 nm, between 10 nm-800 nm, between 20 nm to 600 nm, between 50 nm to 500 nm, between 50 to 400 nm, between 20 to 300 nm, between 300 to 800 nm or between 200 to 600 nm.

In various embodiments, the oil comprises a triglyceride or a fatty acid, which can be saturated or unsaturated. Triglycerides and fatty acids as described herein are also contemplated for use with the hydrophobic CNP salt compositions. In various embodiments, the fatty acid is hexanoic acid, octanoic acid, decanoic acid, or dodecanoic acid. In various embodiments, the fatty acid is hexanoic acid, octanoic acid, decanoic acid, dodecanoic acid or docusate.

In various embodiments, for the extended release composition, (i) less than about 20% of peptide is released by day 1; and (ii) about 90% of peptide is released weekly, or about 90% of peptide is released bi-weekly, or about 90% of peptide is released monthly, at pH 7 to 7.6.

In various embodiments, less than about 20% of peptide is released by day 1 at pH 7 to 7.6. It is further contemplated that (i) less than about 30%, or about 40%, or about 50% of peptide is released by day 1, at pH 7.0 to 7.6; and (ii) about 90% of peptide is released weekly, or about 90% of peptide is released bi-weekly, or about 90% of peptide is released monthly, at pH 7 to 7.6. It is further contemplated that (i) less than about 30%, or about 40%, or about 50%, or about 60% of peptide is released by day 1, at pH 7.0 to 7.6; and (ii) about 70%, about 80%, or about 90% of peptide is released weekly; or about 70%, about 80%, or about 90% of peptide is released bi-weekly; or about 70%, about 80%, or about 90% of peptide is released every three weeks; or about 70%, about 80%, or about 90% of peptide is released monthly, at pH 7 to 7.6. In various embodiments, about 90% of peptide is released weekly, at pH 7 to 7.6. In various embodiments, about 90% of peptide is released biweekly, at pH 7 to 7.6. In various embodiments, about 90% of peptide is released monthly at pH 7 to 7.6. It is further contemplated that the release can be at a pH between pH 7.0 to 7.6, between pH 7.1 to 7.5, between pH 7.2 to 7.4, between pH 7.2 to 7.6, or between pH 7.0 to 7.4.

In various embodiments, (i) less than about 25%, about 30%, about 35%, about 40%, about 45%, about 50%, about 55%, about 60%, about 65%, about 70%, or about 75% of peptide is released by day 1, at pH 7.0 to 7.6; and (ii) about 90% of peptide is released weekly, or about 90% of peptide is released bi-weekly, or about 90% of peptide is released monthly, at pH 7 to 7.6. It is further contemplated that (i) less than about 25%, about 30%, about 35%, about 40%, about 45%, about 50%, about 55%, about 60% about 65%, about 70%, or about 75% of peptide is released by day 1, at pH 7.0 to 7.6; and (ii) about 70%, about 80%, or about 90% of peptide is released weekly; or about 70%, about 80%, or about 90% of peptide is released bi-weekly; or about 70%, about 80%, or about 90% of peptide is released every three weeks; or about 70%, about 80%, or about 90% of peptide is released monthly, at pH 7 to 7.6; or alternatively ii) about 70%, about 75%, about 80%, about 85%, or about 90% of peptide is released weekly; or about 70%, about 75%, about 80%, about 85%, or about 90% of peptide is released bi-weekly; or about 70%, about 75%, about 80%, about 85%, or about 90% of peptide is released every three weeks; or about 70%, about 75%, about 80%, about 85%, or about 90% of peptide is released monthly, at pH 7 to 7.6

In various embodiments, the extended release composition comprises an excipient, diluent or carrier. In various embodiments, the excipient, diluent or carrier is a pharmaceutically acceptable excipient, diluent or carrier.

Non-limiting examples of excipients, carriers and diluents include vehicles, liquids, buffers, isotonicity agents, additives, stabilizers, preservatives, solubilizers, surfactants, emulsifiers, wetting agents, adjuvants, and so on. The compositions can contain liquids (e.g., water, ethanol); diluents of various buffer content (e.g., Tris-HCl, phosphate, acetate buffers, citrate buffers), pH and ionic strength; detergents and solubilizing agents (e.g., Polysorbate 20, Polysorbate 80); anti-oxidants (e.g., methionine, ascorbic acid, sodium metabisulfite); preservatives (e.g., Thimerosol, benzyl alcohol, m-cresol); and bulking substances (e.g., lactose, mannitol, sucrose). The use of excipients, diluents and carriers in the formulation of pharmaceutical compositions is known in the art; see, e.g., Remington's Pharmaceutical Sciences, 18th Edition, pages 1435-1712, Mack Publishing Co. (Easton, Pennsylvania (1990)).

For example, carriers include without limitation diluents, vehicles and adjuvants, as well as implant carriers, and inert, non-toxic solid or liquid fillers and encapsulating materials that do not react with the active ingredient(s). Non-limiting examples of carriers include phosphate buffered saline, physiological saline, water, and emulsions (e.g., oil/water emulsions). A carrier can be a solvent or dispersing medium containing, e.g., ethanol, a polyol (e.g., glycerol, propylene glycol, liquid polyethylene glycol, and the like), a vegetable oil, and mixtures thereof.

In some embodiments, the compositions are liquid formulations. In certain embodiments, the formulations comprise a hydrophobic CNP salt in a concentration range from about 0.1 mg/ml to about 20 mg/ml, or from about 0.5 mg/ml to about 20 mg/ml, or from about 1 mg/ml to about 20 mg/ml, or from about 0.1 mg/ml to about 10 mg/ml, or from about 0.5 mg/ml to about 10 mg/ml, or from about 0.5 to 5 mg/ml, or from about 0.5 to 3 mg/ml, or from about 1 mg/ml to about 10 mg/ml. In various embodiments, the CNP variant is in a concentration of 0.8 mg/mL to 2 mg/mL. In various embodiments, the CNP variant is at a concentration of 0.8 mg/mL In various embodiments, the CNP variant is at a concentration of 2.0 mg/mL. In various embodiments, the CNP variant is reconstituted from a lyophilized powder.

In further embodiments, the compositions comprise a buffer solution or buffering agent to maintain the pH of a CNP-containing solution or suspension within a desired range. Non-limiting examples of buffer solutions include phosphate buffered saline, Tris buffered saline, and Hank's buffered saline. Buffering agents include without limitation sodium acetate, sodium phosphate, and sodium citrate. Mixtures of buffering agents can also be used. In certain embodiments, the buffering agent is acetic acid/acetate or citric acid/citrate. The amount of buffering agent suitable in a composition depends in part on the particular buffer used and the desired pH of the solution or suspension. In some embodiments, the buffering agent has a concentration of about 10 mM ± 5 mM. In certain embodiments, the pH of a composition is from about pH 3 to about pH 9, or from about pH 3 to about pH 7.5, or from about pH 3.5 to about pH 7, or from about pH 3.5 to about pH 6.5, or from about pH 4 to about pH 6, or from about pH 4 to about pH 5, or is at about pH 5.0 ± 1.0. In various embodiments, the pH is about 5.0, 5.1, 5.2, 5.3, 5.4, 5.5, 5.6, 5.7, 5.8, 5.9 or 6.0. In various embodiments, the pH is 5.5.

In other embodiments, the compositions contain an isotonicity-adjusting agent to render the solution or suspension isotonic and more compatible for administration. Non-limiting examples of isotonicity agents include NaCl, dextrose, glucose, glycerin, sorbitol, xylitol, and ethanol. In certain embodiments, the isotonicity agent is NaCl. In certain embodiments, NaCl is in a concentration of about 160 ± 20 mM , or about 140 mM ± 20 mM, or about 120 ± 20 mM , or about 100 mM ± 20 mM, or about 80 mM ± 20 mM, or about 60 mM ± 20 mM.

In yet other embodiments, the compositions comprise a preservative. Preservatives include, but are not limited to, m-cresol and benzyl alcohol. In certain embodiments, the preservative is in a concentration of about 0.4% ± 0.2%, or about 1% ± 0.5%, or about 1.5% ± 0.5%, or about 2.0% ± 0.5%.

In still other embodiments, the compositions contain an anti-adsorbent (e.g., to mitigate adsorption of a CNP salt to glass or plastic). Anti-adsorbents include without limitation benzyl alcohol, Polysorbate 20, and Polysorbate 80. In certain embodiments, the anti-adsorbent is in a concentration from about 0.001% to about 0.5%, or from about 0.01% to about 0.5%, or from about 0.1% to about 1%, or from about 0.5% to about 1%, or from about 0.5% to about 1.5%, or from about 0.5% to about 2%, or from about 1% to about 2%.

In additional embodiments, the compositions comprise a stabilizer. Non-limiting examples of stabilizers include glycerin, glycerol, thioglycerol, methionine, and ascorbic acid and salts thereof. In some embodiments, when the stabilizer is thioglycerol or ascorbic acid or a salt thereof, the stabilizer is in a concentration from about 0.1% to about 1%. In other embodiments, when the stabilizer is methionine, the stabilizer is in a concentration from about 0.01% to about 0.5%, or from about 0.01% to about 0.2%. In still other embodiments, when the stabilizer is glycerin, the stabilizer is in a concentration from about 5% to about 100% (neat).

In further embodiments, the compositions contain an antioxidant. Exemplary anti-oxidants include without limitation methionine and ascorbic acid. In certain embodiments, the molar ratio of antioxidant to CNP is from about 0.1:1 to about 15:1, or from about 1:1 to about 15:1, or from about 0.5:1 to about 10:1, or from about 1:1 to about 10:1 or from about 3:1 to about 10:1.

Pharmaceutically acceptable salts can be used in the compositions, including without limitation mineral acid salts (e.g., hydrochloride, hydrobromide, phosphate, sulfate), salts of organic acids (e.g., acetate, propionate, malonate, benzoate, mesylate, tosylate), and salts of amines (e.g., isopropylamine, trimethylamine, dicyclohexylamine, diethanolamine). A thorough discussion of pharmaceutically acceptable salts is found in Remington's Pharmaceutical Sciences, 18th Edition, Mack Publishing Company, (Easton, Pennsylvania (1990)).

The pharmaceutical compositions can be administered in various forms, such as tablets, capsules, granules, powders, solutions, suspensions, emulsions, ointments, and transdermal patches. The dosage forms of the compositions can be tailored to the desired mode of administration of the compositions. For oral administration, the compositions can take the form of, e.g., a tablet or capsule (including softgel capsule), or can be, e.g., an aqueous or nonaqueous solution, suspension or syrup. Tablets and capsules for oral administration can include one or more commonly used excipients, diluents and carriers, such as mannitol, lactose, glucose, sucrose, starch, corn starch, sodium saccharin, talc, cellulose, magnesium carbonate, and lubricating agents (e.g., magnesium stearate, sodium stearyl fumarate). If desired, flavoring, coloring and/or sweetening agents can be added to the solid and liquid formulations. Other optional ingredients for oral formulations include without limitation preservatives, suspending agents, and thickening agents. Oral formulations can also have an enteric coating to protect the CNP salt from the acidic environment of the stomach. Methods of preparing solid and liquid dosage forms are known, or will be apparent, to those skilled in this art (see, e.g., Remington's Pharmaceutical Sciences, referenced above).

Formulations for parenteral administration can be prepared, e.g., as liquid solutions or suspensions, as solid forms suitable for solubilization or suspension in a liquid medium prior to injection, or as emulsions. For example, sterile injectable solutions and suspensions can be formulated according to techniques known in the art using suitable diluents, carriers, solvents (e.g., buffered aqueous solution, Ringer's solution, isotonic sodium chloride solution), dispersing agents, wetting agents, emulsifying agents, suspending agents, and the like. In addition, sterile fixed oils, fatty esters, polyols and/or other inactive ingredients can be used. As further examples, formulations for parenteral administration include aqueous sterile injectable solutions, which can contain antioxidants, buffers, bacteriostats, and solutes that render the formulation isotonic with the blood of the intended recipient; and aqueous and nonaqueous sterile suspensions, which can contain suspending agents and thickening agents.

Compositions comprising a hydrophobic CNP salt can also be lyophilized formulations. In certain embodiments, the lyophilized formulations comprise a buffer and bulking agent, and optionally an antioxidant. Exemplary buffers include without limitation acetate buffers and citrate buffers. Exemplary bulking agents include without limitation mannitol, sucrose, dexran, lactose, trehalose, and povidone (PVP K24). In certain embodiments, mannitol is in an amount from about 3% to about 10%, or from about 4% to about 8%, or from about 4% to about 6%. In certain embodiments, sucrose is in an amount from about 6% to about 20%, or from about 6% to about 15%, or from about 8% to about 12%. Exemplary anti-oxidants include, but are not limited to, methionine and ascorbic acid.

In various embodiments, the formulation comprises citric acid, sodium citrate, trehalose, mannitol, methionine, polysorbate 80, and optionally sterile water for injection (WFI).

The disclosure also provides kits (not part of the present invention) containing, e.g., bottles, vials, ampoules, tubes. cartridges and/or syringes that comprise a liquid (e.g., sterile injectable) formulation or a solid (e.g., lyophilized) formulation. The kits can also contain pharmaceutically acceptable vehicles or carriers (e.g., solvents, solutions and/or buffers) for reconstituting a solid (e.g., lyophilized) formulation into a solution or suspension for administration (e.g., by injection), including without limitation reconstituting a lyophilized formulation in a syringe for injection or for diluting concentrate to a lower concentration. Furthermore, extemporaneous injection solutions and suspensions can be prepared from, e.g., sterile powder, granules, or tablets comprising a CNP-containing composition. The kits can also include dispensing devices, such as aerosol or injection dispensing devices, pen injectors, autoinjectors, needleless injectors, syringes, and/or needles.

As a non-limiting example, a kit can include syringes having a single chamber or dual chambers. For single-chamber syringes, the single chamber can contain a liquid CNP formulation ready for injection, or a solid (e.g., lyophilized) CNP formulation or a liquid formulation of a CNP salt in a relatively small amount of a suitable solvent system (e.g., glycerin) that can be reconstituted into a solution or suspension for injection. For dual-chamber syringes, one chamber can contain a pharmaceutically acceptable vehicle or carrier (e.g., solvent system, solution or buffer), and the other chamber can contain a solid (e.g., lyophilized) CNP formulation or a liquid formulation of a CNP salt in a relatively small amount of a suitable solvent system (e.g., glycerin) which can be reconstituted into a solution or suspension, using the vehicle or carrier from the first chamber, for injection.

As a further example, a kit can include one or more pen injector or autoinjector devices, and dual-chamber cartridges. One chamber of a cartridge can contain a pharmaceutically acceptable vehicle or carrier (e.g., solvent system, solution or buffer), and the other chamber can contain a solid (e.g., lyophilized) CNP formulation or a liquid formulation of a CNP salt in a relatively small amount of a suitable solvent system (e.g., glycerin) which can be reconstituted into a solution or suspension, using the vehicle or carrier from the first chamber, for injection. A cartridge can comprise an amount of the CNP salt that is sufficient for dosing over a desired time period (e.g., 2 days, 3 days, 1 week, 2 weeks, 3 weeks, 4 weeks, etc.). The pen injector or autoinjector can be adjusted to administer a desired amount of the CNP formulation from a cartridge.

### Administration and Dosing

The hydrophobic CNP salts, or pharmaceutical compositions or formulations comprising them, can be administered to subjects in various ways such as, e.g., subcutaneously, intraarticularly, intraperitoneally, intramuscularly, intradermally or orally. In one embodiment, the CNP peptide salt composition is administered once daily, once weekly, once every two weeks, once every three weeks, once every 4 weeks, once every 6 weeks, once every two months, once every three months or once every six months.

The hydrophobic CNP salts or salt compositions can also be administered by implantation of a depot at the target site of action (e.g., an abnormal or degenerated joint or cartilage area). Alternatively, the CNP salt can be administered sublingually under the tongue (e.g., sublingual tablet) by transdermal delivery (e.g., by means of a patch on the skin) or orally in the form of microspheres, microcapsules, liposomes (uncharged or charged (e.g., cationic)), polymeric microparticles (e.g., polyamides, polylactide, polyglycolide, poly(lactide-glycolide)), microemulsions, and the like.

The hydrophobic CNP salt compositions described herein can be administered to patients in need thereof at therapeutically effective doses to treat, ameliorate or prevent bone-related disorders (e.g., skeletal dysplasias, including achondroplasia). The safety and therapeutic efficacy of the CNP salts can be determined by standard pharmacological procedures in cell cultures or experimental animals, such as, for example, by determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀ /ED₅₀. Active agents exhibiting a large therapeutic index are normally preferred.

In certain embodiments, the hydrophobic CNP salt compositions described herein are administered at a dose in the range from about 3 or 10 nmol/kg to about 300 nmol/kg, or from about 20 nmol/kg to about 200 nmol/kg, or from about 3 nmol/kg to 100 nmol/kg. In some embodiments, the CNP salt compositions are administered at a dose of about 3, 4, 5, 6, 7, 8, 9, 10, 15, 20, 25, 30, 35, 40, 45, 50, 55, 60, 65, 70, 75, 80, 85, 90, 95, 100, 110, 120, 125, 130, 140, 150, 160, 170, 175, 180, 190, 200, 210, 220, 230, 240, 250, 260, 270, 280, 290, 300, 350, 400, 450, 500, 750, 1000, 1250, 1500, 1750 or 2000 nmol/kg or other dose deemed appropriate by the treating physician. In other embodiments, the CNP salt compositions are administered at a dose of about 5, 6, 7, 8, 9, 10, 15, 20, 30, 40, 50, 60, 70, 80, 90, 100, 110, 120, 130, 140, 150, 160, 170, 180, 190, 200, 250, 300, 350, 400, 450, 500, 550, 600, 650, 700, 750, 800, 850, 900, 950 or 1000 µg/kg, or about 0.5, 0.8, 1.0, 1.25, 1.5, 2, 2.5, 3, 3.5, 4, 4.5, 5, 5.5, 6, 6.5, 7, 7.5, 8, 8.5, 9, 9.5 or 10 mg/kg, or other dose deemed appropriate by the treating physician. The doses of hydrophobic CNP salts described herein can be administered according to the dosing frequency/frequency of administration described herein, including without limitation daily, 2 or 3 times per week, weekly, every 2 weeks, every 3 weeks, monthly, etc. In various embodiments, the CNP salt is administered daily subcutaneously. In various embodiments, the CNP salt is administered weekly subcutaneously. In various embodiments, the CNP variant is administered at a dose of 2.5 µg/kg/day to 60 µg/kg/day, 10µg/kg/day to 45 µg/kg/day, or 15µg/kg/day to 30 µg/kg/day. In various embodiments, the CNP variant is administered at a dose of 15 µg/kg/day. In various embodiments, the CNP variant is administered at a dose of 30 µg/kg/day.

The frequency of dosing/administration of a hydrophobic CNP salt for a particular subject may vary depending upon various factors, including the disorder being treated and the condition and response of the subject to the therapy. The hydrophobic CNP salt can be administered in a single dose or in multiple doses per dosing. In certain embodiments, the hydrophobic CNP salt composition is administered, in a single dose or in multiple doses, once daily, once weekly, once every two weeks, once every three weeks, once every 4 weeks, once every 6 weeks, once every two months, once every three months or once every six months, or as deemed appropriate by the treating physician. In various embodiments, the CNP variant is administered for 3 months, 6 months, 12 months or more.

In some embodiments, a hydrophobic CNP salt composition is administered so as to allow for periods of growth (e.g., chondrogenesis), followed by a recovery period (e.g., osteogenesis). For example, the CNP salt composition may be administered subcutaneously or by another mode daily or multiple times per week for a period of time, followed by a period of no treatment, then the cycle is repeated. In some embodiments, the initial period of treatment (e.g., administration of the CNP salt composition daily or multiple times per week) is for 3 days, 1 week, 2 weeks, 3 weeks, 4 weeks, 5 weeks, 6 weeks, 7 weeks, 8 weeks, 9 weeks, 10 weeks, 11 weeks or 12 weeks. In a related embodiment, the period of no treatment lasts for 3 days, 1 week, 2 weeks, 3 weeks or 4 weeks. In certain embodiments, the dosing regimen of the CNP salt compositions is daily for 3 days followed by 3 days off; or daily or multiple times per week for 1 week followed by 3 days or 1 week off; or daily or multiple times per week for 2 weeks followed by 1 or 2 weeks off; or daily or multiple times per week for 3 weeks followed by 1, 2 or 3 weeks off; or daily or multiple times per week for 4, 5, 6, 7, 8, 9, 10, 11 or 12 weeks followed by 1, 2, 3 or 4 weeks off.

### Biomarkers (embodiments relating to biomarkers and their use are not part of the present invention)

For treatment of bone-related disorders, indicators of growth can be measured, such as long bone growth measurements *in utero* and neonatal and measurements of bone growth biomarkers such as CNP, cGMP, Collagen II, Collagen X, osteocalcin, and Proliferating Cell Nuclear Antigen (PCNA).

One CNP signaling marker is cGMP (guanosine 3',5' cyclic monophosphate). The level of this intracellular signaling molecule increases after CNP binds to and activates its cognate receptor NPR-B. Elevated levels of cGMP can be measured from cell culture extracts *(in vitro)* after CNP exposure, conditioned media from bone ex-plant studies *(ex vivo)* after CNP exposure, and in the plasma *(in vivo)* within minutes of CNP administration subcutaneously, intravenously, or via other routes of administration known in the art.

Cartilage and bone-specific analytes (or cartilage- and bone-associated markers) can also be measured to assess CNP efficacy. For example, fragments of cleaved collagen type II are a cartilage-specific marker for cartilage turnover. Type II collagen is the major organic constituent of cartilage and fragments of type II collagen (cleaved collagen) are released into circulation, and subsequently secreted into the urine, following cartilage turnover. Cartilage turnover precedes new bone formation.

A bone-specific biomarker for bone formation which can be measured is N-terminal propeptides of type I procollagen (PINP). The synthesis of type I collagen is an important step in bone formation, as type I collagen is the major organic component in bone matrix. During collagen synthesis, propeptides are released from the procollagen molecule and can be detected in serum. In addition, fragments of collagen type I can be measured as a marker for bone resorption.

Other potential biomarkers for cartilage and bone formation and growth include aggrecan chondroitin sulfate (cartilage-specific marker for cartilage turnover), propeptides of type II collagen (cartilage-specific marker for cartilage formation), alkaline phosphatase (bone-specific) and osteocalcin (bone-specific marker for bone formation). Cartilage- and bone-associated biomarkers can be measured, e.g., in serum from efficacy/pharmacodynamic *in vivo* studies and from the conditioned media of ex *vivo* studies, using commercially available kits.

In one embodiment, the level of at least one bone- or cartilage-associated biomarker is assayed or measured in a subject that has been administered a CNP salt or sustained release composition described herein in order to monitor the effects of the CNP composition on bone and cartilage formation and growth *in vivo.* For example, an increase in the level of at least one bone- or cartilage-associated biomarker may indicate that administration of a CNP salt or sustained release composition has a positive effect on bone growth and is a useful treatment for skeletal dysplasias and other bone- or cartilage-related diseases or disorders associated with decreased CNP activity. Exemplary bone- or cartilage-associated biomarkers include, but are not limited to, CNP (e.g., endogenous levels of CNP), cGMP, propeptides of collagen type II and fragments thereof, collagen type II and fragments thereof, osteocalcin, proliferating cell nuclear antigen (PCNA), propeptides of type I procollagen (PINP) and fragments thereof, collagen type I and fragments thereof, aggrecan chondroitin sulfate, and alkaline phosphatase.

In various embodiments, biomarkers are measured by obtaining a biological sample from a subject who will be administered, is being administered or has been administered a CNP salt or sustained release composition. Biomarkers can be measured using techniques known in the art, including, but not limited to, Western Blot, enzyme linked immunosorbant assay (ELISA), and enzymatic activity assay. The biological sample can be blood, serum, urine, or other biological fluids.

Additional aspects and details of the disclosure will be apparent from the following examples, which are intended to be illustrative.

### EXAMPLES

### Example 1: Generation of Hydrophobic CNP Salts

In order to determine whether complexing the hydrophilic peptide CNP with a hydrophobic counterion to stabilize its charge would be effective to improve the formulation of CNP for therapeutic purposes, experiments to alter the electrostatic charge of CNP in combination with other ionic components were undertaken.

To optimize the electrostatic complexation between a CNP peptide and hydrophobic counterion species, and hence controlled precipitation of the peptide complex, the solution environment should allow a large population of both species to be ionized. pH adjustments of both solutions to an intermediate value between 10 (pl of CNP) and the acidic pKa of the counterion (for example, the pKa of an example counterion species oleic acid is ~4-5) is important for the yield of peptide complex precipitation.

To make hydrophobic CNP salts, including CNP variants, CNP-acetate is placed into water or a buffer. In a first experiment, a stock solution comprising CNP at 20 mg/mL in water was prepared. 100 mM Tris, pH 9.00 is also effective as a solvent. Disodium pamoic acid, oleic acid, or sodium docusate were placed in a buffer at a concentration that is in a molar excess to the number of oppositely charged groups on CNP, in this instance a 12 molar excess of counterion to CNP. CNP has 6 positively charged groups at pH 7, and this is a two-fold excess over the number of positively charged groups. It was found that in buffered solutions (76 mM acetate buffer pH 4.84 and 100 mM phosphate buffer pH 6.61) the counterions were not easily soluble and formed a suspension of what appeared to be insoluble or low solubility counterion in buffer, however in water and in 100 mM Tris pH 9.00 counterions oleic acid, disodium pamoic acid, and sodium docusate were soluble.

When a metal cation was added to facilitate salt precipitation, the metal cation was dissolved in water and added to the CNP solution and not the counterion solution, it was observed that if the metal cation is added directly to the counterion it induced precipitation of the counterion solutions. The metal cation solution (e.g., ZnCl₂) was dissolved in water at high concentration (>100 mg/mL) such that a small volume could be added to the CNP solution and not significantly change the concentration or pH. The metal cation could be added at various concentrations, but in the initial experiment it was added in a 4 molar excess to the CNP concentration (there are two negatively charged amino acids in CNP and this was added such that there were 2 moles of zinc per mol negatively charged amino acid). The counterion solution was then added to the CNP solution containing or not containing metal cation dropwise, vortexing the tube at the highest setting for one second after every addition.

After enough solution was added for the desired ratio of CNP to counterion, the reaction tube was spun in a centrifuge at 10,000xg for 5 minutes to pellet the salt precipitate. After spinning, the supernatant was then removed and an equal volume of water to supernatant removed was added and the pellet was resuspended. This time the tube was spun for 7500xg for 3-5 minutes and the salt could be observed to pellet. After spinning, the salt pellet was again washed with water and resuspended. The tube was spun again and supernatant removed. The contents were then moved to a vial (e.g., a 6R borosilicate glass vial), stoppered, frozen, and lyophilized.

The subsequent salt powder was then evaluated for solubility in various buffers. Initially, ~1 mg of powder was weighed into a tube and the proper volume of solvent added to get to 1 mg/mL. The tube was left overnight rocking at about 37° C. It was found that the oleate salts were dissolvable in 20% acetic acid. The pamoate salt was soluble in dimethyl sulfoxide (DMSO).

These experiments show that precipitation of highly aqueous soluble peptides into water insoluble/low solubility aggregates with sizes ranging from 5 nanometers to 1 millimeter in diameter is possible.

### Example 2--Characterization of CNP Salts

Hydrophobic salts of CNP were made as in Example 1 and tested for precipitation and solubility. Table 1 describes that CNP complexed with various hydrophobic counterions results in precipitation or formation of solids.

**Table 1**

| **Counterion** | **Solvent for Reaction** | **PPT** |
|---|---|---|
| CNP-Oleate | H₂O | Yes |
| CNP-Pamoate | H₂O | Yes |
| CNP-Deoxycholate | H₂O | Yes |
| CNP-Decanoate | H₂O | Gelation |
| CNP -Oleate | Na-Phos | Yes |
| CNP -Pamoate | Na-Phos | Yes |
| CNP -Deoxycholate | Na-Phos | Yes |
| CNP -Decanoate | Na-Phos | Gelation |
| CNP -Ca⁺² (Oleate) | H₂O | Yes |
| CNP -Ca⁺² (Pamoate) | H₂O | Yes |
| CNP -Ca⁺² (deoxycholate) | H₂O | Yes |
| CNP -Ca⁺² (decanoate) | H₂O | Yes |
| CNP -Zn⁺² (Oleate) | H₂O | Yes |
| CNP-Zn⁺² (Pamoate) | H₂O | Yes |
| CNP -Zn⁺² (deoxycholate) | H₂O | Yes |
| CNP -Zn⁺² (decanoate) | H₂O | Yes |

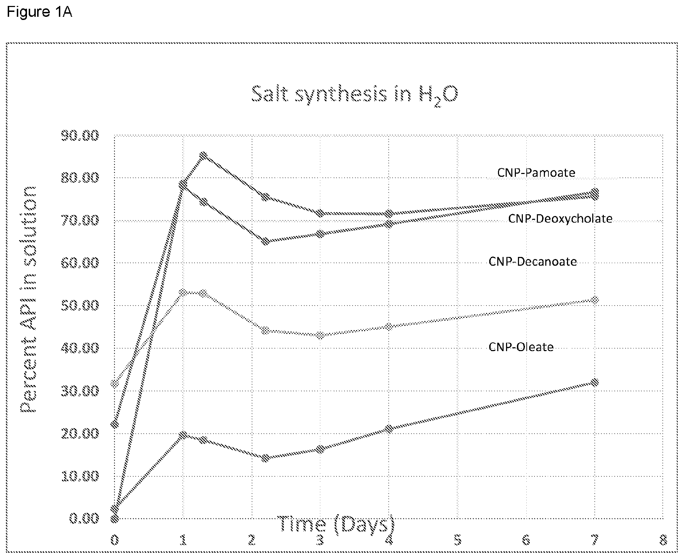

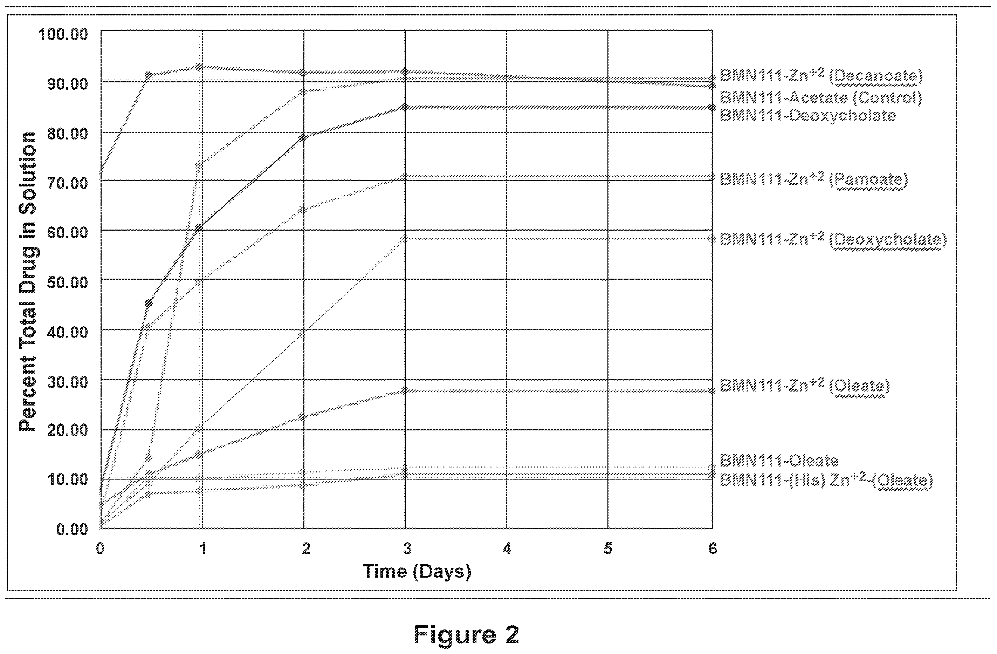

Dissolution studies were also carried out with the salt precipitate. 1 mg CNP salt was resuspended in 50 mL 1X PBS, pH 6.5, at 37°C and measured for dissolving of solids and release of CNP into solution for 7 days. Buffer was not replaced daily. Figures 1A-1D and Figure 2 show that dissolution of the hydrophobic salts was slower than that for the CNP-acetate composition.

### Example 3: Heterozygous NPR2 Mutations Are Responsive to CNP Treatment

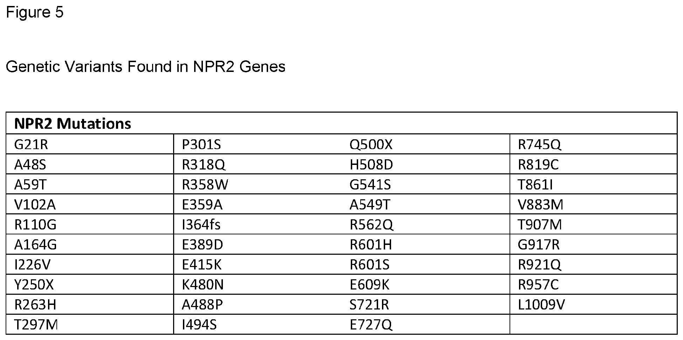

To determine the effects of CNP on subjects with short stature resulting from NPR2 mutations, cellular models of NPR2 mutations were developed. Exemplary NPR2 mutations analyzed are set out in Figure 5. Rat chondrosarcoma (RCS) cells having either a knockout of, or heterozygous loss of function mutations in, the NPR2 gene were made by RNP transfection into RCS cells using 125ng NPR2 variants or wild-type NPR2 plasmid DNA transfected into RCS or HEK293 cells. Single cell clones were seeded and genotyped by Sanger sequencing. Cell models are able to reproduce published cGMP phenotypes of the different mutations.

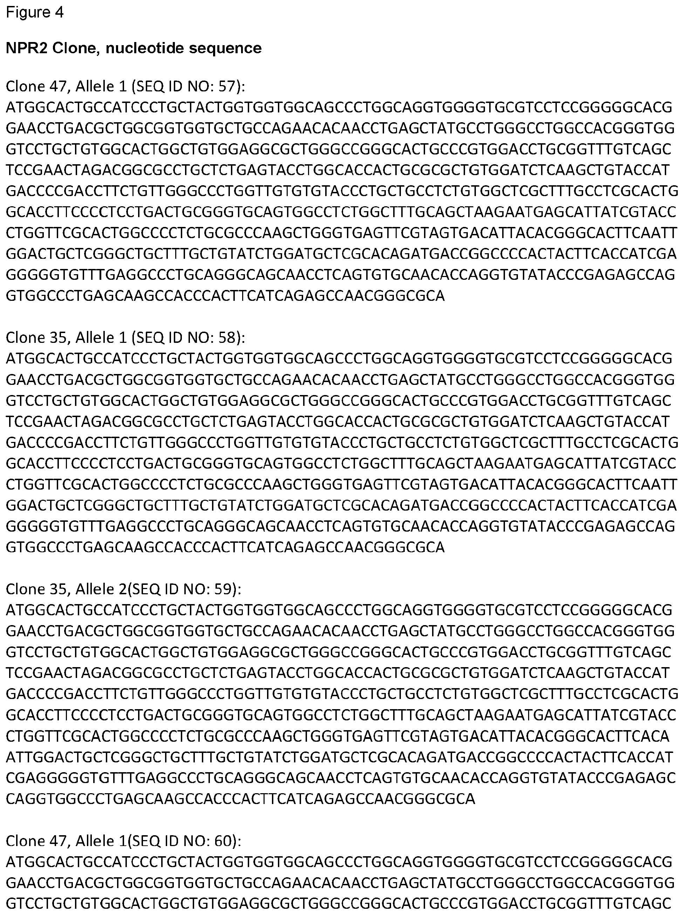

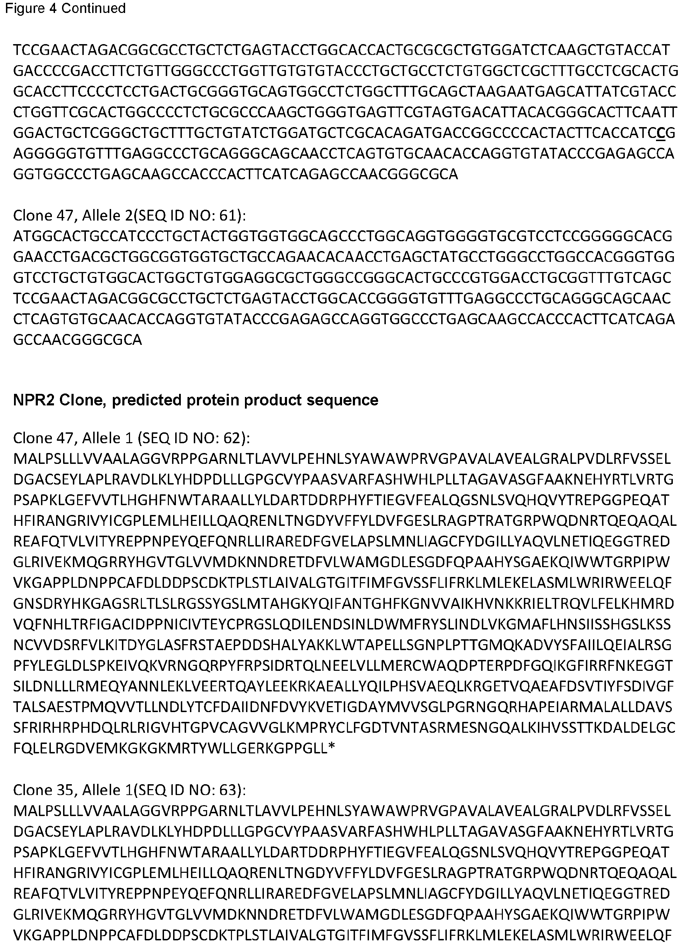

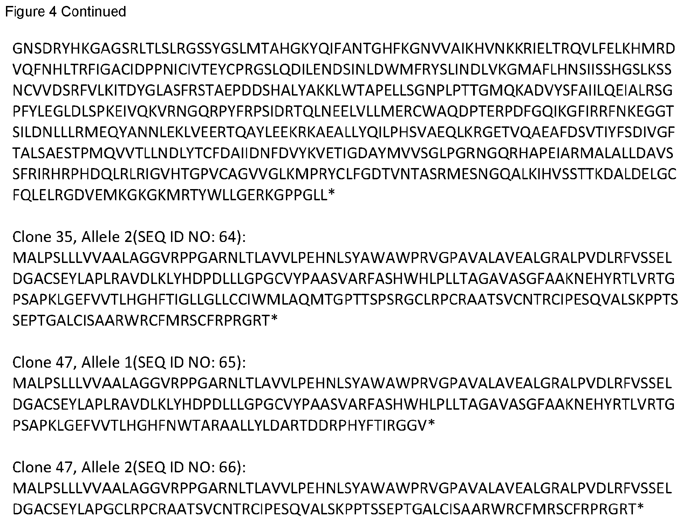

The NPR2 clones were created by creating insertions and deletions in the first exon of NPR2 in RCS cells. The sequence of the first exon in NPR2 was confirmed by next-generation sequencing and is set out in Figure 4. NPR2 mutant cells were tested for activity in response to CNP administration by a cGMP stimulation assay using a CatchPoint Cyclic-GMP Fluorescent Assay after treatment with 6nM of Pro-Gly CNP37. Briefly, NIH3T3 cells (ATCC, CRL-1658) and NPR-transfected HEK293 cells were seeded at 60,000 cells/well in a 96-well plate (96-well black imaging plates, Grenier, #655090). RCS (rat chondrosarcoma) cells were seeded at 40,000 cells/well. Culture media was as follows: NIH3T3 culture media: DMEM high glucose, pyruvate (Thermo, 11995-073) + 10% FBS + 1x Pen Strep (abbrev P/S, Thermo, cat# 15140122). NIH3T3 was the control system for the cGMP assay HEK293 culture media: EMEM + 10% FBS + 1x P/S + 1x GMAX. RCS culture media: DMEM + 10% FBS + 1x Pen Strep. Serum free NIH3T3 media: DMEM + 1x P/S for treatment of cells with IBMX (CAS 28822-58-4); Serum free NIH3T3 media with BSA: DMEM + 1x P/S + 0.5 mg/mL BSA (Thermo, A9418-100G) for treatment of cells with CNP.

Cells were incubated for 24 hours at 37° C, 5% CO₂. For cells to be treated with CNP variants, plates were pre-treated with IBMX (Enzo life sciences, 89161-340, 1g) 15 minutes prior to use. IBMX is a potent, non-specific inhibitor of phosphodiesterases. An 800 mM stock solution of IBMX is diluted in IBMX dilution media (serum-free media (DMEM + 1x PBS mixed 1:1 with 1x PBS) to a 0.75 mM working stock.

For cell treatment, cells were removed from the incubator, growth media was removed from cells and cells treated with IBMX. 80 µL 0.75 mM IBMX was added to each well and cells returned to 37 °C incubator for 15 minutes. After 15 minutes CNP (40 µL/well) is added to each test well and cells returned to 37° C incubator for 15 minutes. The plate was mixed by gentle tapping and imaged on a Solentim cell metric to visualize cells and determine if there is any cell lifting and then placed back into 37° C incubator.

The reaction was stopped and cells lysed by adding 40 µL lysis buffer (from cGMP kit). Plate was placed on a shaker for 5 minutes to complete lysis. The cell lysate was used in the cGMP assay.

The cGMP assay was carried out using a cGMP calibrator, rabbit anti-cGMP antibody and HRP-cGMP prepared as according to manufacturer's protocol. 40 µL of calibrator was added to wells of an anti-cGMP antibody coated plated, and 40 µL of lysate to be analyzed added to the appropriate wells. 40 µL of reconstituted rabbit anti-cGMP antibody was added to all wells and plates placed on shaker five minutes for mixing. 40 µL of reconstituted HRP-cGMP was added to each well and incubated for 2 hours at room temperature. Plates were manually aspirated and washed 4x with 300 µL wash buffer. 100 µL of stoplight red substrate was added to each well, the plate, covered and left at room temperature for at least 10 mins, protected from light. The plate was read for fluorescence intensity on a Spectramax M, or similar instrument, at excitation 530 nm and emission at 590 nm.

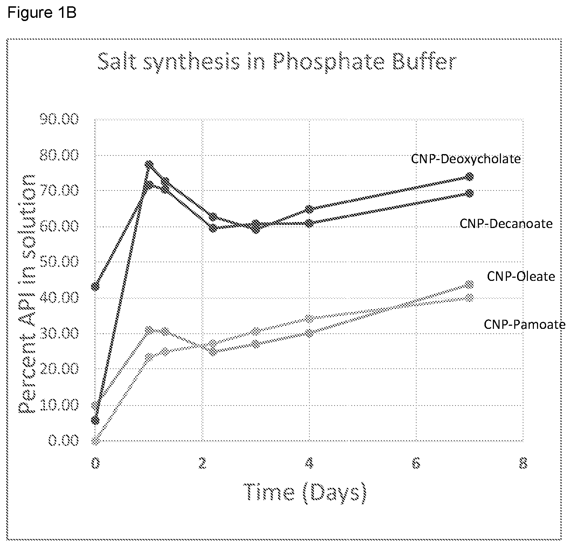

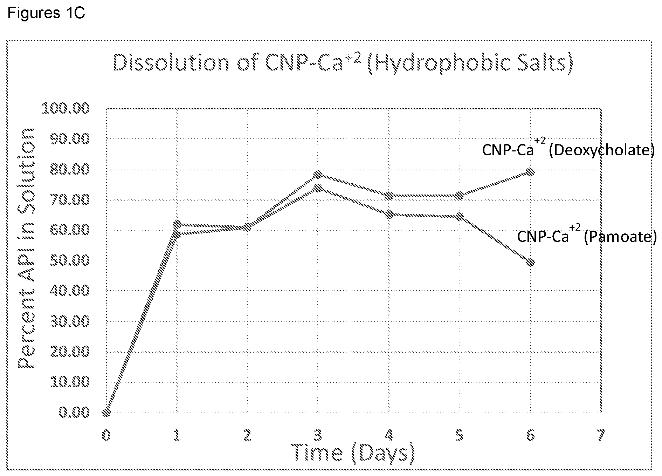

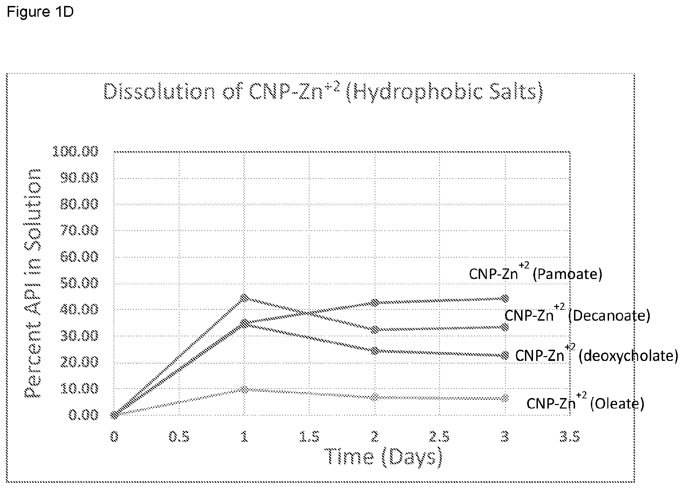

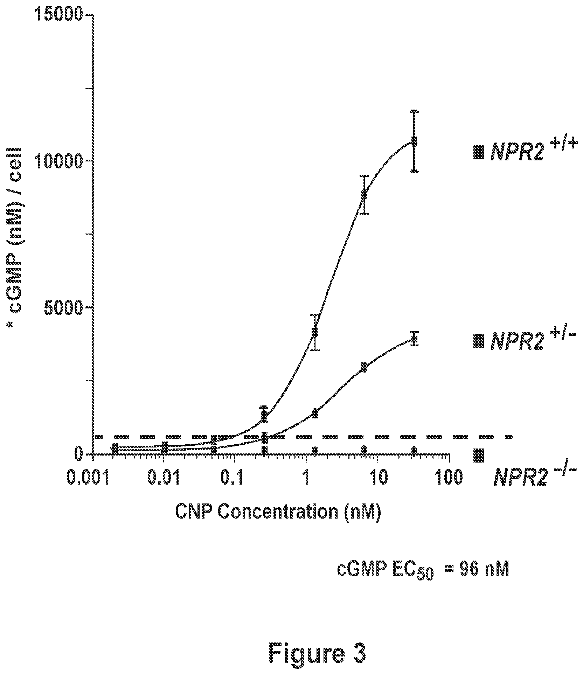

Figure 3 shows that adding exogenous Pro-Gly-CNP37 variant rescues cGMP readout in a NPR2 +/- rat chondrosarcoma cell model. Previous activation data reports cGMP EC50 in the range of 40 to 360nM for activation of PRKG2 (Campbell et al., ACS Chem Biol 12, 2388-2398, 2017); Vaandrager et al., J Biol Chem 272, 11816-23, 1997); Pohler et al., FEBS Lett 374, 419-25, 1995). In the heterozygous NPR2 knockout cells, a CNP dose >0.163nM is able to achieve an intracellular concentration exceeding the EC50 range for PRKG2 activation cGMP (Figure 1). Whereas, in wild-type cells a CNP dose of 0.040nM is able to achieve the same cGMP concentration. These results demonstrate that CNP supplementation can achieve the cGMP levels necessary for PRKG2 activation and growth in cells with loss-of-function mutations of NPR2.

These results also suggest that administration of CNP variants is useful to restore bone growth in subjects with short stature that have reduced activity of NPR2. It is further contemplated that treatment with CNP variants will be beneficial in subjects having mutations in other growth plate genes in which cGMP signaling may be impaired.

### Example 4: Identification of Mutations Associated With Short Stature

It is hypothesized that genes showing clear evidence of genetically-driven bi-directional effects are more likely to represent therapeutic targets that can be effectively modulated in a broad patient population. To identify genes that are core regulators of growth, the intersections of five gene lists were analyzed including, the list of genes from genome-wide association study (GWAS). Core growth regulators would be the most likely to contain rare coding mutations with bidirectional effects (i.e. short stature or skeletal dysplasia AND tall stature or overgrowth).

Databases queried include: *GWAS* 2,067 non-repeating closest genes for each of the 3,290 independent genetic variants reported by a large GWAS meta-analysis of height using ~700,000 individuals were extracted; *HGMD* The "allmut" table from HGMD version v2019_2 was queried looking for all pathogenic variants labelled as "DM" having either "short stature" and "tall stature or overgrowth" in the same genes; *OMIM* The list of OMIM genes related to growth disorders was previously described and was created using the keywords: short stature, overgrowth, skeletal dysplasia, brachydactyly.

First, the Human Gene Mutation Database (HGMD version v2019_2) was queried for genes associated with short or tall stature (Stenson et al., Hum Genet 136:665-677, 2017). There were 47 genes annotated with at least one pathogenic variant reported in the literature to cause "short stature". Only 20 genes were annotated as tall stature or overgrowth genes. Secondly, a manually curated list of 258 OMIM genes was used (248 short, 20 tall) which was created using the keywords: short stature, overgrowth, skeletal dysplasia, brachydactyly (Wood et al., Nat Genet 46:1173-86, 2014). Third, the intersection of these lists was compared with the list of genes from GWAS. At the intersection of these list there were three genes known to be associated with height (IGF1R, NPPC, NPR2) and two additional genes were identified (FGFR3, SHOX).

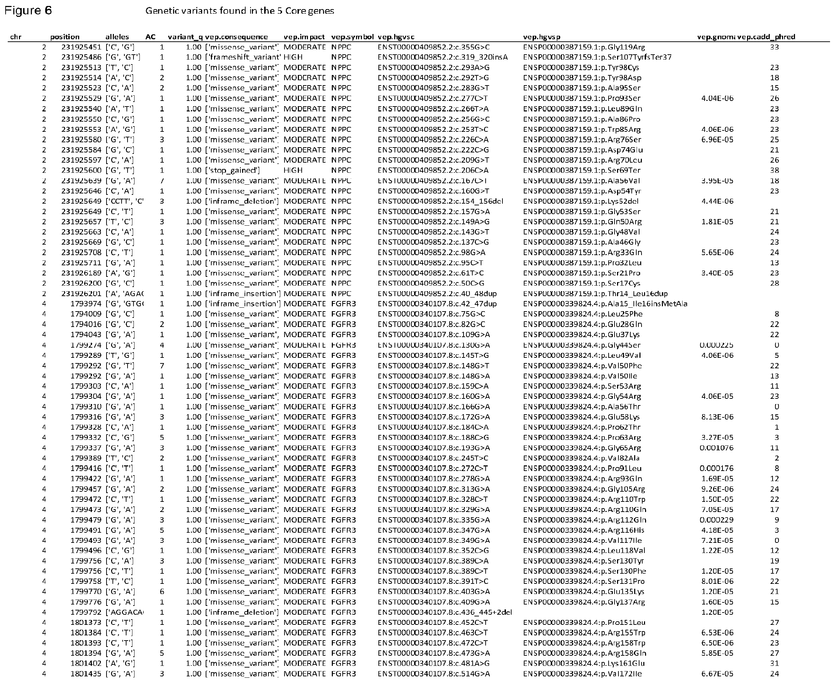

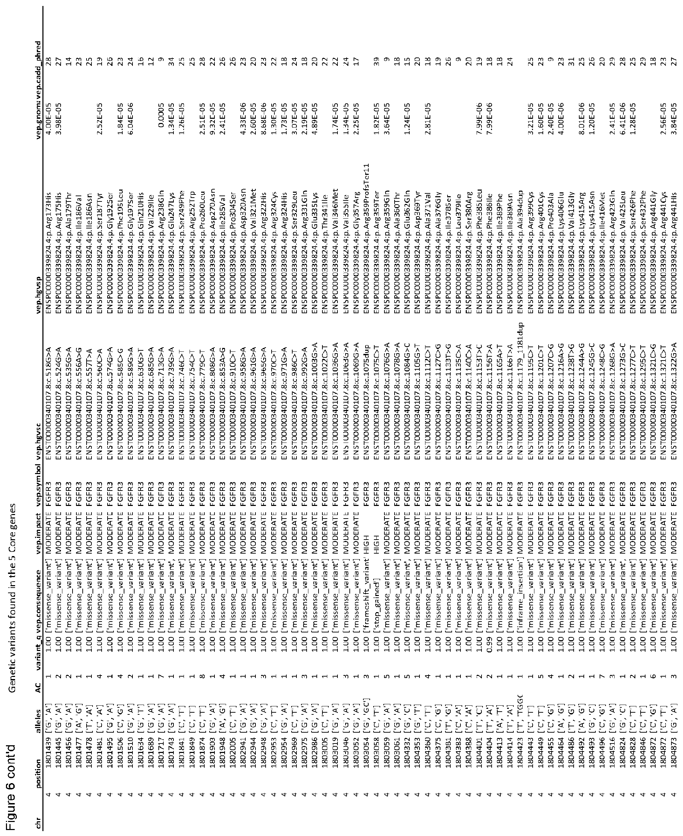

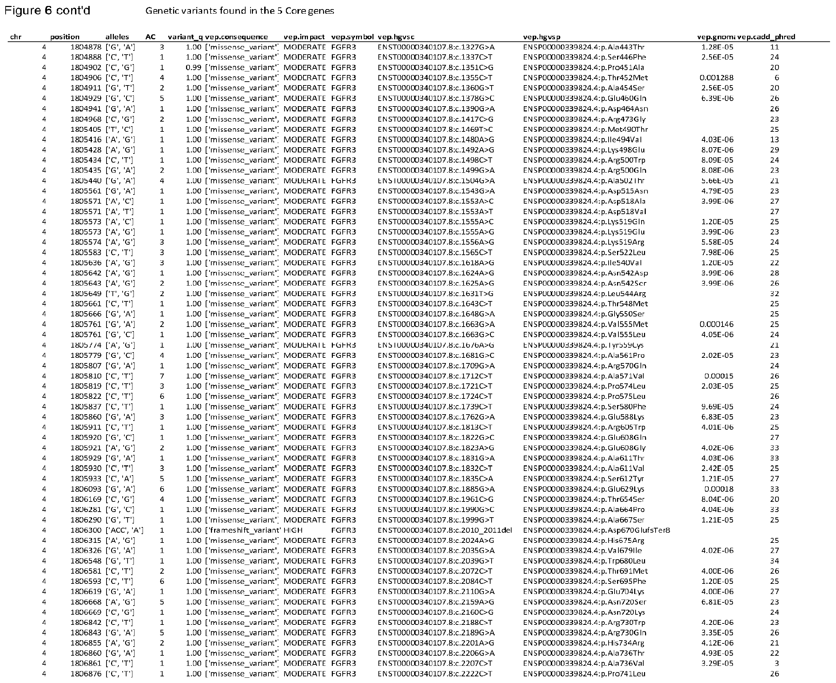

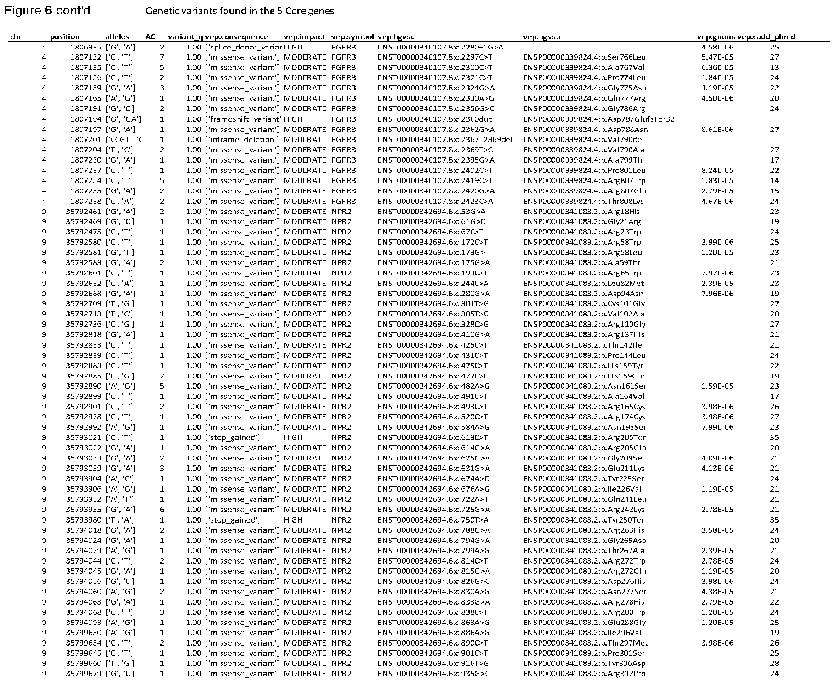

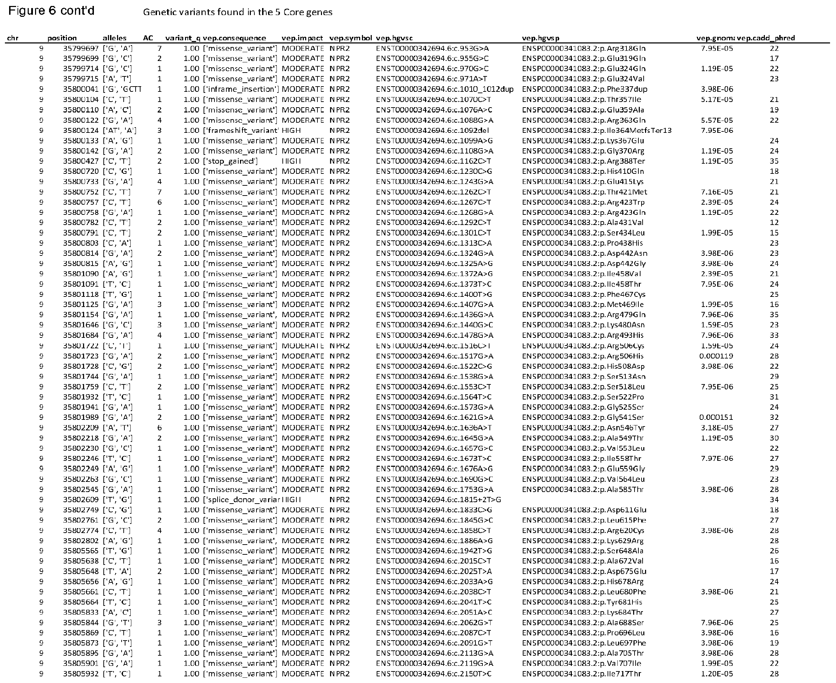

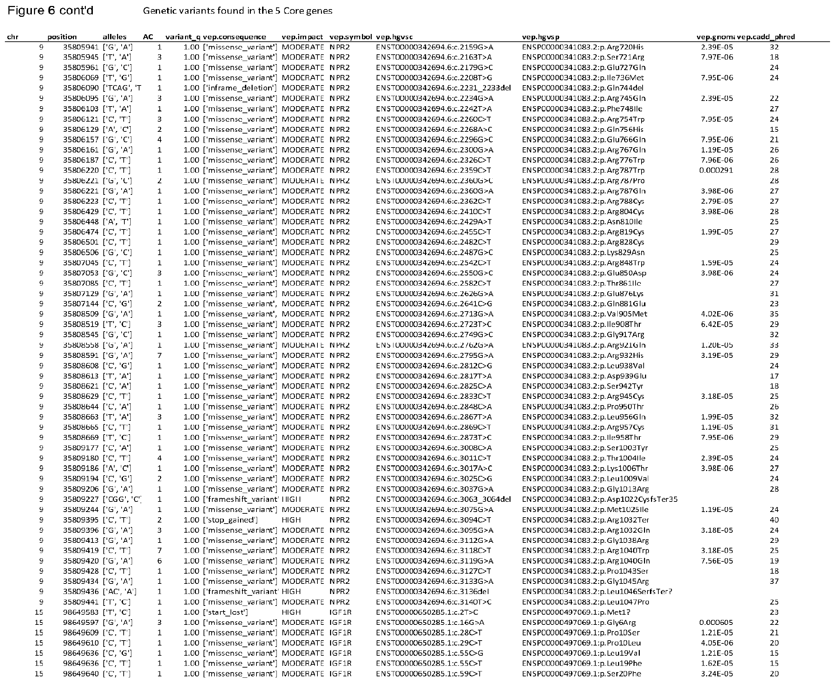

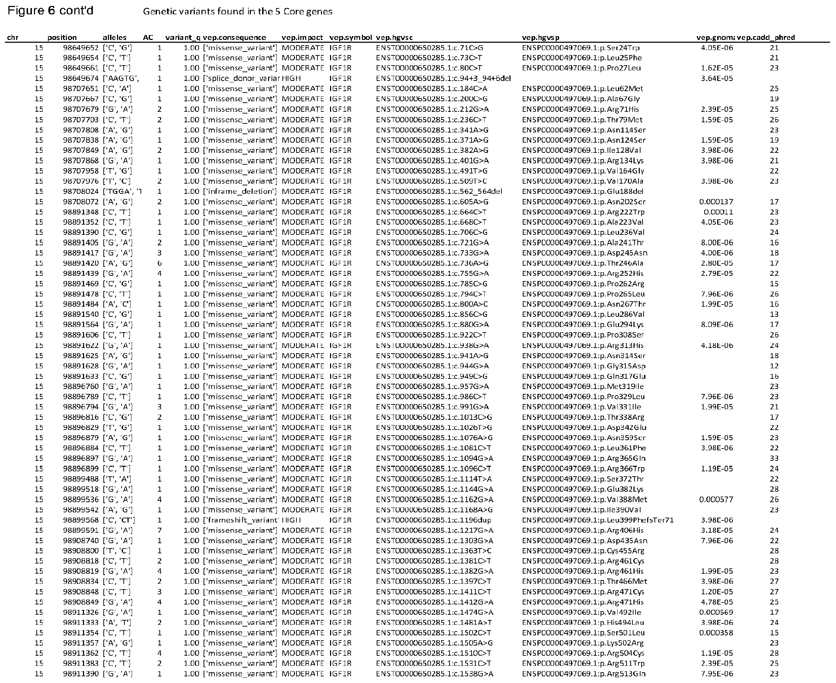

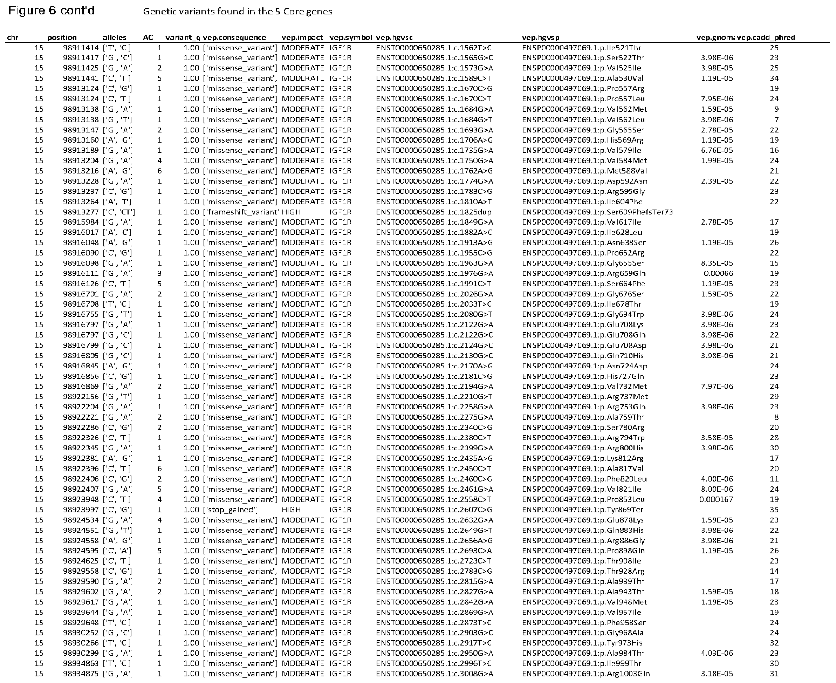

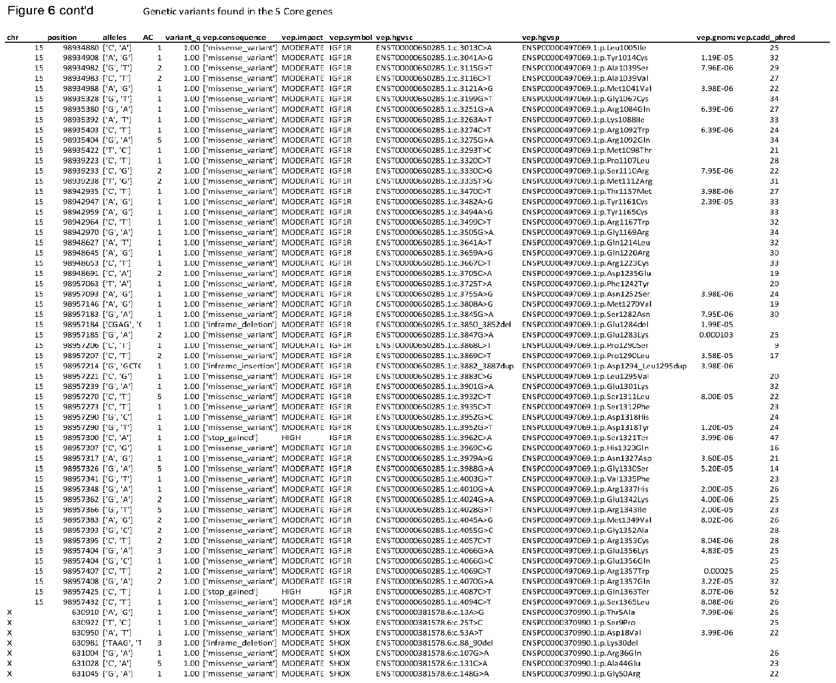

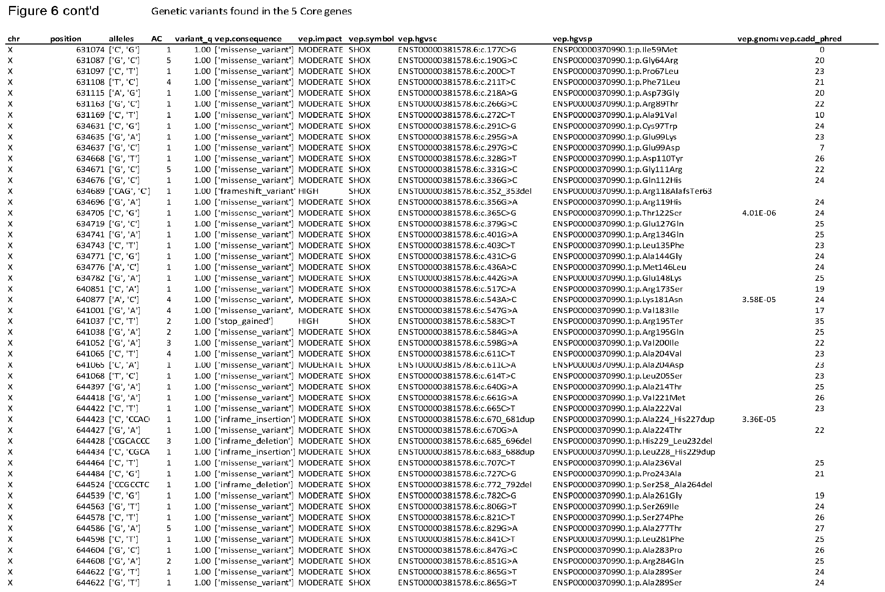

Additional analysis led to generation of a new group of five Core genes that showed significantly decreased height (β=-0.20, 95%CI [-0.26 to -0.14], p=4.04x10-11) and significantly increased risk for Idiopathic Short Stature (ISS) (OR=2.75, 95%CI [1.92 - 3.96]. Each of the core five genes (FGFR3, IGF1R, NPPC, NPR2 and SHOX) were associated with height when considered individually, and also were associated with short stature when taken in combination with other mutations. Exemplary mutations in FGFR3, IGF1R, NPPC, NPR2 and SHOX are set out in Figure 6.

Combined LoF (loss of function) and Missense variants in NPR2 and IGF1R were also associated with increased risk for ISS (OR=3.31, P= 0.001, OR= 2.85, P=0.002, respectively). Entire gene deletions and/or mutations causing loss of protein function in SHOX, IGF1R, NPPC, NPR2 have been reported in familial short stature with various degrees of severity.

Analysis shows that carriers of variants in any of the five core genes are at approximately a 3-fold increased risk for ISS and account for 6.7% of the total ISS population. Furthermore, a dose-dependent rescue of NPR2 signaling in a cell model of NPR2 haploinsufficiency after adding exogenous CNP was demonstrated.

According to the omnigenic model (Liu, et al., Cell 177:1022-1034 e6 (2019); Boyle et al., Cell 169:1177-1186 (2017)) if these genes are core human growth genes, then their effects should be modulated by multiple weaker common genetic variants driving regulatory networks. To indirectly test this hypothesis, polygenic risk scores (PRS) were calculated for height using the largest published GWAS meta-analysis for height that did not include any samples from the UK Biobank project. The cohort was divided into five equally-sized (n=6,824) PRS quintiles (PRS 1 being the lowest height, PRS 5 the tallest height). There was a dose-dependent relationship between increasing PRS score and mean height (β=0.30 per each PRS quintile increase) (Fig. 7A). Carriers of LoF variants in the five core genes were consistently shorter than non-carriers across the five different PRS backgrounds. See Figure 7. The data suggest that the combined effect of PRS and rare protein variants is consistent with an additive model: polygenic effects modulated height in both carriers and non-carriers.

The risk for ISS across PRS groups was calculated using PRS=3 as a reference. The lowest PRS group was associated with increased risk for ISS and the highest PRS group with a decreased risk (OR=5.43, P=8.58x10-34; OR=0.22, P=4.49x10-7 for PRS 1 and PRS 5 respectively). The effect of rare coding variants of the five core genes was evaluated for ISS stratified by PRS group. Carriers of any of the five core genes were at increased risk for ISS in the first three quintiles (OR=2.64, P=3.09x10-5; OR=2.17, P=0.04; OR=5.29, P=1.58x10-5; OR=2.72, P=0.09 Figs. 7C-F). Consistent directions of effect were observed for carriers of each individual core gene on ISS risk stratified by PRS (Figs. 7C-F).

Further, additive effects of PRS, mostly coming from multiple common genetic variation with individual small effects, predicted 20.1% of the variance in height in the dataset. These additive effects of PRS appeared to have similar magnitude on carriers of rare coding variation of core genes as well as in non-carriers. This observation indicates that PRS may be a strong contributor in the differences in penetrance of rare pathogenic variants (especially in models of haploinsufficiency such as the ones described here). Supporting this idea, it was observed that two of eight NPR2 variant carriers with low NPR2 activity had a low-normal height. This data suggests that most ISS individuals possessing mutations in NPR2 may also have a polygenic background that made them more susceptible to the pathogenic effect of losing NPR2 activity.

These results support the idea that CNP-based treatments could be effective in NPR2 haploinsufficient patient populations. Further, results showing a significant bi-directional (LoF and GoF) correlation of cGMP levels and height in NPR2 carriers of the general population suggest that targeting this receptor with CNP analogs could be an effective therapy for all ISS individuals.

### Example 5: Release Profiles of CNP Salts In Vitro

Release analysis of the hydrophobic CNP salts were also carried out. Briefly, Pro-Gly-CNP37 Zn Pamoate, and Pro-Gly-CNP37 Zn-oleate were made fresh and placed into a dissolution apparatus, a Pion microDiss, in 15 mL media, impeller spinning at 250 RPM, temperature control setpoint 37.4 °C, the same day it was made. Every 24 hours for 4 days, vessel contents were transferred to VWR polypropylene "Falcon" tubes, spun down at 4000 x g for 30 min, RP-UPLC samples were taken/frozen down, and solids resuspended in fresh media (15 mL). The salt was run on RP-UPLC to obtain signal, and concentration was obtained from comparison to calibration curve. Percent salt released was obtained by comparing mass released to initial mass Pro-Gly-CNP37 used in making the salt.

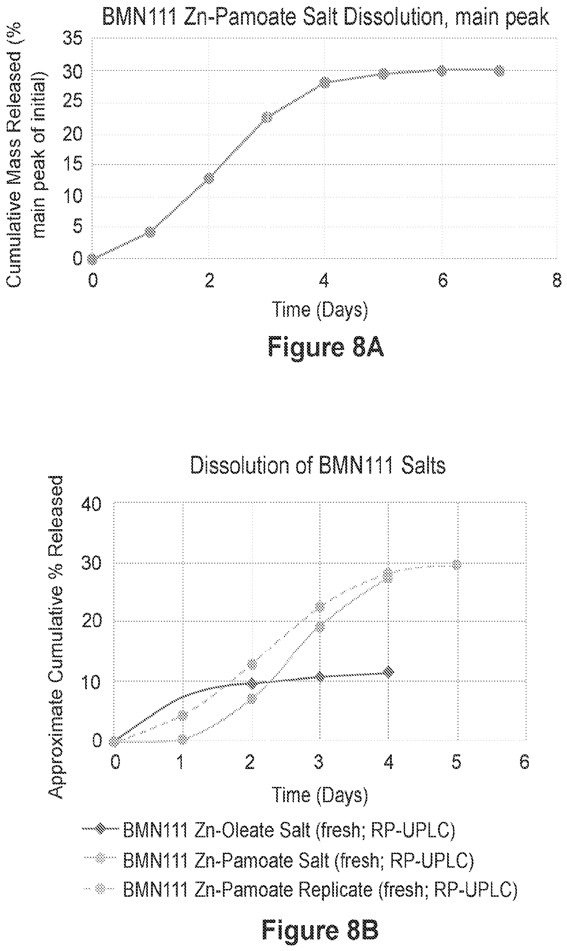

Figure 8A and Figure 8B show the cumulative release profiles of the CNP peptide salts over 7 days, as a percentage of total amount of CNP peptide salt.

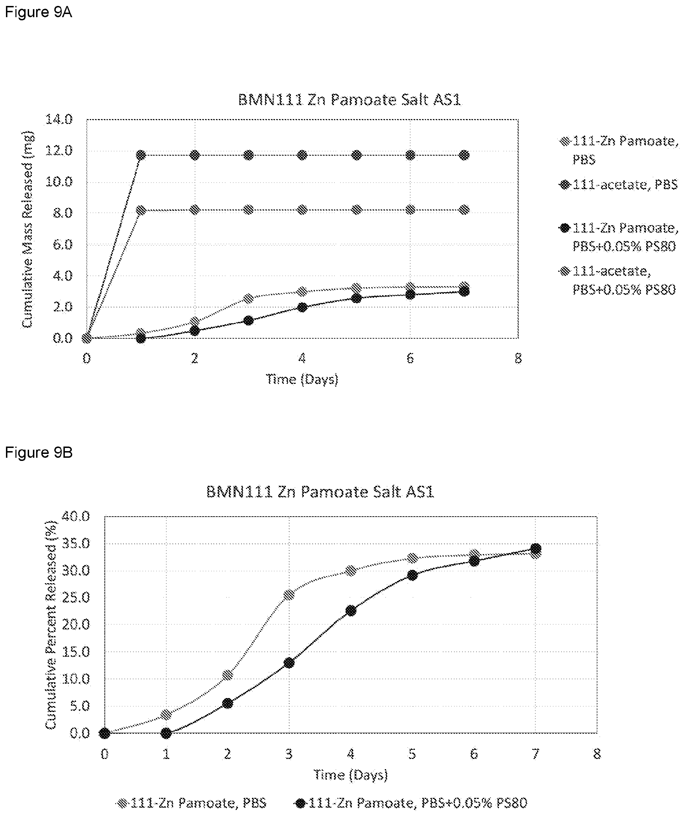

An additional release profile was run using the protocol above. ~16.6 mg of Pro-Gly-CNP37-acetate (starting material to make salts) or Pro-Gly-CNP37 Zn-Pamoate salt placed into each well. CNP salt was made, lyophilized, sealed, and stored at 4°C before use. For CNP acetate controls, vessel 1 in 1x PBS, Vessel 5 in 1x PBS+0.05% PS80. Every 24 hours for 7 days, vessel contents transferred to VWR polypropylene "Falcon" tubes, spun down at 4000 x g for 30 min, UPLC sample taken/frozen down, and solids resuspended in fresh media (15 mL). CNP salts were run on RP-UPLC to obtain signal and the concentration obtained from comparison to a calibration curve. Cumulative release profiles over 7 days are shown in Figures 9A and 9B.

Pro-Gly-CNP-37- Docusate and Pro-Gly-CNP-37-Zn-Docusate salts were made fresh, or made and lyophilized, and sealed in glass vial prior to use in Pion for Dissolution. Every 24 hours for 4 days, vessel contents transferred to VWR polypropylene "Falcon" tubes, spun down at 4000 x g for 30 min, sample taken/frozen down, and solids resuspended in fresh media (15 mL). Salts were run on LC-MS to quantify, and % salt released by comparing to initial salt placed in dissolution vessel.

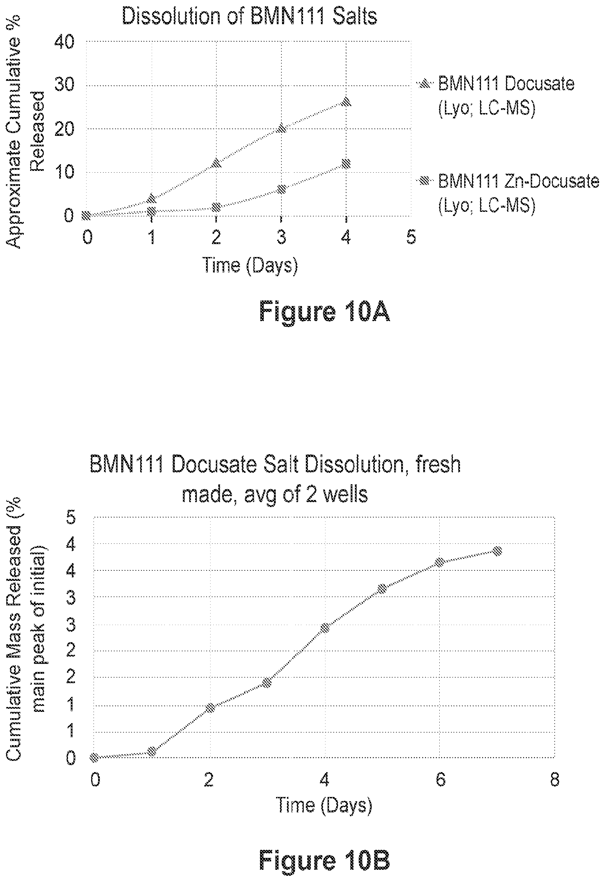

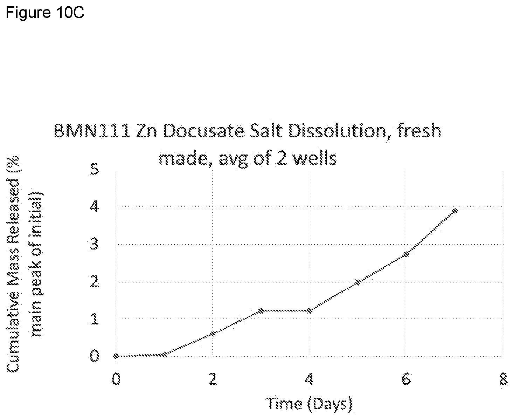

Figures 10A to 10C show the cumulative release profiles of the docusate salts over 4 days (Figure 10A) or 7 days (Figures 10B and 10C).

### Example 6: Release Profiles of CNP Salts In Vivo

The release profile of an sample salt, CNP-pamoate was then analyzed for release profiles in vivo in rats over 7 days after subcutaneous injection of the CNP salt.

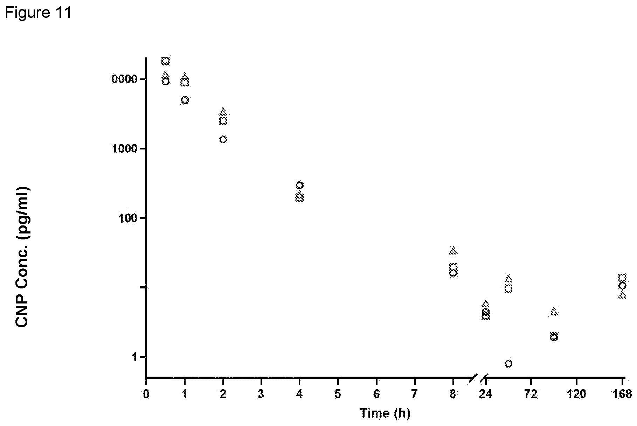

Figure 11 shows the release profile of Pro-Gly-CNP37 from the salt into plasma over 7 days. Release of the salts was observed as an initial burst over 24 hours with some salt release over time.

## Claims

1. A composition comprising a hydrophobic salt of a C-type natriuretic peptide (CNP), the salt comprising the CNP complexed with a hydrophobic counterion, wherein the CNP is selected from the group consisting of:
DLRVDTKSRAAWARLLQEHPNARKYKGANKKGLSKGCFGLKLDRIGSMSGLGC (CNP-53) (SEQ ID NO: 56);
LRVDTKSRAAWARLLQEHPNARKYKGANKKGLSKGCFGLKLDRIGSMSGLGC (CNP-52) (SEQ ID NO:15);
RVDTKSRAAWARLLQEHPNARKYKGANKKGLSKGCFGLKLDRIGSMSGLGC (CNP-51) (SEQ ID NO:16);
VDTKSRAAWARLLQEHPNARKYKGANKKGLSKGCFGLKLDRIGSMSGLGC (CNP-50) (SEQ ID NO17:);
DTKSRAAWARLLQEHPNARKYKGANKKGLSKGCFGLKLDRIGSMSGLGC (CNP-49) (SEQ ID NO: 18)
TKSRAAWARLLQEHPNARKYKGANKKGLSKGCFGLKLDRIGSMSGLGC (CNP-48) (SEQ ID NO:19);
KSRAAWARLLQEHPNARKYKGANKKGLSKGCFGLKLDRIGSMSGLGC (CNP-47) (SEQ ID NO:20);
SRAAWARLLQEHPNARKYKGANKKGLSKGCFGLKLDRIGSMSGLGC (CNP-46) (SEQ ID NO:21);
RAAWARLLQEHPNARKYKGANKKGLSKGCFGLKLDRIGSMSGLGC (CNP-45) (SEQ ID NO:22);
AAWARLLQEHPNARKYKGANKKGLSKGCFGLKLDRIGSMSGLGC (CNP-44) (SEQ ID NO:23);
AWARLLQEHPNARKYKGANKKGLSKGCFGLKLDRIGSMSGLGC (CNP-43) (SEQ ID NO:24);
WARLLQEHPNARKYKGANKKGLSKGCFGLKLDRIGSMSGLGC (CNP-42) (SEQ ID NO:25);
ARLLQEHPNARKYKGANKKGLSKGCFGLKLDRIGSMSGLGC (CNP-41) (SEQ ID NO:26);
RLLQEHPNARKYKGANKKGLSKGCFGLKLDRIGSMSGLGC (CNP-40) (SEQ ID NO:27);
LLQEHPNARKYKGANKKGLSKGCFGLKLDRIGSMSGLGC (CNP-39) (SEQ ID NO:28);
LQEHPNARKYKGANKKGLSKGCFGLKLDRIGSMSGLGC (SEQ ID NO:2);
QEHPNARKYKGANKKGLSKGCFGLKLDRIGSMSGLGC (CNP-37) (SEQ ID NO:3);
EHPNARKYKGANKKGLSKGCFGLKLDRIGSMSGLGC (CNP-36) (SEQ ID NO:29);
HPNARKYKGANKKGLSKGCFGLKLDRIGSMSGLGC (CNP-35) (SEQ ID NO:30);
PNARKYKGANKKGLSKGCFGLKLDRIGSMSGLGC (CNP-34) (SEQ ID NO:4);
NARKYKGANKKGLSKGCFGLKLDRIGSMSGLGC (CNP-33) (SEQ ID NO:31);
ARKYKGANKKGLSKGCFGLKLDRIGSMSGLGC (CNP-32) (SEQ ID NO:32);
RKYKGANKKGLSKGCFGLKLDRIGSMSGLGC (CNP-31) (SEQ ID NO:33);
KYKGANKKGLSKGCFGLKLDRIGSMSGLGC (CNP-30) (SEQ ID NO:34);
YKGANKKGLSKGCFGLKLDRIGSMSGLGC (CNP-29) (SEQ ID NO:35);
KGANKKGLSKGCFGLKLDRIGSMSGLGC (CNP-28) (SEQ ID NO:36);
GANKKGLSKGCFGLKLDRIGSMSGLGC (CNP-27) (SEQ ID NO:37);
ANKKGLSKGCFGLKLDRIGSMSGLGC (CNP-26) (SEQ ID NO:38);
NKKGLSKGCFGLKLDRIGSMSGLGC (CNP-25) (SEQ ID NO:39);
KKGLSKGCFGLKLDRIGSMSGLGC (CNP-24) (SEQ ID NO:40);
KGLSKGCFGLKLDRIGSMSGLGC (CNP-23) (SEQ ID NO:41);
GLSKGCFGLKLDRIGSMSGLGC (CNP-22) (SEQ ID NO: 68);
LSKGCFGLKLDRIGSMSGLGC (CNP-21) (SEQ ID NO:42);
SKGCFGLKLDRIGSMSGLGC (CNP-20) (SEQ ID NO:43);
KGCFGLKLDRIGSMSGLGC (CNP-19) (SEQ ID NO:44);
GCFGLKLDRIGSMSGLGC (CNP-18) (SEQ ID NO:45);
CFGLKLDRIGSMSGLGC (CNP-17) (SEQ ID NO: 67),
QEHPNARKYKGANKKGLSKGCFGLKLDRIGSNSGLGC [CNP-37(M32N); SEQ ID NO: 46];
MQEHPNARKYKGANKKGLSKGCFGLKLDRIGSMSGLGC (Met-CNP-37; SEQ ID NO:47);
PQEHPNARKYKGANKKGLSKGCFGLKLDRIGSMSGLGC (Pro-CNP-37; SEQ ID NO: 48);
GQEHPNARKYKGANKKGLSKGCFGLKLDRIGSNSGLGC [Gly-CNP-37 (M32N); SEQ ID NO: 49];
MGQEHPNARKYKGANKKGLSKGCFGLKLDRIGSMSGLGC (Met-Gly-CNP-37; SEQ ID NO: 50);
GQEHPNARKYKGANKKGLSKGCFGLKLDRIGSMSGLGC (Gly-CNP-37: SEQ ID NO:51)
GQEHPNARKYKGANPKGLSKGCFGLKLDRIGSMSGLGC (SEQ ID NO:52);
GQEHPNARKYKGANQKGLSKGCFGLKLDRIGSMSGLGC (SEQ ID NO:53);
GQEHPNARKYKGANQQGLSKGCFGLKLDRIGSMSGLGC (SEQ ID NO:54);
GQEHPNARKYKGANKPGLSKGCFGLKLDRIGSMSGLGC (SEQ ID NO:55),
PGQEHPNARKYKGANKKGLSKGCFGLKLDRIGSMSGLGC(SEQ ID NO: 1);
PGQEHPNARRYRGANRRGLSRGCFGLKLDRIGSMSGLGC(SEQ ID NO: 6);
PGQEHPQARRYRGAQRRGLSRGCFGLKLDRIGSMSGLGC(SEQ ID NO: 5); and
PGQEHPQARKYKGAQKKGLSKGCFGLKLDRIGSMSGLGC(SEQ ID NO: 7), optionally wherein the salt further comprises a polyvalent cation complexed to the peptide-counterion complex.

2. The composition of claim 1, wherein the CNP, hydrophobic counterion, and cation are complexed via a non-covalent bond.

3. The composition of any one of claims 1 to 2 wherein the cation is a metal cation, optionally wherein the cation comprises a metal selected from the group consisting of beryllium (Be), magnesium (Mg), calcium (Ca), strontium (Sr), barium (Ba), zinc (Zn), cadmium (Cd), boron (B), aluminum (Al), gallium (Ga), indium (In), thallium (TI), Iron (Fe), Manganese (Mn), Cobalt (Co), Nickel (Ni), Titanium (Ti), Vanadium (V), platinum (Pt), copper (Cu) and Gold (Au).

4. The composition of any one of claims 1-3, wherein the hydrophobic counterion is selected from the group consisting of a deprotonated fatty acid, a deprotonated bile acid, an ionic surfactant, naphthoate and derivatives thereof, nicotinate and derivatives thereof, an alkyl sulfonate, a dialkyl sulfosuccinate, a phospholipid, an alkyl sulfonate, an aryl sulfonate, an alkylbenzene sulfonate, an alkyl sulfate, an aryl sulfate, a dextran sulfate, an alkylbenzene sulfate, and a combination of any of the foregoing, optionally wherein the hydrophobic counterion is selected from the group consisting of palmitate, deoxycholate, oleate, pamoate, nicotinate, dodecyl sulfate, docusate, myristate, stearate, phosphatidylethanolamine (PE), phosphatidylcholine (PC), phosphatidylserine (PS), phosphatidylinositol (PL), phosphatidate, decanoate, 2-naphthalenesulfonate, 1-heptanesulfonate, 1-octanesulfonate monohydrate, 1-decanesulfonate, dodecyl sulfate, dextran sulfate, and dodecyl benzenesulfonate.

5. The composition of any one of claims 1 to 4 wherein the CNP is a CNP variant, optionally wherein the CNP is selected from the group consisting of:
PGQEHPNARKYKGANKKGLSKGCFGLKLDRIGSMSGLGC (Pro-Gly-CNP-37; SEQ ID NO: 1)
LQEHPNARKYKGANKKGLSKGCFGLKLDRIGSMSGLGC (SEQ ID NO:2);
QEHPNARKYKGANKKGLSKGCFGLKLDRIGSMSGLGC (CNP-37) (SEQ ID NO:3);
PNARKYKGANKKGLSKGCFGLKLDRIGSMSGLGC (CNP-34) (SEQ ID NO:4), and salts thereof.

6. The composition of any one of claims 1 to 5 further comprising an excipient, diluent or carrier.

7. A sterile pharmaceutical composition comprising the composition of any one of claims 1-6.

8. An extended release composition comprising a salt of a C-type natriuretic peptide (CNP), the salt comprising the electrostatically charged peptide complexed with a hydrophobic counterion, optionally wherein the salt further comprises a cation complexed to the peptide-counterion complex, wherein the CNP is selected from the group consisting of:
DLRVDTKSRAAWARLLQEHPNARKYKGANKKGLSKGCFGLKLDRIGSMSGLGC (CNP-53) (SEQ ID NO: 56);
LRVDTKSRAAWARLLQEHPNARKYKGANKKGLSKGCFGLKLDRIGSMSGLGC (CNP-52) (SEQ ID NO:15);
RVDTKSRAAWARLLQEHPNARKYKGANKKGLSKGCFGLKLDRIGSMSGLGC (CNP-51) (SEQ ID NO:16);
VDTKSRAAWARLLQEHPNARKYKGANKKGLSKGCFGLKLDRIGSMSGLGC (CNP-50) (SEQ ID NO17:);
DTKSRAAWARLLQEHPNARKYKGANKKGLSKGCFGLKLDRIGSMSGLGC (CNP-49) (SEQ ID NO: 18)
TKSRAAWARLLQEHPNARKYKGANKKGLSKGCFGLKLDRIGSMSGLGC (CNP-48) (SEQ ID NO:19);
KSRAAWARLLQEHPNARKYKGANKKGLSKGCFGLKLDRIGSMSGLGC (CNP-47) (SEQ ID NO:20);
SRAAWARLLQEHPNARKYKGANKKGLSKGCFGLKLDRIGSMSGLGC (CNP-46) (SEQ ID NO:21);
RAAWARLLQEHPNARKYKGANKKGLSKGCFGLKLDRIGSMSGLGC (CNP-45) (SEQ ID NO:22);
AAWARLLQEHPNARKYKGANKKGLSKGCFGLKLDRIGSMSGLGC (CNP-44) (SEQ ID NO:23);
AWARLLQEHPNARKYKGANKKGLSKGCFGLKLDRIGSMSGLGC (CNP-43) (SEQ ID NO:24);
WARLLQEHPNARKYKGANKKGLSKGCFGLKLDRIGSMSGLGC (CNP-42) (SEQ ID NO:25);
ARLLQEHPNARKYKGANKKGLSKGCFGLKLDRIGSMSGLGC (CNP-41) (SEQ ID NO:26);
RLLQEHPNARKYKGANKKGLSKGCFGLKLDRIGSMSGLGC (CNP-40) (SEQ ID NO:27);
LLQEHPNARKYKGANKKGLSKGCFGLKLDRIGSMSGLGC (CNP-39) (SEQ ID NO:28);
LQEHPNARKYKGANKKGLSKGCFGLKLDRIGSMSGLGC (SEQ ID NO:2);
QEHPNARKYKGANKKGLSKGCFGLKLDRIGSMSGLGC (CNP-37) (SEQ ID NO:3);
EHPNARKYKGANKKGLSKGCFGLKLDRIGSMSGLGC (CNP-36) (SEQ ID NO:29);
HPNARKYKGANKKGLSKGCFGLKLDRIGSMSGLGC (CNP-35) (SEQ ID NO:30);
PNARKYKGANKKGLSKGCFGLKLDRIGSMSGLGC (CNP-34) (SEQ ID NO:4);
NARKYKGANKKGLSKGCFGLKLDRIGSMSGLGC (CNP-33) (SEQ ID NO:31);
ARKYKGANKKGLSKGCFGLKLDRIGSMSGLGC (CNP-32) (SEQ ID NO:32);
RKYKGANKKGLSKGCFGLKLDRIGSMSGLGC (CNP-31) (SEQ ID NO:33);
KYKGANKKGLSKGCFGLKLDRIGSMSGLGC (CNP-30) (SEQ ID NO:34);
YKGANKKGLSKGCFGLKLDRIGSMSGLGC (CNP-29) (SEQ ID NO:35);
KGANKKGLSKGCFGLKLDRIGSMSGLGC (CNP-28) (SEQ ID NO:36);
GANKKGLSKGCFGLKLDRIGSMSGLGC (CNP-27) (SEQ ID NO:37);
ANKKGLSKGCFGLKLDRIGSMSGLGC (CNP-26) (SEQ ID NO:38);
NKKGLSKGCFGLKLDRIGSMSGLGC (CNP-25) (SEQ ID NO:39);
KKGLSKGCFGLKLDRIGSMSGLGC (CNP-24) (SEQ ID NO:40);
KGLSKGCFGLKLDRIGSMSGLGC (CNP-23) (SEQ ID NO:41);
GLSKGCFGLKLDRIGSMSGLGC (CNP-22) (SEQ ID NO: 68);
LSKGCFGLKLDRIGSMSGLGC (CNP-21) (SEQ ID NO:42);
SKGCFGLKLDRIGSMSGLGC (CNP-20) (SEQ ID NO:43);
KGCFGLKLDRIGSMSGLGC (CNP-19) (SEQ ID NO:44);
GCFGLKLDRIGSMSGLGC (CNP-18) (SEQ ID NO:45);
CFGLKLDRIGSMSGLGC (CNP-17) (SEQ ID NO: 67),
QEHPNARKYKGANKKGLSKGCFGLKLDRIGSNSGLGC [CNP-37(M32N); SEQ ID NO: 46];
MQEHPNARKYKGANKKGLSKGCFGLKLDRIGSMSGLGC (Met-CNP-37; SEQ ID NO:47);
PQEHPNARKYKGANKKGLSKGCFGLKLDRIGSMSGLGC (Pro-CNP-37; SEQ ID NO: 48);
GQEHPNARKYKGANKKGLSKGCFGLKLDRIGSNSGLGC [Gly-CNP-37 (M32N); SEQ ID NO: 49];
MGQEHPNARKYKGANKKGLSKGCFGLKLDRIGSMSGLGC (Met-Gly-CNP-37; SEQ ID NO: 50);
GQEHPNARKYKGANKKGLSKGCFGLKLDRIGSMSGLGC (Gly-CNP-37: SEQ ID NO:51)
GQEHPNARKYKGANPKGLSKGCFGLKLDRIGSMSGLGC (SEQ ID NO:52);
GQEHPNARKYKGANQKGLSKGCFGLKLDRIGSMSGLGC (SEQ ID NO:53);
GQEHPNARKYKGANQQGLSKGCFGLKLDRIGSMSGLGC (SEQ ID NO:54);
GQEHPNARKYKGANKPGLSKGCFGLKLDRIGSMSGLGC (SEQ ID NO:55),
PGQEHPNARKYKGANKKGLSKGCFGLKLDRIGSMSGLGC(SEQ ID NO: 1);
PGQEHPNARRYRGANRRGLSRGCFGLKLDRIGSMSGLGC(SEQ ID NO: 6);
PGQEHPQARRYRGAQRRGLSRGCFGLKLDRIGSMSGLGC(SEQ ID NO: 5); and
PGQEHPQARKYKGAQKKGLSKGCFGLKLDRIGSMSGLGC(SEQ ID NO: 7).

9. The extended release composition of claim 8 wherein the peptide salt is resuspended in an aqueous solution or in oil, optionally wherein the oil comprises a triglyceride or a fatty acid, optionally wherein the fatty acid is saturated or unsaturated, optionally wherein the fatty acid is a C-6 to C-20 fatty acid, optionally wherein the fatty acid is hexanoic acid, octanoic acid, decanoic acid, or dodecanoic acid.

10. The extended release composition of any one of claims 8 to 9, wherein
(i) less than 20% of peptide is released by day 1; and
(ii) about 90% of peptide is released by day 7, or about 90% of peptide is released by day 14, or about 90% of peptide is released by day 31,
at pH 7 to 7.6.

11. A composition comprising a hydrophobic CNP salt of any one of claims 1 to 10 for use in treating a bone growth disorder or skeletal dysplasia, or for elongating a bone or increasing long bone growth.

12. The composition for use according to claim 11, wherein the bone growth disorder or skeletal dysplasia is selected from the group consisting of osteoarthritis, hypophosphatemic rickets, achondroplasia, hypochondroplasia, short stature, dwarfism, osteochondrodysplasias, thanatophoric dysplasia, osteogenesis imperfecta, achondrogenesis, chondrodysplasia punctata, homozygous achondroplasia, chondrodysplasia punctata, camptomelic dysplasia, congenital lethal hypophosphatasia, perinatal lethal type of osteogenesis imperfecta, short-rib polydactyly syndromes, hypochondroplasia, rhizomelic type of chondrodysplasia punctata, Jansen-type metaphyseal dysplasia, spondyloepiphyseal dysplasia congenita, atelosteogenesis, diastrophic dysplasia, congenital short femur, Langer-type mesomelic dysplasia, Nievergelt-type mesomelic dysplasia, Robinow syndrome, Reinhardt syndrome, acrodysostosis, peripheral dysostosis, Kniest dysplasia, fibrochondrogenesis, Roberts syndrome, acromesomelic dysplasia, micromelia, Morquio syndrome, Kniest syndrome, metatrophic dysplasia, and spondyloepimetaphyseal dysplasia, NPR2 mutation, SHOX mutation (Turner's syndrome/Leri Weill), PTPN11 mutations (Noonan's syndrome), insulin growth factor 1 receptor (IGF1R) mutation, and idiopathic short stature.

13. The composition for use according to any one of claims 11 or 12, wherein the composition is administered subcutaneously, intradermally, intraarticularly, orally, or intramuscularly, optionally wherein the composition is administered once daily, once weekly, once every two weeks, once every three weeks, once every 4 weeks, once every 6 weeks, once every two months, once every three months or once every six months.

14. A hydrophobic salt of C-type natriuretic peptide (CNP) comprising a CNP in complex with a hydrophobic counterion, optionally further comprising a cation complexed with the peptide and counterion, optionally wherein the hydrophobic counterion is selected from the group consisting of oleate, deoxycholate, decanoate, pamoate, docusate or dodecyl sulfate
wherein the CNP is selected from the group consisting of:
DLRVDTKSRAAWARLLQEHPNARKYKGANKKGLSKGCFGLKLDRIGSMSGLGC (CNP-53) (SEQ ID NO: 56);
LRVDTKSRAAWARLLQEHPNARKYKGANKKGLSKGCFGLKLDRIGSMSGLGC (CNP-52) (SEQ ID NO:15);
RVDTKSRAAWARLLQEHPNARKYKGANKKGLSKGCFGLKLDRIGSMSGLGC (CNP-51) (SEQ ID NO:16);
VDTKSRAAWARLLQEHPNARKYKGANKKGLSKGCFGLKLDRIGSMSGLGC (CNP-50) (SEQ ID NO17:);
DTKSRAAWARLLQEHPNARKYKGANKKGLSKGCFGLKLDRIGSMSGLGC (CNP-49) (SEQ ID NO: 18)
TKSRAAWARLLQEHPNARKYKGANKKGLSKGCFGLKLDRIGSMSGLGC (CNP-48) (SEQ ID NO:23);
KSRAAWARLLQEHPNARKYKGANKKGLSKGCFGLKLDRIGSMSGLGC (CNP-47) (SEQ ID NO:24);
SRAAWARLLQEHPNARKYKGANKKGLSKGCFGLKLDRIGSMSGLGC (CNP-46) (SEQ ID NO:25);
RAAWARLLQEHPNARKYKGANKKGLSKGCFGLKLDRIGSMSGLGC (CNP-45) (SEQ ID NO:26);
AAWARLLQEHPNARKYKGANKKGLSKGCFGLKLDRIGSMSGLGC (CNP-44) (SEQ ID NO:23);
AWARLLQEHPNARKYKGANKKGLSKGCFGLKLDRIGSMSGLGC (CNP-43) (SEQ ID NO:24);
WARLLQEHPNARKYKGANKKGLSKGCFGLKLDRIGSMSGLGC (CNP-42) (SEQ ID NO:25);
ARLLQEHPNARKYKGANKKGLSKGCFGLKLDRIGSMSGLGC (CNP-41) (SEQ ID NO:26);
RLLQEHPNARKYKGANKKGLSKGCFGLKLDRIGSMSGLGC (CNP-40) (SEQ ID NO:27);
LLQEHPNARKYKGANKKGLSKGCFGLKLDRIGSMSGLGC (CNP-39) (SEQ ID NO:28);
LQEHPNARKYKGANKKGLSKGCFGLKLDRIGSMSGLGC (SEQ ID NO:2);
QEHPNARKYKGANKKGLSKGCFGLKLDRIGSMSGLGC (CNP-37) (SEQ ID NO:3);
EHPNARKYKGANKKGLSKGCFGLKLDRIGSMSGLGC (CNP-36) (SEQ ID NO:29);
HPNARKYKGANKKGLSKGCFGLKLDRIGSMSGLGC (CNP-35) (SEQ ID NO:30);
PNARKYKGANKKGLSKGCFGLKLDRIGSMSGLGC (CNP-34) (SEQ ID NO:4);
NARKYKGANKKGLSKGCFGLKLDRIGSMSGLGC (CNP-33) (SEQ ID NO:31);
ARKYKGANKKGLSKGCFGLKLDRIGSMSGLGC (CNP-32) (SEQ ID NO:32);
RKYKGANKKGLSKGCFGLKLDRIGSMSGLGC (CNP-31) (SEQ ID NO:33);
KYKGANKKGLSKGCFGLKLDRIGSMSGLGC (CNP-30) (SEQ ID NO:34);
YKGANKKGLSKGCFGLKLDRIGSMSGLGC (CNP-29) (SEQ ID NO:35);
KGANKKGLSKGCFGLKLDRIGSMSGLGC (CNP-28) (SEQ ID NO:36);
GANKKGLSKGCFGLKLDRIGSMSGLGC (CNP-27) (SEQ ID NO:37);
ANKKGLSKGCFGLKLDRIGSMSGLGC (CNP-26) (SEQ ID NO:38);
NKKGLSKGCFGLKLDRIGSMSGLGC (CNP-25) (SEQ ID NO:39);
KKGLSKGCFGLKLDRIGSMSGLGC (CNP-24) (SEQ ID NO:40);
KGLSKGCFGLKLDRIGSMSGLGC (CNP-23) (SEQ ID NO:41);
GLSKGCFGLKLDRIGSMSGLGC (CNP-22) (SEQ ID NO: 68);
LSKGCFGLKLDRIGSMSGLGC (CNP-21) (SEQ ID NO:42);
SKGCFGLKLDRIGSMSGLGC (CNP-20) (SEQ ID NO:43);
KGCFGLKLDRIGSMSGLGC (CNP-19) (SEQ ID NO:44);
GCFGLKLDRIGSMSGLGC (CNP-18) (SEQ ID NO:45);
CFGLKLDRIGSMSGLGC (CNP-17) (SEQ ID NO: 67),
QEHPNARKYKGANKKGLSKGCFGLKLDRIGSNSGLGC [CNP-37(M32N); SEQ ID NO: 46];
MQEHPNARKYKGANKKGLSKGCFGLKLDRIGSMSGLGC (Met-CNP-37; SEQ ID NO:47);
PQEHPNARKYKGANKKGLSKGCFGLKLDRIGSMSGLGC (Pro-CNP-37; SEQ ID NO: 48);
GQEHPNARKYKGANKKGLSKGCFGLKLDRIGSNSGLGC [Gly-CNP-37 (M32N); SEQ ID NO: 49];
MGQEHPNARKYKGANKKGLSKGCFGLKLDRIGSMSGLGC (Met-Gly-CNP-37; SEQ ID NO: 50);
GQEHPNARKYKGANKKGLSKGCFGLKLDRIGSMSGLGC (Gly-CNP-37: SEQ ID NO:51)
GQEHPNARKYKGANPKGLSKGCFGLKLDRIGSMSGLGC (SEQ ID NO:52);
GQEHPNARKYKGANQKGLSKGCFGLKLDRIGSMSGLGC (SEQ ID NO:53);
GQEHPNARKYKGANQQGLSKGCFGLKLDRIGSMSGLGC (SEQ ID NO:54);
GQEHPNARKYKGANKPGLSKGCFGLKLDRIGSMSGLGC (SEQ ID NO:55),
PGQEHPNARKYKGANKKGLSKGCFGLKLDRIGSMSGLGC(SEQ ID NO: 1);
PGQEHPNARRYRGANRRGLSRGCFGLKLDRIGSMSGLGC(SEQ ID NO: 6);
PGQEHPQARRYRGAQRRGLSRGCFGLKLDRIGSMSGLGC(SEQ ID NO: 5); and
PGQEHPQARKYKGAQKKGLSKGCFGLKLDRIGSMSGLGC(SEQ ID NO: 7).

15. The hydrophobic salt of claim 14, wherein the salt is selected from the group consisting of CNP-Ca⁺²(Oleate), CNP-Ca⁺²(Pamoate), CNP-Ca⁺²(deoxycholate), CNP-Ca⁺²(decanoate), CNP-Ca⁺²(docusate), CNP-Zn⁺²(Oleate), CNP-Zn⁺²(Pamoate), CNP-Zn⁺²(deoxycholate), CNP-Zn⁺²(decanoate), and CNP-Zn⁺²(docusate).

16. The hydrophobic salt of any one of claims 14 to 15, wherein the CNP is selected from the group consisting of:
PGQEHPNARKYKGANKKGLSKGCFGLKLDRIGSMSGLGC (Pro-Gly-CNP-37; SEQ ID NO: 1)
LQEHPNARKYKGANKKGLSKGCFGLKLDRIGSMSGLGC (SEQ ID NO:2);
QEHPNARKYKGANKKGLSKGCFGLKLDRIGSMSGLGC (CNP-37) (SEQ ID NO:3);
PNARKYKGANKKGLSKGCFGLKLDRIGSMSGLGC (CNP-34) (SEQ ID NO:4), and salts thereof.

## Patentansprüche

1. Zusammensetzung, umfassend ein hydrophobes Salz eines CNP (C-type Natriuretic Peptide), wobei das Salz das CNP im Komplex mit einem hydrophoben Gegenion umfasst, wobei das CNP ausgewählt ist aus der Gruppe bestehend aus:
DLRVDTKSRAAWARLLQEHPNARKYKGANKKGLSKGCFGLKLDRIGSMSGLGC (CNP-53) (SEQ ID NO: 56);
LRVDTKSRAAWARLLQEHPNARKYKGANKKGLSKGCFGLKLDRIGSMSGLGC (CNP-52) (SEQ ID NO:15);
RVDTKSRAAWARLLQEHPNARKYKGANKKGLSKGCFGLKLDRIGSMSGLGC (CNP-51) (SEQ ID NO:16);
VDTKSRAA WARLLQEH PNARKYKGAN KKGLSKGCFG LKLDRIGSMSG LGC (CNP-50) (SEQ ID NO17:);
DTKSRAAWARLLQEHPNARKYKGANKKGLSKGCFGLKLDRIGSMSGLGC (CNP-49) (SEQ ID NO: 18)
TKSRAAWARLLQEHPNARKYKGANKKGLSKGCFGLKLDRIGSMSGLGC (CNP-48) (SEQ ID NO:19);
KSRAAWARLLQEHPNARKYKGANKKGLSKGCFGLKLDRIGSMSGLGC (CNP-47) (SEQ ID NO:20);
SRAAWARLLQEHPNARKYKGANKKGLSKGCFGLKLDRIGSMSGLGC (CNP-46) (SEQ ID NO:21);
RAAWARLLQEHPNARKYKGANKKGLSKGCFGLKLDRIGSMSGLGC (CNP-45) (SEQ ID NO:22);
AAWARLLQEHPNARKYKGANKKGLSKGCFGLKLDRIGSMSGLGC (CNP-44) (SEQ ID NO:23);
AWARLLQEHPNARKYKGANKKGLSKGCFGLKLDRIGSMSGLGC (CNP-43) (SEQ ID NO:24);
WARLLQEHPNARKYKGANKKGLSKGCFGLKLDRIGSMSGLGC (CNP-42) (SEQ ID NO:25);
ARLLQEHPNARKYKGANKKGLSKGCFGLKLDRIGSMSGLGC (CNP-41) (SEQ ID NO:26);
RLLQEHPNARKYKGANKKGLSKGCFGLKLDRIGSMSGLGC (CNP-40) (SEQ ID NO:27);
LLQEHPNARKYKGANKKGLSKGCFGLKLDRIGSMSGLGC (CNP-39) (SEQ ID NO:28);
LQEHPNARKYKGANKKGLSKGCFGLKLDRIGSMSGLGC (SEQ ID NO:2);
QEHPNARKYKGANKKGLSKGCFGLKLDRIGSMSGLGC (CNP-37) (SEQ ID NO:3);
EHPNARKYKGANKKGLSKGCFGLKLDRIGSMSGLGC (CNP-36) (SEQ ID NO:29);
HPNARKYKGANKKGLSKGCFGLKLDRIGSMSGLGC (CNP-35) (SEQ ID NO:30);
PNARKYKGANKKGLSKGCFGLKLDRIGSMSGLGC (CNP-34) (SEQ ID NO:4);
NARKYKGANKKGLSKGCFGLKLDRIGSMSGLGC (CNP-33) (SEQ ID NO:31);
ARKYKGANKKGLSKGCFGLKLDRIGSMSGLGC (CNP-32) (SEQ ID NO:32);
RKYKGANKKGLSKGCFGLKLDRIGSMSGLGC (CNP-31) (SEQ ID NO:33);
KYKGANKKGLSKGCFGLKLDRIGSMSGLGC (CNP-30) (SEQ ID NO:34);
YKGANKKGLSKGCFGLKLDRIGSMSGLGC (CNP-29) (SEQ ID NO:35);
KGANKKGLSKGCFGLKLDRIGSMSGLGC (CNP-28) (SEQ ID NO:36);
GANKKGLSKGCFGLKLDRIGSMSGLGC (CNP-27) (SEQ ID NO:37);
ANKKGLSKGCFGLKLDRIGSMSGLGC (CNP-26) (SEQ ID NO:38);
NKKGLSKGCFGLKLDRIGSMSGLGC (CNP-25) (SEQ ID NO:39);
KKGLSKGCFGLKLDRIGSMSGLGC (CNP-24) (SEQ ID NO:40);
KGLSKGCFGLKLDRIGSMSGLGC (CNP-23) (SEQ ID NO:41);
GLSKGCFGLKLDRIGSMSGLGC (CNP-22) (SEQ ID NO: 68);
LSKGCFGLKLDRIGSMSGLGC (CNP-21) (SEQ ID NO:42);
SKGCFGLKLDRIGSMSGLGC (CNP-20) (SEQ ID NO:43);
KGCFGLKLDRIGSMSGLGC (CNP-19) (SEQ ID NO:44);
GCFGLKLDRIGSMSGLGC (CNP-18) (SEQ ID NO:45);
CFGLKLDRIGSMSGLGC (CNP-17) (SEQ ID NO: 67),
QEHPNARKYKGANKKGLSKGCFGLKLDRIGSNSGLGC [CNP-37(M32N); SEQ ID NO: 46];
MQEHPNARKYKGANKKGLSKGCFGLKLDRIGSMSGLGC (Met-CNP-37; SEQ ID NO:47);
PQEHPNARKYKGANKKGLSKGCFGLKLDRIGSMSGLGC (Pro-CNP-37; SEQ ID NO: 48);
GQEHPNARKYKGANKKGLSKGCFGLKLDRIGSNSGLGC [Gly-CNP-37 (M32N); SEQ ID NO: 49];
MGQEHPNARKYKGANKKGLSKGCFGLKLDRIGSMSGLGC (Met-Gly-CNP-37; SEQ ID NO: 50);
GQEHPNARKYKGANKKGLSKGCFGLKLDRIGSMSGLGC (Gly-CNP-37: SEQ ID NO:51)
GQEHPNARKYKGANPKGLSKGCFGLKLDRIGSMSGLGC (SEQ ID NO:52);
GQEHPNARKYKGANQKGLSKGCFGLKLDRIGSMSGLGC (SEQ ID NO:53);
GQEHPNARKYKGANQQGLSKGCFGLKLDRIGSMSGLGC (SEQ ID NO:54);
GQEHPNARKYKGANKPGLSKGCFGLKLDRIGSMSGLGC (SEQ ID NO:55),
PGQEHPNARKYKGANKKGLSKGCFGLKLDRIGSMSGLGC(SEQ ID NO: 1);
PGQEHPNARRYRGANRRGLSRGCFGLKLDRIGSMSGLGC(SEQ ID NO: 6);
PGQEHPQARRYRGAQRRGLSRGCFGLKLDRIGSMSGLGC(SEQ ID NO: 5); und
PGQEHPQARKYKGAQKKGLSKGCFGLKLDRIGSMSGLGC(SEQ ID NO: 7),
gegebenenfalls wobei das Salz ferner ein mehrwertiges Kation im Komplex mit dem Peptid-Gegenion-Komplex umfasst.

2. Zusammensetzung nach Anspruch 1, wobei das CNP, das hydrophobe Gegenion und das Kation über eine nichtkovalente Bindung komplexiert sind.

3. Zusammensetzung nach einem der Ansprüche 1 bis 2, wobei es sich bei dem Kation um ein Metallkation handelt, gegebenenfalls wobei das Kation ein Metall umfasst, das aus der Gruppe bestehend aus Beryllium (Be), Magnesium (Mg), Calcium (Ca), Strontium (Sr), Barium (Ba), Zink (Zn), Cadmium (Cd), Bor (B), Aluminium (Al), Gallium (Ga), Indium (In), Thallium (Tl), Eisen (Fe), Mangan (Mn), Cobalt (Co), Nickel (Ni), Titan (Ti), Vanadium (V), Platin (Pt), Kupfer (Cu) und Gold (Au) ausgewählt ist.

4. Zusammensetzung nach einem der Ansprüche 1-3, wobei das hydrophobe Gegenion aus der Gruppe bestehend aus einer deprotonierten Fettsäure, einer deprotonierten Gallensäure, einem ionischen Tensid, Naphthoat und Derivaten davon, Nicotinat und Derivaten davon, einem Alkylsulfonat, einem Dialkylsulfosuccinat, einem Phospholipid, einem Alkylsulfonat, einem Arylsulfonat, einem Alkylbenzolsulfonat, einem Alkylsulfat, einem Arylsulfat, einem Dextransulfat, einem Alkylbenzolsulfat und einer beliebigen Kombination der Vorstehenden ausgewählt ist, gegebenenfalls wobei das hydrophobe Gegenion aus der Gruppe bestehend aus Palmitat, Desoxycholat, Oleat, Pamoat, Nicotinat, Dodecylsulfat, Docusat, Myristat, Stearat, Phosphatidylethanolamin (PE), Phosphatidylcholin (PC), Phosphatidylserin (PS), Phosphatidylinositol (PL), Phosphatidat, Decanoat, 2-Naphthalensulfonat, 1-Heptansulfonat, 1-Octansulfonat-Monohydrat, 1-Decansulfonat, Dodecylsulfat, Dextransulfat und Dodecylbenzolsulfonat ausgewählt ist.

5. Zusammensetzung nach einem der Ansprüche 1 bis 4, wobei es sich bei dem CNP um eine CNP-Variante handelt, gegebenenfalls wobei das CNP ausgewählt ist aus der Gruppe bestehend aus:
PGQEHPNARKYKGANKKGLSKGCFGLKLDRIGSMSGLGC (Pro-Gly-CNP-37; SEQ ID NO: 1)
LQEHPNARKYKGANKKGLSKGCFGLKLDRIGSMSGLGC (SEQ ID NO:2);
QEHPNARKYKGANKKGLSKGCFGLKLDRIGSMSGLGC (CNP-37) (SEQ ID NO:3);
PNARKYKGANKKGLSKGCFGLKLDRIGSMSGLGC (CNP-34) (SEQ ID NO:4)
und Salzen davon.

6. Zusammensetzung nach einem der Ansprüche 1 bis 5, ferner umfassend ein Hilfs-, Verdünnungs- oder Trägermittel.

7. Sterile pharmazeutische Zusammensetzung, umfassend die Zusammensetzung nach einem der Ansprüche 1-6.

8. Extended-Release-Zusammensetzung, umfassend ein Salz eines CNP (C-type Natriuretic Peptide), wobei das Salz das elektrostatisch geladene Peptid im Komplex mit einem hydrophoben Gegenion umfasst, gegebenenfalls wobei das Salz ferner ein Kation im Komplex mit dem Peptid-Gegenion-Komplex umfasst, wobei das CNP ausgewählt ist aus der Gruppe bestehend aus:
DLRVDTKSRAAWARLLQEHPNARKYKGANKKGLSKGCFGLKLDRIGSMSGLGC (CNP-53) (SEQ ID NO: 56);
LRVDTKSRAAWARLLQEHPNARKYKGANKKGLSKGCFGLKLDRIGSMSGLGC (CNP-52) (SEQ ID NO:15);
RVDTKSRAAWARLLQEHPNARKYKGANKKGLSKGCFGLKLDRIGSMSGLGC (CNP-51) (SEQ ID NO:16);
VDTKSRAA WARLLQEH PNARKYKGAN KKGLSKGCFG LKLDRIGSMSG LGC (CNP-50) (SEQ ID NO17:);
DTKSRAAWARLLQEHPNARKYKGANKKGLSKGCFGLKLDRIGSMSGLGC (CNP-49) (SEQ ID NO: 18)
TKSRAAWARLLQEHPNARKYKGANKKGLSKGCFGLKLDRIGSMSGLGC (CNP-48) (SEQ ID NO:19);
KSRAAWARLLQEHPNARKYKGANKKGLSKGCFGLKLDRIGSMSGLGC (CNP-47) (SEQ ID NO:20);
SRAAWARLLQEHPNARKYKGANKKGLSKGCFGLKLDRIGSMSGLGC (CNP-46) (SEQ ID NO:21);
RAAWARLLQEHPNARKYKGANKKGLSKGCFGLKLDRIGSMSGLGC (CNP-45) (SEQ ID NO:22);
AAWARLLQEHPNARKYKGANKKGLSKGCFGLKLDRIGSMSGLGC (CNP-44) (SEQ ID NO:23);
AWARLLQEHPNARKYKGANKKGLSKGCFGLKLDRIGSMSGLGC (CNP-43) (SEQ ID NO:24);
WARLLQEHPNARKYKGANKKGLSKGCFGLKLDRIGSMSGLGC (CNP-42) (SEQ ID NO:25);
ARLLQEHPNARKYKGANKKGLSKGCFGLKLDRIGSMSGLGC (CNP-41) (SEQ ID NO:26);
RLLQEHPNARKYKGANKKGLSKGCFGLKLDRIGSMSGLGC (CNP-40) (SEQ ID NO:27);
LLQEHPNARKYKGANKKGLSKGCFGLKLDRIGSMSGLGC (CNP-39) (SEQ ID NO:28);
LQEHPNARKYKGANKKGLSKGCFGLKLDRIGSMSGLGC (SEQ ID NO:2);
QEHPNARKYKGANKKGLSKGCFGLKLDRIGSMSGLGC (CNP-37) (SEQ ID NO:3);
EHPNARKYKGANKKGLSKGCFGLKLDRIGSMSGLGC (CNP-36) (SEQ ID NO:29);
HPNARKYKGANKKGLSKGCFGLKLDRIGSMSGLGC (CNP-35) (SEQ ID NO:30);
PNARKYKGANKKGLSKGCFGLKLDRIGSMSGLGC (CNP-34) (SEQ ID NO:4);
NARKYKGANKKGLSKGCFGLKLDRIGSMSGLGC (CNP-33) (SEQ ID NO:31);
ARKYKGANKKGLSKGCFGLKLDRIGSMSGLGC (CNP-32) (SEQ ID NO:32);
RKYKGANKKGLSKGCFGLKLDRIGSMSGLGC (CNP-31) (SEQ ID NO:33);
KYKGANKKGLSKGCFGLKLDRIGSMSGLGC (CNP-30) (SEQ ID NO:34);
YKGANKKGLSKGCFGLKLDRIGSMSGLGC (CNP-29) (SEQ ID NO:35);
KGANKKGLSKGCFGLKLDRIGSMSGLGC (CNP-28) (SEQ ID NO:36);
GANKKGLSKGCFGLKLDRIGSMSGLGC (CNP-27) (SEQ ID NO:37);
ANKKGLSKGCFGLKLDRIGSMSGLGC (CNP-26) (SEQ ID NO:38);
NKKGLSKGCFGLKLDRIGSMSGLGC (CNP-25) (SEQ ID NO:39);
KKGLSKGCFGLKLDRIGSMSGLGC (CNP-24) (SEQ ID NO:40);
KGLSKGCFGLKLDRIGSMSGLGC (CNP-23) (SEQ ID NO:41);
GLSKGCFGLKLDRIGSMSGLGC (CNP-22) (SEQ ID NO: 68);
LSKGCFGLKLDRIGSMSGLGC (CNP-21) (SEQ ID NO:42);
SKGCFGLKLDRIGSMSGLGC (CNP-20) (SEQ ID NO:43);
KGCFGLKLDRIGSMSGLGC (CNP-19) (SEQ ID NO:44);
GCFGLKLDRIGSMSGLGC (CNP-18) (SEQ ID NO:45);
CFGLKLDRIGSMSGLGC (CNP-17) (SEQ ID NO: 67),
QEHPNARKYKGANKKGLSKGCFGLKLDRIGSNSGLGC [CNP-37(M32N); SEQ ID NO: 46];
MQEHPNARKYKGANKKGLSKGCFGLKLDRIGSMSGLGC (Met-CNP-37; SEQ ID NO:47);
PQEHPNARKYKGANKKGLSKGCFGLKLDRIGSMSGLGC (Pro-CNP-37; SEQ ID NO: 48);
GQEHPNARKYKGANKKGLSKGCFGLKLDRIGSNSGLGC [Gly-CNP-37 (M32N); SEQ ID NO: 49];
MGQEHPNARKYKGANKKGLSKGCFGLKLDRIGSMSGLGC (Met-Gly-CNP-37; SEQ ID NO: 50);
GQEHPNARKYKGANKKGLSKGCFGLKLDRIGSMSGLGC (Gly-CNP-37: SEQ ID NO:51)
GQEHPNARKYKGANPKGLSKGCFGLKLDRIGSMSGLGC (SEQ ID NO:52);
GQEHPNARKYKGANQKGLSKGCFGLKLDRIGSMSGLGC (SEQ ID NO:53);
GQEHPNARKYKGANQQGLSKGCFGLKLDRIGSMSGLGC (SEQ ID NO:54);
GQEHPNARKYKGANKPGLSKGCFGLKLDRIGSMSGLGC (SEQ ID NO:55),
PGQEHPNARKYKGANKKGLSKGCFGLKLDRIGSMSGLGC(SEQ ID NO: 1);
PGQEHPNARRYRGANRRGLSRGCFGLKLDRIGSMSGLGC(SEQ ID NO: 6);
PGQEHPQARRYRGAQRRGLSRGCFGLKLDRIGSMSGLGC(SEQ ID NO: 5); und
PGQEHPQARKYKGAQKKGLSKGCFGLKLDRIGSMSGLGC(SEQ ID NO: 7).

9. Extended-Release-Zusammensetzung nach Anspruch 8, wobei das Peptidsalz in einer wässrigen Lösung oder in Öl suspendiert ist, gegebenenfalls wobei das Öl ein Triglycerid oder eine Fettsäure umfasst, gegebenenfalls wobei die Fettsäure gesättigt oder ungesättigt ist, gegebenenfalls wobei es sich bei der Fettsäure um eine C-6- bis C-20-Fettsäure handelt, gegebenenfalls wobei es sich bei der Fettsäure um Hexansäure, Octansäure, Decansäure oder Dodecansäure handelt.

10. Extended-Release-Zusammensetzung nach einem der Ansprüche 8 bis 9, wobei
(i) weniger als 20 % Peptid bis Tag 1 freigesetzt werden; und
(ii) etwa 90 % Peptid bis Tag 7 freigesetzt werden oder etwa 90 % Peptid bis Tag 14 freigesetzt werden oder etwa 90 % Peptid bis Tag 31 freigesetzt werden,
jeweils bei pH 7 bis 7,6.

11. Zusammensetzung, umfassend ein hydrophobes CNP-Salz nach einem der Ansprüche 1 bis 10 zur Verwendung beim Behandeln einer Knochenwachstumsstörung oder Skelettdysplasie oder zur Verlängerung eines Knochens oder Steigerung von Langknochenwachstum.

12. Zusammensetzung zur Verwendung gemäß Anspruch 11, wobei die Knochenwachstumsstörung oder Skelettdysplasie aus der Gruppe bestehend aus Osteoarthritis, hypophosphatämischer Rachitis, Achondroplasie, Hypochondroplasie, Kleinwuchs, Zwergwuchs, Osteochondrodysplasien, thanatophorer Dysplasie, Osteogenesis imperfecta, Achondrogenese, Chondrodysplasia punctata, homozygoter Achondroplasie, Chondrodysplasia punctata, kampomeler Dysplasie, kongenitaler letaler Hypophosphatasie, perinataler letaler Osteogenesis imperfecta, Kurze-Rippen-Polydaktylie-Syndromen, Hypochondroplasie, rhizomeler Chondrodysplasia punctata, metaphysärer Dysplasie vom Jansen-Typ, kongenitaler spondyloepiphysärer Dysplasie, Atelosteogenese, diastrophischer Dysplasie, angeborenem kurzem Femur, mesomeler Dysplasie vom Langer-Typ, mesomeler Dysplasie vom Nievergelt-Typ, Robinow-Syndrom, Reinhardt-Syndrom, Akrodysostose, peripherer Dysostose, Kniest-Dysplasie, Fibrochondrogenese, Roberts-Syndrom, akromesomeler Dysplasie, Mikromelie, Morquio-Syndrom, Kniest-Syndrom, metatrophischer Dysplasie und spondyloepimetaphysärer Dysplasie, NPR2-Mutation, SHOX-Mutation (Turner-Syndrom/Leri-Weill), PTPN11-Mutationen (Noonan-Syndrom), Insulin-Wachstumsfaktor-1-Rezeptor (IGF1R)-Mutation und idiopathischer Kleinwüchsigkeit ausgewählt ist.

13. Zusammensetzung zur Verwendung gemäß einem der Ansprüche 11 oder 12, wobei die Zusammensetzung subkutan, intradermal, intraartikulär, oral oder intramuskulär verabreicht wird, gegebenenfalls wobei die Zusammensetzung einmal täglich, einmal wöchentlich, einmal alle zwei Wochen, einmal alle drei Wochen, einmal alle 4 Wochen, einmal alle 6 Wochen, einmal alle zwei Monate, einmal alle drei Monate oder einmal alle sechs Monate verabreicht wird.

14. Hydrophobes Salz von CNP (C-type Natriuretic Peptide), umfassend ein CNP im Komplex mit einem hydrophoben Gegenion, gegebenenfalls ferner umfassend ein Kation im Komplex mit dem Peptid und Gegenion, gegebenenfalls wobei das hydrophobe Gegenion aus der Gruppe bestehend aus Oleat, Desoxycholat, Decanoat, Pamoat, Docusat oder Dodecylsulfat ausgewählt ist, wobei das CNP ausgewählt ist aus der Gruppe bestehend aus:
DLRVDTKSRAAWARLLQEHPNARKYKGANKKGLSKGCFGLKLDRIGSMSGLGC (CNP-53) (SEQ ID NO: 56);
LRVDTKSRAAWARLLQEHPNARKYKGANKKGLSKGCFGLKLDRIGSMSGLGC (CNP-52) (SEQ ID NO:15);
RVDTKSRAAWARLLQEHPNARKYKGANKKGLSKGCFGLKLDRIGSMSGLGC (CNP-51) (SEQ ID NO:16);
VDTKSRAA WARLLQEH PNARKYKGAN KKGLSKGCFG LKLDRIGSMSG LGC (CNP-50) (SEQ ID NO17:);
DTKSRAAWARLLQEHPNARKYKGANKKGLSKGCFGLKLDRIGSMSGLGC (CNP-49) (SEQ ID NO: 18)
TKSRAAWARLLQEHPNARKYKGANKKGLSKGCFGLKLDRIGSMSGLGC (CNP-48) (SEQ ID NO:23);
KSRAAWARLLQEHPNARKYKGANKKGLSKGCFGLKLDRIGSMSGLGC (CNP-47) (SEQ ID NO:24);
SRAAWARLLQEHPNARKYKGANKKGLSKGCFGLKLDRIGSMSGLGC (CNP-46) (SEQ ID NO:25);
RAAWARLLQEHPNARKYKGANKKGLSKGCFGLKLDRIGSMSGLGC (CNP-45) (SEQ ID NO:26);
AAWARLLQEHPNARKYKGANKKGLSKGCFGLKLDRIGSMSGLGC (CNP-44) (SEQ ID NO:23);
AWARLLQEHPNARKYKGANKKGLSKGCFGLKLDRIGSMSGLGC (CNP-43) (SEQ ID NO:24);
WARLLQEHPNARKYKGANKKGLSKGCFGLKLDRIGSMSGLGC (CNP-42) (SEQ ID NO:25);
ARLLQEHPNARKYKGANKKGLSKGCFGLKLDRIGSMSGLGC (CNP-41) (SEQ ID NO:26);
RLLQEHPNARKYKGANKKGLSKGCFGLKLDRIGSMSGLGC (CNP-40) (SEQ ID NO:27);
LLQEHPNARKYKGANKKGLSKGCFGLKLDRIGSMSGLGC (CNP-39) (SEQ ID NO:28);
LQEHPNARKYKGANKKGLSKGCFGLKLDRIGSMSGLGC (SEQ ID NO:2);
QEHPNARKYKGANKKGLSKGCFGLKLDRIGSMSGLGC (CNP-37) (SEQ ID NO:3);
EHPNARKYKGANKKGLSKGCFGLKLDRIGSMSGLGC (CNP-36) (SEQ ID NO:29);
HPNARKYKGANKKGLSKGCFGLKLDRIGSMSGLGC (CNP-35) (SEQ ID NO:30);
PNARKYKGANKKGLSKGCFGLKLDRIGSMSGLGC (CNP-34) (SEQ ID NO:4);
NARKYKGANKKGLSKGCFGLKLDRIGSMSGLGC (CNP-33) (SEQ ID NO:31);
ARKYKGANKKGLSKGCFGLKLDRIGSMSGLGC (CNP-32) (SEQ ID NO:32);
RKYKGANKKGLSKGCFGLKLDRIGSMSGLGC (CNP-31) (SEQ ID NO:33);
KYKGANKKGLSKGCFGLKLDRIGSMSGLGC (CNP-30) (SEQ ID NO:34);
YKGANKKGLSKGCFGLKLDRIGSMSGLGC (CNP-29) (SEQ ID NO:35);
KGANKKGLSKGCFGLKLDRIGSMSGLGC (CNP-28) (SEQ ID NO:36);
GANKKGLSKGCFGLKLDRIGSMSGLGC (CNP-27) (SEQ ID NO:37);
ANKKGLSKGCFGLKLDRIGSMSGLGC (CNP-26) (SEQ ID NO:38);
NKKGLSKGCFGLKLDRIGSMSGLGC (CNP-25) (SEQ ID NO:39);
KKGLSKGCFGLKLDRIGSMSGLGC (CNP-24) (SEQ ID NO:40);
KGLSKGCFGLKLDRIGSMSGLGC (CNP-23) (SEQ ID NO:41);
GLSKGCFGLKLDRIGSMSGLGC (CNP-22) (SEQ ID NO: 68);
LSKGCFGLKLDRIGSMSGLGC (CNP-21) (SEQ ID NO:42);
SKGCFGLKLDRIGSMSGLGC (CNP-20) (SEQ ID NO:43);
KGCFGLKLDRIGSMSGLGC (CNP-19) (SEQ ID NO:44);
GCFGLKLDRIGSMSGLGC (CNP-18) (SEQ ID NO:45);
CFGLKLDRIGSMSGLGC (CNP-17) (SEQ ID NO: 67),
QEHPNARKYKGANKKGLSKGCFGLKLDRIGSNSGLGC [CNP-37(M32N); SEQ ID NO: 46];
MQEHPNARKYKGANKKGLSKGCFGLKLDRIGSMSGLGC (Met-CNP-37; SEQ ID NO:47);
PQEHPNARKYKGANKKGLSKGCFGLKLDRIGSMSGLGC (Pro-CNP-37; SEQ ID NO: 48);
GQEHPNARKYKGANKKGLSKGCFGLKLDRIGSNSGLGC [Gly-CNP-37 (M32N); SEQ ID NO: 49];
MGQEHPNARKYKGANKKGLSKGCFGLKLDRIGSMSGLGC (Met-Gly-CNP-37; SEQ ID NO: 50);
GQEHPNARKYKGANKKGLSKGCFGLKLDRIGSMSGLGC (Gly-CNP-37: SEQ ID NO:51)
GQEHPNARKYKGANPKGLSKGCFGLKLDRIGSMSGLGC (SEQ ID NO:52);
GQEHPNARKYKGANQKGLSKGCFGLKLDRIGSMSGLGC (SEQ ID NO:53);
GQEHPNARKYKGANQQGLSKGCFGLKLDRIGSMSGLGC (SEQ ID NO:54);
GQEHPNARKYKGANKPGLSKGCFGLKLDRIGSMSGLGC (SEQ ID NO:55),
PGQEHPNARKYKGANKKGLSKGCFGLKLDRIGSMSGLGC(SEQ ID NO: 1);
PGQEHPNARRYRGANRRGLSRGCFGLKLDRIGSMSGLGC(SEQ ID NO: 6);
PGQEHPQARRYRGAQRRGLSRGCFGLKLDRIGSMSGLGC(SEQ ID NO: 5); und
PGQEHPQARKYKGAQKKGLSKGCFGLKLDRIGSMSGLGC(SEQ ID NO: 7).

15. Hydrophobes Salz nach Anspruch 14, wobei das Salz aus der Gruppe bestehend aus CNP-Ca⁺²(Oleat), CNP-Ca⁺²(Pamoat), CNP-Ca⁺²(desoxycholat), CNP-Ca⁺²(decanoat), CNP-Ca⁺²(docusat), CNP-Zn⁺²(Oleat), CNP-Zn⁺²(Pamoat), CNP-Zn⁺²(desoxycholat), CNP-Zn⁺²(decanoat) und CNP-Zn⁺²(docusat) ausgewählt ist.

16. Hydrophobes Salz nach einem der Ansprüche 14 bis 15, wobei das CNP ausgewählt ist aus der Gruppe bestehend aus:
PGQEHPNARKYKGANKKGLSKGCFGLKLDRIGSMSGLGC (Pro-Gly-CNP-37; SEQ ID NO: 1)
LQEHPNARKYKGANKKGLSKGCFGLKLDRIGSMSGLGC (SEQ ID NO:2);
QEHPNARKYKGANKKGLSKGCFGLKLDRIGSMSGLGC (CNP-37) (SEQ ID NO:3);
PNARKYKGANKKGLSKGCFGLKLDRIGSMSGLGC (CNP-34) (SEQ ID NO:4)
und Salzen davon.

## Revendications

1. Composition comprenant un sel hydrophobe d'un peptide natriurétique de type C (CNP), le sel comprenant le CNP complexé avec un contre-ion hydrophobe, dans laquelle le CNP est choisi dans le groupe constitué par :
DLRVDTKSRAAWARLLQEHPNARKYKGANKKGLSKGCFGLKLDRIGSMSGLGC (CNP-53) (SEQ ID N° : 56) ;
LRVDTKSRAAWARLLQEHPNARKYKGANKKGLSKGCFGLKLDRIGSMSGLGC (CNP-52) (SEQ ID N° : 15) ;
RVDTKSRAAWARLLQEHPNARKYKGANKKGLSKGCFGLKLDRIGSMSGLGC (CNP-51) (SEQ ID N° : 16) ;
VDTKSRAAWARLLQEHPNARKYKGANKKGLSKGCFGLKLDRIGSMSGLGC (CNP-50) (SEQ ID N° : 17) ;
DTKSRAAWARLLQEHPNARKYKGANKKGLSKGCFGLKLDRIGSMSGLGC (CNP-49) (SEQ ID N° : 18)
TKSRAAWARLLQEHPNARKYKGANKKGLSKGCFGLKLDRIGSMSGLGC (CNP-48) (SEQ ID N° : 19) ;
KSRAAWARLLQEHPNARKYKGANKKGLSKGCFGLKLDRIGSMSGLGC (CNP-47) (SEQ ID N° : 20) ;
SRAAWARLLQEHPNARKYKGANKKGLSKGCFGLKLDRIGSMSGLGC (CNP-46) (SEQ ID N° : 21) ;
RAAWARLLQEHPNARKYKGANKKGLSKGCFGLKLDRIGSMSGLGC (CNP-45) (SEQ ID N° : 22) ;
AAWARLLQEHPNARKYKGANKKGLSKGCFGLKLDRIGSMSGLGC (CNP-44) (SEQ ID N° : 23) ;
AWARLLQEHPNARKYKGANKKGLSKGCFGLKLDRIGSMSGLGC (CNP-43) (SEQ ID N° : 24) ;
WARLLQEHPNARKYKGANKKGLSKGCFGLKLDRIGSMSGLGC (CNP-42) (SEQ ID N° : 25) ;
ARLLQEHPNARKYKGANKKGLSKGCFGLKLDRIGSMSGLGC (CNP-41) (SEQ ID N° : 26) ;
RLLQEHPNARKYKGANKKGLSKGCFGLKLDRIGSMSGLGC (CNP-40) (SEQ ID N° : 27) ;
LLQEHPNARKYKGANKKGLSKGCFGLKLDRIGSMSGLGC (CNP-39) (SEQ ID N° : 28) ;
LQEHPNARKYKGANKKGLSKGCFGLKLDRIGSMSGLGC (SEQ ID N° : 2) ;
QEHPNARKYKGANKKGLSKGCFGLKLDRIGSMSGLGC (CNP-37) (SEQ ID N° : 3) ;
EHPNARKYKGANKKGLSKGCFGLKLDRIGSMSGLGC (CNP-36) (SEQ ID N° : 29) ;
HPNARKYKGANKKGLSKGCFGLKLDRIGSMSGLGC (CNP-35) (SEQ ID N° : 30) ;
PNARKYKGANKKGLSKGCFGLKLDRIGSMSGLGC (CNP-34) (SEQ ID N° : 4) ;
NARKYKGANKKGLSKGCFGLKLDRIGSMSGLGC (CNP-33) (SEQ ID N° : 31) ;
ARKYKGANKKGLSKGCFGLKLDRIGSMSGLGC (CNP-32) (SEQ ID N° : 32) ;
RKYKGANKKGLSKGCFGLKLDRIGSMSGLGC (CNP-31) (SEQ ID N° : 33) ;
KYKGANKKGLSKGCFGLKLDRIGSMSGLGC (CNP-30) (SEQ ID N° : 34) ;
YKGANKKGLSKGCFGLKLDRIGSMSGLGC (CNP-29) (SEQ ID N° : 35) ;
KGANKKGLSKGCFGLKLDRIGSMSGLGC (CNP-28) (SEQ ID N° : 36) ;
GANKKGLSKGCFGLKLDRIGSMSGLGC (CNP-27) (SEQ ID N° : 37) ;
ANKKGLSKGCFGLKLDRIGSMSGLGC (CNP-26) (SEQ ID N° : 38) ;
NKKGLSKGCFGLKLDRIGSMSGLGC (CNP-25) (SEQ ID N° : 39) ;
KKGLSKGCFGLKLDRIGSMSGLGC (CNP-24) (SEQ ID N° : 40) ;
KGLSKGCFGLKLDRIGSMSGLGC (CNP-23) (SEQ ID N0: 41) ;
GLSKGCFGLKLDRIGSMSGLGC (CNP-22) (SEQ ID N° : 68) ;
LSKGCFGLKLDRIGSMSGLGC (CNP-21) (SEQ ID N° : 42) ;
SKGCFGLKLDRIGSMSGLGC (CNP-20) (SEQ ID N° : 43) ;
KGCFGLKLDRIGSMSGLGC (CNP-19) (SEQ ID N° : 44) ;
GCFGLKLDRIGSMSGLGC (CNP-18) (SEQ ID N° : 45) ;
CFGLKLDRIGSMSGLGC (CNP-17) (SEQ ID N° : 67),
QEHPNARKYKGANKKGLSKGCFGLKLDRIGSNSGLGC [CNP-37(M32N) ; SEQ ID N° : 46] ;
MQEHPNARKYKGANKKGLSKGCFGLKLDRIGSMSGLGC (Met-CNP-37 ; SEQ ID N° : 47) ;
PQEHPNARKYKGANKKGLSKGCFGLKLDRIGSMSGLGC (Pro-CNP-37 ; SEQ ID N° : 48) ;
GQEHPNARKYKGANKKGLSKGCFGLKLDRIGSNSGLGC [Gly-CNP-37 (M32N) ; SEQ ID N° : 49] ; MGQEHPNARKYKGANKKGLSKGCFGLKLDRIGSMSGLGC (Met-Gly-CNP-37 ; SEQ ID N° : 50) ; GQEHPNARKYKGANKKGLSKGCFGLKLDRIGSMSGLGC (Gly-CNP-37 : SEQ ID N° : 51)
GQEHPNARKYKGANPKGLSKGCFGLKLDRIGSMSGLGC (SEQ ID N° : 52) ;
GQEHPNARKYKGANQKGLSKGCFGLKLDRIGSMSGLGC (SEQ ID N° : 53) ;
GQEHPNARKYKGANQQGLSKGCFGLKLDRIGSMSGLGC (SEQ ID N° : 54) ;
GQEHPNARKYKGANKPGLSKGCFGLKLDRIGSMSGLGC (SEQ ID N° : 55),
PGQEHPNARKYKGANKKGLSKGCFGLKLDRIGSMSGLGC (SEQ ID N° : 1) ;
PGQEHPNARRYRGANRRGLSRGCFGLKLDRIGSMSGLGC (SEQ ID N° : 6) ;
PGQEHPQARRYRGAQRRGLSRGCFGLKLDRIGSMSGLGC (SEQ ID N° : 5) ; et
PGQEHPQARKYKGAQKKGLSKGCFGLKLDRIGSMSGLGC (SEQ ID N° : 7),
éventuellement dans laquelle le sel comprend en outre un cation multivalent complexé au complexe peptide-contre-ion.

2. Composition selon la revendication 1, dans laquelle le CNP, le contre-ion hydrophobe et le cation sont complexés par le biais d'une liaison non covalente.

3. Composition selon l'une quelconque des revendications 1 à 2 dans laquelle le cation est un cation métallique, éventuellement dans laquelle le cation comprend un métal choisi dans le groupe constitué par le béryllium (Be), le magnésium (Mg), le calcium (Ca), le strontium (Sr), le baryum (Ba), le zinc (Zn), le cadmium (Cd), le bore (B), l'aluminium (Al), le gallium (Ga), l'indium (In), le thallium (Tl), le fer (Fe), le manganèse (Mn), le cobalt (Co), le nickel (Ni), le titane (Ti), le vanadium (V), le platine (Pt), le cuivre (Cu) et l'or (Au).

4. Composition selon l'une quelconque des revendications 1 à 3, dans laquelle le contre-ion hydrophobe est choisi dans le groupe constitué par un acide gras déprotoné, un acide biliaire déprotoné, un tensioactif ionique, le naphtoate et les dérivés de celui-ci, le nicotinate et les dérivés de celui-ci, un alkylsulfonate, un dialkylsulfosuccinate, un phospholipide, un alkylsulfonate, un arylsulfonate, un alkylbenzènesulfonate, un alkylsulfate, un arylsulfate, un sulfate de dextrane, un alkylbenzènesulfate et une combinaison de plusieurs des contre-ions précités quels qu'ils soient, éventuellement dans laquelle le contre-ion hydrophobe est choisi dans le groupe constitué par le palmitate, le désoxycholate, l'oléate, le pamoate, le nicotinate, le dodécylsulfate, le docusate, le myristate, le stéarate, la phosphatidyléthanolamine (PE), la phosphatidylcholine (PC), la phosphatidylsérine (PS), le phosphatidylinositol (PL), le phosphatidate, le décanoate, le 2-naphtalènesulfonate, le 1-heptanesulfonate, le 1-octanesulfonate monohydraté, le 1-décanesulfonate, le dodécylsulfate, le sulfate de dextrane et le dodécylbenzènesulfonate.

5. Composition selon l'une quelconque des revendications 1 à 4 dans laquelle le CNP est un variant de CNP, éventuellement dans laquelle le CNP est choisi dans le groupe constitué par:
PGQEHPNARKYKGANKKGLSKGCFGLKLDRIGSMSGLGC (Pro-Gly-CNP-37 ; SEQ ID N° : 1)
LQEHPNARKYKGANKKGLSKGCFGLKLDRIGSMSGLGC (SEQ ID N° : 2) ;
QEHPNARKYKGANKKGLSKGCFGLKLDRIGSMSGLGC (CNP-37) (SEQ ID N° : 3) ;
PNARKYKGANKKGLSKGCFGLKLDRIGSMSGLGC (CNP-34) (SEQ ID N° : 4) et les sels de ceux-ci.

6. Composition selon l'une quelconque des revendications 1 à 5 comprenant en outre un excipient, diluant ou véhicule.

7. Composition pharmaceutique stérile comprenant la composition selon l'une quelconque des revendications 1 à 6.

8. Composition à libération prolongée comprenant un sel d'un peptide natriurétique de type C (CNP), le sel comprenant le peptide électrostatiquement chargé complexé avec un contre-ion hydrophobe, éventuellement dans laquelle le sel comprend en outre un cation complexé au complexe peptide-contre-ion, dans laquelle le CNP est choisi dans le groupe constitué par :
DLRVDTKSRAAWARLLQEHPNARKYKGANKKGLSKGCFGLKLDRIGSMSGLGC (CNP-53) (SEQ ID N° : 56) ;
LRVDTKSRAAWARLLQEHPNARKYKGANKKGLSKGCFGLKLDRIGSMSGLGC (CNP-52) (SEQ ID N° : 15) ;
RVDTKSRAAWARLLQEHPNARKYKGANKKGLSKGCFGLKLDRIGSMSGLGC (CNP-51) (SEQ ID N° : 16) ;
VDTKSRAAWARLLQEHPNARKYKGANKKGLSKGCFGLKLDRIGSMSGLGC (CNP-50) (SEQ ID N° : 17) ;
DTKSRAAWARLLQEHPNARKYKGANKKGLSKGCFGLKLDRIGSMSGLGC (CNP-49) (SEQ ID N° : 18)
TKSRAAWARLLQEHPNARKYKGANKKGLSKGCFGLKLDRIGSMSGLGC (CNP-48) (SEQ ID N° : 19) ;
KSRAAWARLLQEHPNARKYKGANKKGLSKGCFGLKLDRIGSMSGLGC (CNP-47) (SEQ ID N° : 20) ;
SRAAWARLLQEHPNARKYKGANKKGLSKGCFGLKLDRIGSMSGLGC (CNP-46) (SEQ ID N° : 21) ;
RAAWARLLQEHPNARKYKGANKKGLSKGCFGLKLDRIGSMSGLGC (CNP-45) (SEQ ID N° : 22) ;
AAWARLLQEHPNARKYKGANKKGLSKGCFGLKLDRIGSMSGLGC (CNP-44) (SEQ ID N° : 23) ;
AWARLLQEHPNARKYKGANKKGLSKGCFGLKLDRIGSMSGLGC (CNP-43) (SEQ ID N° : 24) ;
WARLLQEHPNARKYKGANKKGLSKGCFGLKLDRIGSMSGLGC (CNP-42) (SEQ ID N° : 25) ;
ARLLQEHPNARKYKGANKKGLSKGCFGLKLDRIGSMSGLGC (CNP-41) (SEQ ID N° : 26) ;
RLLQEHPNARKYKGANKKGLSKGCFGLKLDRIGSMSGLGC (CNP-40) (SEQ ID N° : 27) ;
LLQEHPNARKYKGANKKGLSKGCFGLKLDRIGSMSGLGC (CNP-39) (SEQ ID N° : 28) ;
LQEHPNARKYKGANKKGLSKGCFGLKLDRIGSMSGLGC (SEQ ID N° : 2) ;
QEHPNARKYKGANKKGLSKGCFGLKLDRIGSMSGLGC (CNP-37) (SEQ ID N° : 3) ;
EHPNARKYKGANKKGLSKGCFGLKLDRIGSMSGLGC (CNP-36) (SEQ ID N° : 29) ;
HPNARKYKGANKKGLSKGCFGLKLDRIGSMSGLGC (CNP-35) (SEQ ID N° : 30) ;
PNARKYKGANKKGLSKGCFGLKLDRIGSMSGLGC (CNP-34) (SEQ ID N° : 4) ;
NARKYKGANKKGLSKGCFGLKLDRIGSMSGLGC (CNP-33) (SEQ ID N° : 31) ;
ARKYKGANKKGLSKGCFGLKLDRIGSMSGLGC (CNP-32) (SEQ ID N° : 32) ;
RKYKGANKKGLSKGCFGLKLDRIGSMSGLGC (CNP-31) (SEQ ID N° : 33) ;
KYKGANKKGLSKGCFGLKLDRIGSMSGLGC (CNP-30) (SEQ ID N° : 34) ;
YKGANKKGLSKGCFGLKLDRIGSMSGLGC (CNP-29) (SEQ ID N° : 35) ;
KGANKKGLSKGCFGLKLDRIGSMSGLGC (CNP-28) (SEQ ID N° : 36) ;
GANKKGLSKGCFGLKLDRIGSMSGLGC (CNP-27) (SEQ ID N° : 37) ;
ANKKGLSKGCFGLKLDRIGSMSGLGC (CNP-26) (SEQ ID N° : 38) ;
NKKGLSKGCFGLKLDRIGSMSGLGC (CNP-25) (SEQ ID N° : 39) ;
KKGLSKGCFGLKLDRIGSMSGLGC (CNP-24) (SEQ ID N° : 40) ;
KGLSKGCFGLKLDRIGSMSGLGC (CNP-23) (SEQ ID N0: 41) ;
GLSKGCFGLKLDRIGSMSGLGC (CNP-22) (SEQ ID N° : 68) ;
LSKGCFGLKLDRIGSMSGLGC (CNP-21) (SEQ ID N° : 42) ;
SKGCFGLKLDRIGSMSGLGC (CNP-20) (SEQ ID N° : 43) ;
KGCFGLKLDRIGSMSGLGC (CNP-19) (SEQ ID N° : 44) ;
GCFGLKLDRIGSMSGLGC (CNP-18) (SEQ ID N° : 45) ;
CFGLKLDRIGSMSGLGC (CNP-17) (SEQ ID N° : 67),
QEHPNARKYKGANKKGLSKGCFGLKLDRIGSNSGLGC [CNP-37(M32N) ; SEQ ID N° : 46] ;
MQEHPNARKYKGANKKGLSKGCFGLKLDRIGSMSGLGC (Met-CNP-37 ; SEQ ID N° : 47) ;
PQEHPNARKYKGANKKGLSKGCFGLKLDRIGSMSGLGC (Pro-CNP-37 ; SEQ ID N° : 48) ;
GQEHPNARKYKGANKKGLSKGCFGLKLDRIGSNSGLGC [Gly-CNP-37 (M32N) ; SEQ ID N° : 49] ;
MGQEHPNARKYKGANKKGLSKGCFGLKLDRIGSMSGLGC (Met-Gly-CNP-37 ; SEQ ID N° : 50) ;
GQEHPNARKYKGANKKGLSKGCFGLKLDRIGSMSGLGC (Gly-CNP-37 : SEQ ID N° : 51)
GQEHPNARKYKGANPKGLSKGCFGLKLDRIGSMSGLGC (SEQ ID N° : 52) ;
GQEHPNARKYKGANQKGLSKGCFGLKLDRIGSMSGLGC (SEQ ID N° : 53) ;
GQEHPNARKYKGANQQGLSKGCFGLKLDRIGSMSGLGC (SEQ ID N° : 54) ;
GQEHPNARKYKGANKPGLSKGCFGLKLDRIGSMSGLGC (SEQ ID N° : 55),
PGQEHPNARKYKGANKKGLSKGCFGLKLDRIGSMSGLGC (SEQ ID N° : 1) ;
PGQEHPNARRYRGANRRGLSRGCFGLKLDRIGSMSGLGC (SEQ ID N° : 6) ;
PGQEHPQARRYRGAQRRGLSRGCFGLKLDRIGSMSGLGC (SEQ ID N° : 5) ; et
PGQEHPQARKYKGAQKKGLSKGCFGLKLDRIGSMSGLGC (SEQ ID N° : 7).

9. Composition à libération prolongée selon la revendication 8 dans laquelle le sel de peptide est remis en suspension dans une solution aqueuse ou dans de l'huile, éventuellement dans laquelle l'huile comprend un triglycéride ou un acide gras, éventuellement dans laquelle l'acide gras est saturé ou insaturé, éventuellement dans laquelle l'acide gras est un acide gras en C-6 à C-20, éventuellement dans laquelle l'acide gras est l'acide hexanoïque, l'acide octanoïque, l'acide décanoïque ou l'acide dodécanoïque.

10. Composition à libération prolongée selon l'une quelconque des revendications 8 à 9, dans laquelle
(i) moins de 20 % de peptide est libéré avant la fin du jour 1 ; et
(ii) environ 90 % de peptide est libéré avant la fin du jour 7, ou environ 90 % du peptide est libéré avant la fin du jour 14, ou environ 90 % du peptide est libéré avant la fin du jour 31,
à un pH de 7 à 7,6.

11. Composition comprenant un sel hydrophobe de CNP selon l'une quelconque des revendications 1 à 10 destinée à être utilisée en traitement d'un trouble de la croissance osseuse ou d'une dysplasie squelettique ou pour l'allongement d'un os ou l'augmentation de la croissance d'un os long.

12. Composition destinée à être utilisée selon la revendication 11, le trouble de la croissance osseuse ou la dysplasie squelettique étant choisis dans le groupe constitué par l'arthrose, le rachitisme hypophosphatémique, une achondroplasie, une hypochondroplasie, une petite taille, le nanisme, les ostéochondrodysplasies, une dysplasie thanatophore, une ostéogenèse imparfaite, une achondrogenèse, une chondrodysplasie ponctuée, une achondroplasie homozygote, une chondrodysplasie ponctuée, une dysplasie campomélique, une hypophosphatasie congénitale létale, un type périnatal létal d'ostéogenèse imparfaite, les syndromes de polydactylie des côtes courtes, une hypochondroplasie, un type rhizomélique de chondrodysplasie ponctuée, une dysplasie métaphysaire type Jansen, une dysplasie spondylo-épiphisaire congénitale, une atélostéogenèse, une dysplasie diastrophique, un fémur court congénital, une dysplasie mésomélique type Langer, une dysplasie mésomélique type Nievergelt, le syndrome de Robinow, le syndrome de Reinhardt, une acrodysostose, une dysostose périphérique, la dysplasie de Kniest, une fibrochondrogenèse, le syndrome de Roberts, une dysplasie acromésomélique, une micromélie, le syndrome de Morquio, le syndrome de Kniest, une dysplasie métatrophique et une dysplasie spondylo-épimétaphysaire, une mutation de NPR2, une mutation de SHOX (syndrome de Turner/syndrome de Leri Weill), des mutations de PTPN11 (syndrome de Noonan), une mutation du récepteur du facteur de croissance apparenté à l'insuline de type 1 (IGF1R) et une petite taille idiopathique.

13. Composition destinée à être utilisée selon l'une quelconque des revendications 11 ou 12, la composition étant administrée par voie sous-cutanée, intradermique, intra-articulaire, orale ou intramusculaire, éventuellement la composition étant administrée une fois par jour, une fois par semaine, une fois toutes les deux semaines, une fois toutes les trois semaines, une fois toutes les 4 semaines, une fois toutes les 6 semaines, une fois tous les deux mois, une fois tous les trois mois ou une fois tous les six mois.

14. Sel hydrophobe de peptide natriurétique de type C (CNP) comprenant un CNP en complexe avec un contre-ion hydrophobe, comprenant éventuellement en outre un cation complexé avec le peptide et le contre-ion, éventuellement dans lequel le contre-ion hydrophobe est choisi dans le groupe constitué par l'oléate, le désoxycholate, le décanoate, le pamoate, le docusate et le dodécylsulfate, dans lequel le CNP est choisi dans le groupe constitué par :
DLRVDTKSRAAWARLLQEHPNARKYKGANKKGLSKGCFGLKLDRIGSMSGLGC (CNP-53) (SEQ ID N° : 56) ;
LRVDTKSRAAWARLLQEHPNARKYKGANKKGLSKGCFGLKLDRIGSMSGLGC (CNP-52) (SEQ ID N° : 15) ;
RVDTKSRAAWARLLQEHPNARKYKGANKKGLSKGCFGLKLDRIGSMSGLGC (CNP-51) (SEQ ID N° : 16) ;
VDTKSRAAWARLLQEHPNARKYKGANKKGLSKGCFGLKLDRIGSMSGLGC (CNP-50) (SEQ ID N° : 17) ;
DTKSRAAWARLLQEHPNARKYKGANKKGLSKGCFGLKLDRIGSMSGLGC (CNP-49) (SEQ ID N° : 18)
TKSRAAWARLLQEHPNARKYKGANKKGLSKGCFGLKLDRIGSMSGLGC (CNP-48) (SEQ ID N° : 23) ;
KSRAAWARLLQEHPNARKYKGANKKGLSKGCFGLKLDRIGSMSGLGC (CNP-47) (SEQ ID N° : 24) ;
SRAAWARLLQEHPNARKYKGANKKGLSKGCFGLKLDRIGSMSGLGC (CNP-46) (SEQ ID N° : 25) ;
RAAWARLLQEHPNARKYKGANKKGLSKGCFGLKLDRIGSMSGLGC (CNP-45) (SEQ ID N° : 26) ;
AAWARLLQEHPNARKYKGANKKGLSKGCFGLKLDRIGSMSGLGC (CNP-44) (SEQ ID N° : 23) ;
AWARLLQEHPNARKYKGANKKGLSKGCFGLKLDRIGSMSGLGC (CNP-43) (SEQ ID N° : 24) ;
WARLLQEHPNARKYKGANKKGLSKGCFGLKLDRIGSMSGLGC (CNP-42) (SEQ ID N° : 25) ;
ARLLQEHPNARKYKGANKKGLSKGCFGLKLDRIGSMSGLGC (CNP-41) (SEQ ID N° : 26) ;
RLLQEHPNARKYKGANKKGLSKGCFGLKLDRIGSMSGLGC (CNP-40) (SEQ ID N° : 27) ;
LLQEHPNARKYKGANKKGLSKGCFGLKLDRIGSMSGLGC (CNP-39) (SEQ ID N° : 28) ;
LQEHPNARKYKGANKKGLSKGCFGLKLDRIGSMSGLGC (SEQ ID N° : 2) ;
QEHPNARKYKGANKKGLSKGCFGLKLDRIGSMSGLGC (CNP-37) (SEQ ID N° : 3) ;
EHPNARKYKGANKKGLSKGCFGLKLDRIGSMSGLGC (CNP-36) (SEQ ID N° : 29) ;
HPNARKYKGANKKGLSKGCFGLKLDRIGSMSGLGC (CNP-35) (SEQ ID N° : 30) ;
PNARKYKGANKKGLSKGCFGLKLDRIGSMSGLGC (CNP-34) (SEQ ID N° : 4) ;
NARKYKGANKKGLSKGCFGLKLDRIGSMSGLGC (CNP-33) (SEQ ID N° : 31) ;
ARKYKGANKKGLSKGCFGLKLDRIGSMSGLGC (CNP-32) (SEQ ID N° : 32) ;
RKYKGANKKGLSKGCFGLKLDRIGSMSGLGC (CNP-31) (SEQ ID N° : 33) ;
KYKGANKKGLSKGCFGLKLDRIGSMSGLGC (CNP-30) (SEQ ID N° : 34) ;
YKGANKKGLSKGCFGLKLDRIGSMSGLGC (CNP-29) (SEQ ID N° : 35) ;
KGANKKGLSKGCFGLKLDRIGSMSGLGC (CNP-28) (SEQ ID N° : 36) ;
GANKKGLSKGCFGLKLDRIGSMSGLGC (CNP-27) (SEQ ID N° : 37) ;
ANKKGLSKGCFGLKLDRIGSMSGLGC (CNP-26) (SEQ ID N° : 38) ;
NKKGLSKGCFGLKLDRIGSMSGLGC (CNP-25) (SEQ ID N° : 39) ;
KKGLSKGCFGLKLDRIGSMSGLGC (CNP-24) (SEQ ID N° : 40) ;
KGLSKGCFGLKLDRIGSMSGLGC (CNP-23) (SEQ ID N0: 41) ;
GLSKGCFGLKLDRIGSMSGLGC (CNP-22) (SEQ ID N° : 68) ;
LSKGCFGLKLDRIGSMSGLGC (CNP-21) (SEQ ID N° : 42) ;
SKGCFGLKLDRIGSMSGLGC (CNP-20) (SEQ ID N° : 43) ;
KGCFGLKLDRIGSMSGLGC (CNP-19) (SEQ ID N° : 44) ;
GCFGLKLDRIGSMSGLGC (CNP-18) (SEQ ID N° : 45) ;
CFGLKLDRIGSMSGLGC (CNP-17) (SEQ ID N° : 67),
QEHPNARKYKGANKKGLSKGCFGLKLDRIGSNSGLGC [CNP-37(M32N) ; SEQ ID N° : 46] ;
MQEHPNARKYKGANKKGLSKGCFGLKLDRIGSMSGLGC (Met-CNP-37 ; SEQ ID N° : 47) ;
PQEHPNARKYKGANKKGLSKGCFGLKLDRIGSMSGLGC (Pro-CNP-37 ; SEQ ID N° : 48) ;
GQEHPNARKYKGANKKGLSKGCFGLKLDRIGSNSGLGC [Gly-CNP-37 (M32N) ; SEQ ID N° : 49] ;
MGQEHPNARKYKGANKKGLSKGCFGLKLDRIGSMSGLGC (Met-Gly-CNP-37 ; SEQ ID N° : 50) ;
GQEHPNARKYKGANKKGLSKGCFGLKLDRIGSMSGLGC (Gly-CNP-37 : SEQ ID N° : 51)
GQEHPNARKYKGANPKGLSKGCFGLKLDRIGSMSGLGC (SEQ ID N° : 52) ;
GQEHPNARKYKGANQKGLSKGCFGLKLDRIGSMSGLGC (SEQ ID N° : 53) ;
GQEHPNARKYKGANQQGLSKGCFGLKLDRIGSMSGLGC (SEQ ID N° : 54) ;
GQEHPNARKYKGANKPGLSKGCFGLKLDRIGSMSGLGC (SEQ ID N° : 55),
PGQEHPNARKYKGANKKGLSKGCFGLKLDRIGSMSGLGC (SEQ ID N° : 1) ;
PGQEHPNARRYRGANRRGLSRGCFGLKLDRIGSMSGLGC (SEQ ID N° : 6) ;
PGQEHPQARRYRGAQRRGLSRGCFGLKLDRIGSMSGLGC (SEQ ID N° : 5) ; et
PGQEHPQARKYKGAQKKGLSKGCFGLKLDRIGSMSGLGC (SEQ ID N° : 7).

15. Sel hydrophobe selon la revendication 14, le sel étant choisi dans le groupe constitué par l'oléate de CNP-Ca²⁺, le pamoate de CNP-Ca²⁺, le désoxycholate de CNP-Ca²⁺, le décanoate de CNP-Ca²⁺, le docusate de CNP-Ca²⁺, l'oléate de CNP-Zn²⁺, le pamoate de CNP-Zn²⁺, le désoxycholate de CNP-Zn²⁺, le décanoate de CNP-Zn²⁺ et le docusate de CNP-Zn²⁺.

16. Sel hydrophobe selon l'une quelconque des revendications 14 à 15, dans lequel le CNP est choisi dans le groupe constitué par:
PGQEHPNARKYKGANKKGLSKGCFGLKLDRIGSMSGLGC (Pro-Gly-CNP-37 ; SEQ ID N° : 1)
LQEHPNARKYKGANKKGLSKGCFGLKLDRIGSMSGLGC (SEQ ID N° : 2) ;
QEHPNARKYKGANKKGLSKGCFGLKLDRIGSMSGLGC (CNP-37) (SEQ ID N° : 3) ;
PNARKYKGANKKGLSKGCFGLKLDRIGSMSGLGC (CNP-34) (SEQ ID N° : 4) et les sels de ceux-ci.
